# EUROPÄISCHE PATENTANMELDUNG

(11) **EP 0 236 874 A2**
(43) Veröffentlichungstag der Anmeldung: **16.09.1987**
(21) Anmeldenummer: 87102781.9
(22) Anmeldetag: 27.02.1987
(51) Int. Cl.: C07K 5/02, C07D 233/64, C07C 101/30, C07C 103/50, C07C 125/065, C07C 95/00, A61K 37/64, A61K 31/415, A61K 31/16, A61K 31/22

(54) **Renininhibitoren, deren Herstellung und Verwendung sowie Aminosäure- und Aminoaldehyd-Derivate**

(30) Priorität: 12.03.1986 DE 3608209; 23.08.1986 DE 3628650
(71) Anmelder: BAYER AG, 51368 Leverkusen (DE)
(72) Erfinder: Bender, Wolfgang, Dr., D-5600 Wuppertal 1 (DE); Henning, Rolf, Dr., D-5600 Wuppertal 1 (DE); Knorr, Andreas, Dr., D-4006 Erkrath 2 (DE); Stasch, Johannes-Peter, Dr., D-5600 Wuppertal 1 (DE)

(57) **Zusammenfassung**

Die Erfindung betrifft renininhibitorische Peptide der allgemeinen Formel 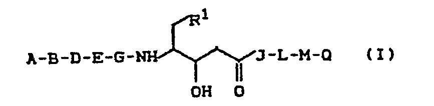 in welcher A, B, D, E, G, R¹, J, L, M und Q die in der Beschreibung angegebene Bedeutung haben, Verfahren zu ihrer Herstellung und ihre Verwendung in Arzneimitteln, insbesondere in kreislaufbeeinflussenden Arzneimitteln, sowie Aminosäure- und Aminoaldehyd-Derivate.

## Beschreibung

Die Erfindung betrifft renininhibitorische Peptide, Verfahren zu ihrer Herstellung und ihre Verwendung in Arzneimitteln, insbesondere in kreislaufbeeinflussenden Arzneimitteln, sowie Aminosäure- und Aminoaldehyd-Derivate.

Renin ist ein proteolytisches Enzym, das überwiegend von den Nieren produziert und ins Plasma sezerniert wird. Es ist bekannt, daß Renin in vivo vom Angiotensinogen das Dekapeptid Angiotensin 1 abspaltet. Angiotensin 1 wiederum wird in der Lunge, den Nieren oder anderen Geweben zu dem blutdruckwirksamen Oktapeptid Angiotensin II abgebaut. Die verschiedenen Effekte des Angiotensin II wie Vasokonstriktion, Na⁺⁻Retention in der Niere, Aldosteronfreisetzung in der Nebenniere und Tonuserhöhung des sympathischen Nervensystems wirken synergistisch im Sinne einer Blutdruckerhöhung.

Aus diesem Grund spielt das Renin-Angiotensin-System eine wichtige Rolle bei der Regulation der kardiovaskulären Homöostase sowie bei bestimmten Formen der Hypertonie.

Die Aktivität des Renin-Angiotensin-Systems kann durch die Hemmung der Aktivität von Renin oder dem Angiotensin-Konversionsenzym (ACE) sowie durch Blockade von Angiotensin II-Rezeptoren pharmakologisch manipuliert werden. Die Entwicklung von oral einsetzbaren ACE-Hemmern hat somit zu neuen Antihypertensiva geführt.

Ein neuerer Ansatz ist, in die Renin-Angiotensin-Kaskadezu einem früheren Zeitpunkt einzugreifen, nämlich durch Inhibition der hochspezifischen Peptidase Renin.

Bisher wurden verschiedene Arten von Renininhibitoren entwickelt: Reninspezifische Antikörper, Phospholipide, Peptide mit der N-terminalen Sequenz des Prorenins, synthetische Peptide als Substratanaloga und modifizierte Peptide.

Die potentesten bis heute bekannten Renininhibitoren gehören zu den beiden letztgenannten Gruppen. Sie haben jedoch eine Reihe von Nachteilen, so zum Beispiel schlechte Wasserlöslichkeit, geringe Bioverfügbarkeit, kurze Wirkdauer bei parenteraler Verabreichung oder, wenn überhaupt, nur schwache Wirksamkeit bei oraler Gabe. t

Die vorliegende Erfindung betrifft Peptide enthaltend bis zu 8 Aminosäuregruppierungen der allgemeinen Formel (I), 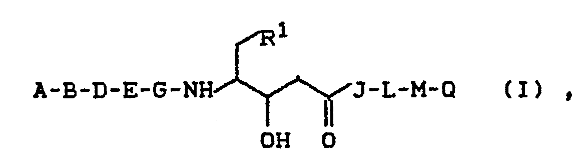 in welcher
A - für Wasserstoff, C₁-C₈-Alkyl, C₇-C₁₄-Aralkyl, Phenylsulfonyl, Tolylsulfonyl, C₁-C₈-Alkylsulfonyl oder
   - für eine Aminoschutzgruppe steht,
B - für eine direkte Bindung steht, oder
   - für Sarcosyl oder für eine Gruppe der Formel 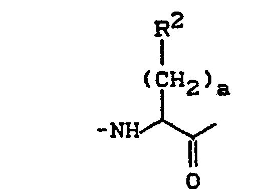 steht, worin
   a - eine Zahl 0,1,2,3 oder 4 bedeutet und
   R²⁻ Wasserstoff, Cₗ-C₆-Alkyl, Hydroxy-Cₗ-C₂-alkyl, eine Gruppe der Formel -CH₂-CO-NHR³ oder -CH₂-NH-R³ bedeutet,
      wobei
      _{R}³⁻ für Wasserstoff, C₁-C₆-Alkyl, C₇-C₁₄-Aralkyl, Tolylsulfonyl, Phenylsulfonyl, C₁-C₆-Alkylsulfonyl oder für eine Aminoschutzgruppe steht, oder
   R^{2 -} Guanidinomethyl, Mercaptomethyl, Methylthiomethyl, Carboxymethyl, C₁-C₆-Alkoxycarbonylmethyl, C₇-C₁₄-Aralkoxycarbonylmethyl, Halogen, Indolylmethyl, 4-Imidazolylmethyl, Pyridyl, Triazolylmethyl, Pyrazolylmethyl oder Aryl bedeutet, das bis zu dreifach gleich oder verschieden substituiert sein kann durch C₁-C₄-Alkyl, C₁-C₄-Alkoxy, Trifluormethyl, Halogen, Hydroxy, Nitro oder durch eine Gruppe der Formel 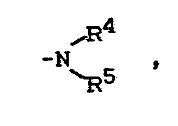 wobei
      R4,R5 - gleich oder verschieden sind und - für Wasserstoff, C₁-C₆-Alkyl, Aryl, Aralkyl, Phenylsulfonyl, Tolylsulfonyl, C_{I}-C₆-Alkylsulfonyl, Acetyl, Benzoyl oder für eine Aminoschutzgruppe stehen,
D - für eine direkte Bindung steht, oder für eine Gruppe der Formel 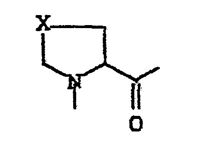 worin
   X - für Methylen, Ethylen oder Schwefel steht,
E - die gleiche Bedeutung hat wie B und mit diesem gleich oder verschieden ist,
G - die gleiche Bedeutung hat wie B und mit diesem gleich oder verschieden ist,
   R¹- für eine Gruppe der Formel 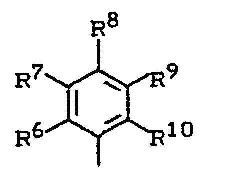 steht, worin
      _{R}⁶, R⁷, R^{S}, R⁹ und R¹⁰- gleich oder verschieden sind
         und
         - für Wasserstoff, C_{I}-C₆-Alkyl, C₁-C₆-Alkoxy, Aryl, Halogen, Hydroxy, Cyano, Trifluormethyl, Trifluormethoxy, Trifluormethylthio, Nitro oder für eine Gruppe der Formel 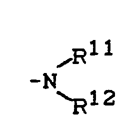 stehen,
         wobei
      R¹¹,R¹²- die gleiche Bedeutung haben wie R⁴,R⁵ und mit diesen gleich oder verschieden sind,
         mit der Maßgabe, daß mindestens einer der Substituenten R⁶, R⁷, R⁸, R⁹ oder R¹⁰ für Nitro oder die Gruppe -NR¹¹R¹² stehen muß,
      J, L, M - jeweils gleich oder verschieden sind und die gleiche Bedeutung haben wie B und mit diesem gleich oder verschieden sind,

      und
Q - für einen Rest der Formel -OR¹³, -NHR¹⁴, oder -N¹⁴R¹⁵ oder -NH-NHR³ steht,
   worin
   R¹³- für Wasserstoff oder - für C₁-C₂₀-Alkyl steht, das gegebenenfalls durch ein Sauerstoffatom in der Kette unterbrochen ist und das substituiert sein kann durch Halogen, Hydroxy, Phenyl oder Pyridyl,
   R¹⁴- für Wasserstoff, C₁-C₁₀-Alkyl steht, das substituiert sein kann durch Aryl, wobei dieser Arylrest wiederum Substituenten aus der Reihe Nitro, C_{I}-C₄-Alkyl, Cₗ-C₄-Alkoxy, Halogen oder Cyano tragen kann, durch Halogen, Adamantyl, Chinuclidin, Piperidin, N-Methylpiperazin, N-Phenylpiperazin, N-Benzylpiperazin, Pyridyl oder Morpholin, oder
      - für Aryl steht, das bis zu dreifach gleich oder verschieden substituiert sein kann durch Nitro, Halogen, C₁-C₄-Alkoxy, C₁-C₄-Alkyl, wobei Alkyl wiederum Substituenten aus der Reihe Hydroxy, Amino, Carboxy und/oder C_{I}-C₄-Alkoxycarbonyl tragen kann, oder
      - für Adamantyl oder Chinuclidin steht, und
   _{R}^{15 -} für C_{I}-C₆-Alkyl steht,
      oder
   _{R}¹⁴ und _{R}^{15 -} gemeinsam einen 5- bis 7-gliedrigen Ring bilden, der als weiteres Heteroatom ein Sauerstoffatom, ein Schwefelatom oder die Gruppe -NH, -N-C₁-C₆-Alkyl, -N-Dimethylamino-C_{I}-C₄-alkyl, -N-Aryl oder -N-Aralkyl enthalten kann oder der durch C₁-C₄-Alkyl oder Aralkyl substituiert sein kann
   und R³ die angegebene Bedeutung hat, und deren physiologisch unbedenklichen Salze.

Im Rahmen der vorliegenden Erfindung steht Aryl für einen aromatischen Kohlenwasserstoffrest mit 6 bis 14 Kohlenstoffatomen. Bevorzugt steht Aryl für Phenyl oder Naphthyl.

Alkyl steht im Rahmen der Erfindung für geradkettige oder verzweigte Alkylgruppen mit der jeweils angegebenen Anzahl an Kohlenwasserstoffatomen. Alkyl umfaßt ebenso Cycloalkyl, Cycloalkylalkyl oder Alkylcycloalkyl.

Aralkyl steht im Rahmen der Erfindung für einen aromatischen Kohlenwasserstoffrest mit 6 bis 14 Kohlenstoffatomen, der über eine Alkylkette mit 1 bis 6 Kohlenstoffatomen gebunden ist. Bevorzugt steht Aralkyl für Benzyl, Phenethyl oder Naphthylmethyl. Aminoschutzgruppe steht im Rahmen der Erfindung für die üblichen in der Peptid-Chemie verwendeten Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbony1,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methyl- silyl)ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Valeroyl, Isovaleroyl, Butyryl, Ethylthiocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)-phosphoryl, Phenoxyphenylphosphoryl, Diethylphosphinyl, Diphenylphosphinyl, Phthaloyl oder Phthalimido.

Besonders bevorzugte Aminoschutzgruppen sind Benzyloxycarbonyl, 3,4-Dimethoxybenzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, Hexoxycarbonyl, Octoxycarbonyl, 2-Bromethoxycarbonyl, 2-Chlorethoxycarbonyl, Phenoxyacetyl, Naphthylcarbonyl, Adamatylcarbonyl, Phthaloyl, 2,2,2-Trichlorethoxycarbonyl, 2,2,2-Trichlor-tert-butoxycarbonyl, Menthyloxycarbonyl, Vinyloxycarbonyl, Allyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido oder Isovaleroyl.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate.

Bevorzugt liegen die Grupppen B, D, E, G, J, L und M unabhängig voneinander in der optisch reinen, bevorzugt in der L-Form vor.

Die Gruppierung der Formel 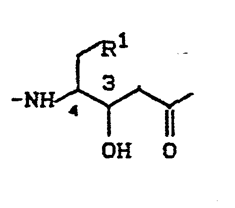 besitzt 2 asymmetrische Kohlenstoffatome, die unabhängig voneinander in der R- oder S-Konfiguration vorliegen können. Bevorzugt liegt diese Gruppierung in der 3S,4S-Konfiguration, 3R,4S-Konfiguration, 3S,4R- oder 3R,4R-Konfiguration vor, besonders bevorzugt in der 3S,4S-oder der 3R,4S-Konfiguration vor. Ebenso wird die Gruppierung als Isomerengemisch der Konfiguration 3RS-4S eingesetzt.

Die erfindungsgemäßen Verbindungen der allgemeinen Formel (I) können in Form ihrer Salze vorliegen. Dies können Salze der erfindungsgemäßen Verbindungen mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugte Verbindungen der allgemeinen Formel (I) sind solche, in denen
A - für Wasserstoff, C₁-C₄-Alkyl, Benzyl, Phenylsulfonyl, Tolylsulfonyl, C_{I}-C₄-Alkylsulfonyl oder
   - für eine Aminoschutzgruppe steht,
B - für eine direkte Bindung oder für einen Rest der Formel steht 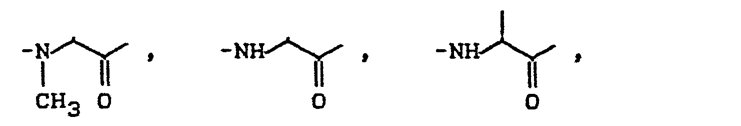 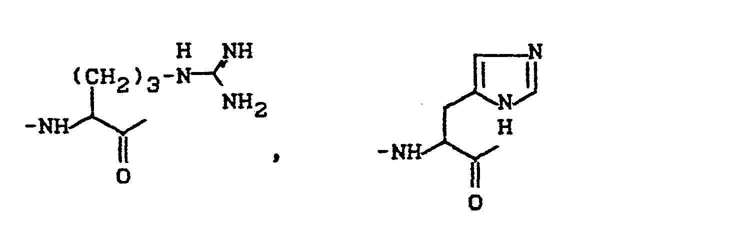 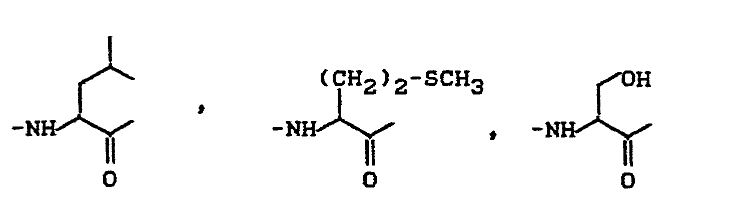 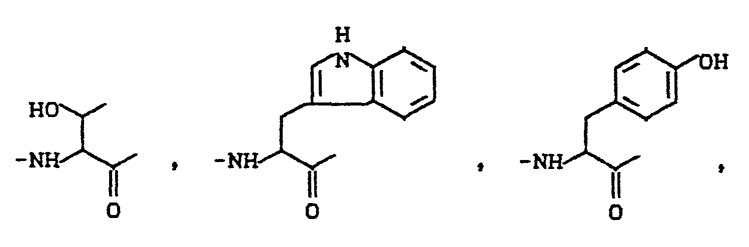 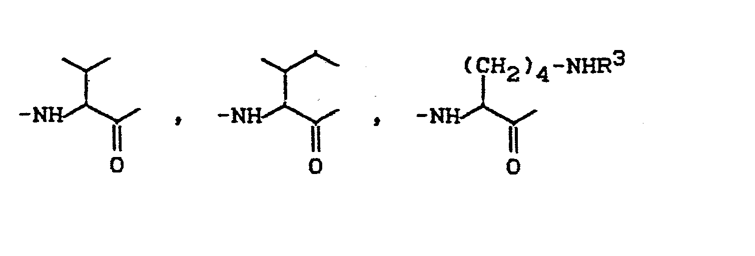 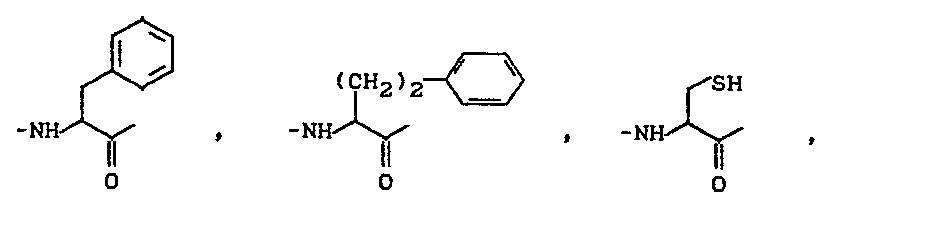 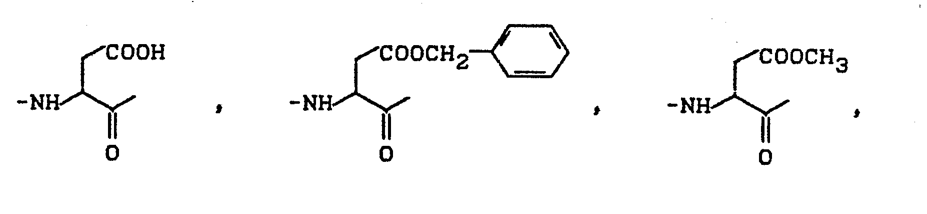 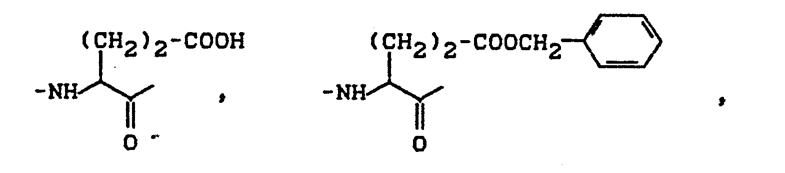 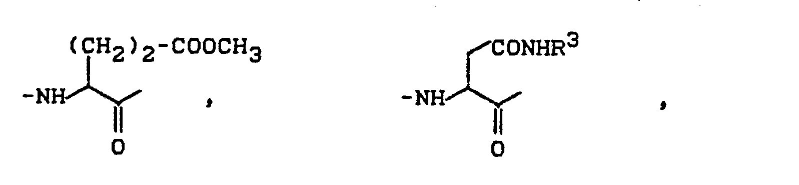 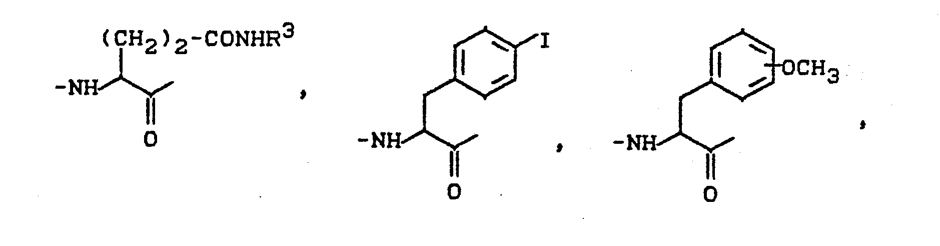 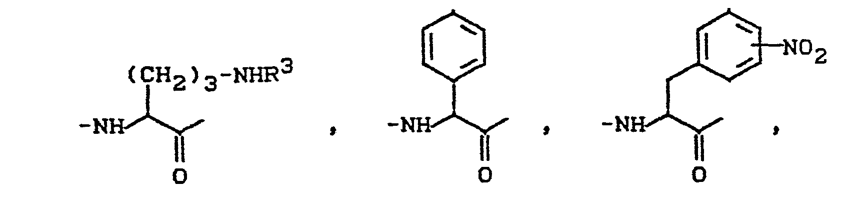 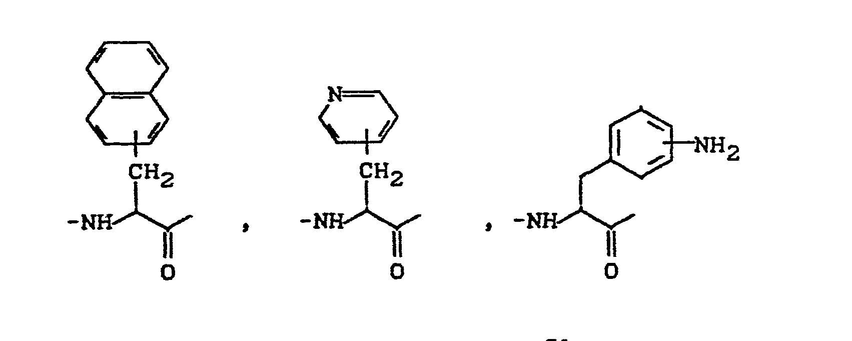 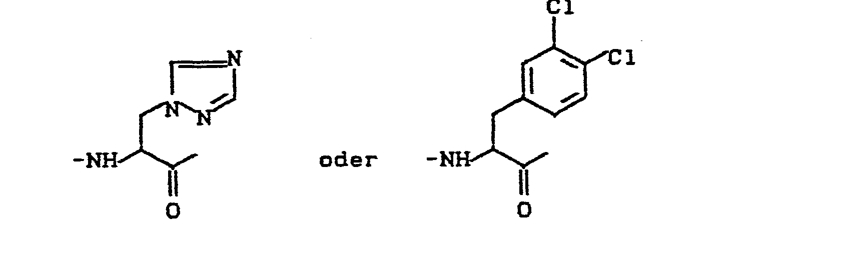 in ihrer D-Form, L-Form oder als D,L-Isomerengemisch, bevorzugt in der L-Form,
   worin
   R³⁻ für Wasserstoff, C₁-C₄-Alkyl, Benzyl, Phenylsulfonyl, Tolylsulfonyl, C₁-C₄-Alkylsulfonyl oder
      - für eine Aminoschutzgruppe steht,
D - für eine direkte Bindung oder für die Gruppe der Formel 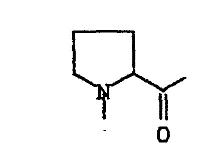 in ihrer L-Form, D-Form oder als D,L-Isomerengemisch steht,
E,G - jeweils gleich oder verschieden sind und die gleiche Bedeutung haben wie B und mit diesem gleich oder verschieden sind,
   R¹⁻ für eine Gruppe der Formel 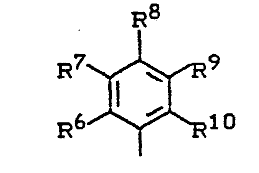 steht,
      worin
      R⁶,R⁷,R⁸,R⁹ und R¹⁰ gleich oder verschieden sind und - für Wasserstoff, Methyl, Methoxy, Fluor, Chlor, Brom, Iod, Hydroxy, Cyano, Trifluormethyl, Nitro oder für eine Gruppe der Formel 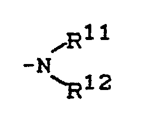 stehen, worin
      R¹¹,R¹²- gleich oder verschieden sind und - für Wasserstoff, C₁-C₄-Alkyl, Phenyl, Benzyl, Phenylsulfonyl, Tosyl, C₁-C₄-Alkylsulfonyl, Acetyl oder - für eine Aminoschutzgruppe stehen,
      mit der Maßgabe, daß mindestens einer der Sub- ₛtiₜᵤenten _{R}⁶, R⁷, R⁸, R⁹ oder R¹⁰ für Nitro oder für die Gruppe -NR¹¹R¹² stehen muß,
J,L,M - jeweils gleich oder verschieden sind und - die gleiche Bedeutung haben wie B und mit diesem gleich oder verschieden sein können,
   und
Q - für einen Rest der Formel -OR¹³, -NH-NHR3, -NHR¹⁴ oder 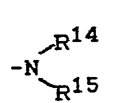 steht, worin
   _{R}^{13 -} für Wasserstoff oder C₁-C₁₈-Alkyl steht, das durch Chlor, Brom, Hydroxy oder Phenyl substituiert sein kann,
   R¹⁴ - für Wasserstoff, C₁-C₄-Alkyl steht, das substituiert sein kann durch gegebenenfalls durch Nitro, Methyl oder Methoxy substituiertes Phenyl, durch Fluor, Chlor, Brom, Pyridyl, Adamantyl, Chinuclidin, Piperidin, N-Methyl-, N-Phenyl- oder N-Benzylpiperazin oder Benzylcyclohexyl oder - für Phenyl steht, das gegebenenfalls substituiert ist durch Nitro, Fluor, Chlor, Methoxy oder durch gegebenenfalls durch Hydroxy, Amino, Carboxy und/oder C₁-C₄-Alkoxycarbonyl substituiertes C₁-C₄-Alkyl, oder - für Adamantyl oder Chinuclidin steht,
   R^{15 -} für C_{I}-C₄-Alkyl steht,
      oder
   _{R}¹⁴ und R¹⁵ gemeinsam einen Ring aus der Reihe Pyrrolidin, Piperidin, Benzylpiperidin, Morpholin, Piperazin, N-Methyl-, N-Phenyl-, N-Benzyl-, -N-Dimethylaminoethylpiperazin bilden und
   _{R}^{3 -} die angegebene Bedeutung hat

   und deren physiologisch unbedenklichen Salze.

Besonders bevorzugte Verbindungen der allgemeinen Formel (I) sind solche, in welchen
A - für Wasserstoff, Methyl, Ethyl, Tosyl oder für eine Aminoschutzgruppe, vorzugsweise aus der Reihe Benzyloxycarbonyl, 4-Methoxybenzyloxycarbonyl, 4-Nitrobenzyloxycarbonyl, 3,4,5-Trimethoxybenzyloxycarbonyl, Methoxycarbonyl, Ethoxycarbonyl, Propoxycarbonyl, Butoxycarbonyl, tert. -Butoxycarbonyl, 2-Bromethoxycarbonyl, 2-Chlorethoxycarbonyl, 2,2,2-Trichlorethoxycarbonyl, Allyloxycarbonyl, Phenoxycarbonyl, 4-Nitrophenoxycarbonyl, Fluorenyl-9-methoxycarbonyl, Acetyl, Pivaloyl, Isovaleroyl, Phthaloyl, 2,2,2-Trichloracetyl, 2,2,2-Trifluoracetyl, Benzyl, Tosyl, Benzoyl, 4-Nitrobenzoyl oder Phthalimido steht,
B - für eine direkte Bindung oder
   - für Sarcosyl (Sar), Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Ornityl (Orn), Phenylalanyl (Phe), Cystyl (Cys), Asparagyl (Asp), Glutamyl (Glu), Asparaginyl (Asn), Glutaminyl (Gln), Phenylglycyl (Phg), 4-Nitrophenylalanyl [Phe(4NO₂)], 3-Nitrophenylalanyl [Phe(3NO₂)], 2-Nitrophenylalanyl [Phe(2NO₂)], 2-, 3-oder 4-Aminophenylalanyl [Phe(2NH₂), Phe(3NH₂), Phe(4NH₂)7], 3,4-Dichlorphenylalanyl [Phe(3,4-Cl₂)], -4-Iodophenylalanyl [Phe(4I)], 4-Methoxyphenylalanyl [Phe(40CH₃)], 1-Triazoiylalanyl [Trz(1)], 2-Pyridylalanyl [Pyr(2)], 3-Pyridylalanyl [Pyr(3)], 4-Pyridylalanyl [Pyr(4)], 1-Naphthylalanyl [Nal(1)] oder 2-Naphthylalanyl [Nal(2)], gegebenenfalls mit Schutzgruppen, in ihrer L- Form oder D-Form, bevorzugt jedoch in ihrer L-Form, steht
D - für eine direkte Bindung oder
   - für D- oder L-Prolyl (Pro) steht,
E,G - jeweils gleich oder verschieden sind und
   - die gleiche Bedeutung wie B haben und mit diesem gleich oder verschieden sind,
   R¹- für einen Phenylrest steht, der in 2-, 3- oder 4-Stellung eine Nitrogruppe oder eine Aminogruppe der Formel 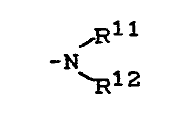 trägt, wobei
   R¹¹,R¹²- gleich oder verschieden sind und - Wasserstoff, Methyl, Ethyl, Acetyl bedeuten,
      oder
   R¹¹- für Wasserstoff und R¹² für eine Aminoschutzgruppe stehen,
   - oder für 2-Methyl-3-nitrophenyl, 4-Methyl-3-nitrophenyl, 6-Methyl-2-nitrophenyl, 3-Methyl-4-nitrophenyl oder 3,5-Dinitrophenyl steht,
J,L,M - jeweils gleich oder verschieden sind und die gleiche Bedeutung haben wie B und mit diesem gleich oder verschieden sind,
   und
Q - für einen Rest der Formel -OR¹³, -NH-NHR³, -_{NHR}¹⁴ oder 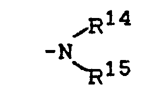 steht, worin
   R³ - für Wasserstoff, tert-Butoxycarbonyl, Benzyloxycarbonyl steht,
   _{R}^{13 -} für Wasserstoff, Benzyl oder C_{I}-C₆-Alkyl steht,
   R¹⁴ - für Wasserstoff, C₁-C₄-Alkyl, Benzyl, 1-Phenylethyl, 2-Phenylethyl, Adamantyl, Chinuclidyl, Pyridylmethyl oder 4-Benzylcyclohexylmethyl steht,
   R¹⁵ - für Cₗ-C₄-Alkyl steht
      oder
   _{R}¹⁴ und _{R}¹⁵ gemeinsam einen Ring wie 4-Benzylpiperidino, N-Benzyl-, N-Phenethylpiperazin oder N-Dimethylaminoethylpiperazin bilden

   und deren physiologisch unbedenklichen Salze.

Ganz besonders bevorzugte Verbindungen der allgemeinen Formel (I) 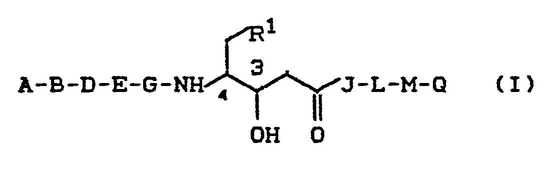 sind solche, in denen
A - für Wasserstoff, tert-Butoxycarbonyl, Benzyloxycarbonyl, Ethoxycarbonyl, Acetyl, Chloracetyl, Isovaleroyl, Benzoyl, Tosyl, Phenoxycarbonyl, Phthaloyl oder Phthalimido steht,
B - für eine direkte Bindung oder Phenylalanyl (Phe) steht,
D - für eine direkte Bindung oder Prolin (Pro) steht,
E - für eine direkte Bindung steht oder für Tyrosyl (Tyr), Phenylalanyl (Phe), 1-Naphthylalanyl [Nal(1)], 4-Nitrophenylalanyl [Phe(4NO₂)], 3,4-Dichlorphenylalanyl [Phe(3,4-Cl₂)], 4-Aminophenylalanyl [Phe(4NH₂)], 4-Iodophenylalanyl [Phe(4I)], 4-Methoxyphenylalanyl [Phe(40CH₃)] oder Phenylglycyl (Phg) steht,
G - für eine direkte Bindung oder - für Phenylalanyl (Phe), Histidyl (His), 3-Pyridylalanyl [Pyr(3)], 4-Aminophenylalanyl [Phe(4NH₂)] wobei die Aminogruppe gegebenenfalls durch Benzyloxycarbonyl (Z), tert-Butoxycarbonyl (Boc) oder Fluorenyl-9-methoxycarbonyl (Fmoc) geschützt ist, oder für 1-Triazolylalanyl [Trz(1)] steht, bevorzugt jeweils in der L-Form oder als D,L-Gemisch,
   R¹⁻ für 2-, 3- oder 4-Nitrophenyl, 2-, 3- oder 4-Aminophenyl steht
      wobei die Gruppe 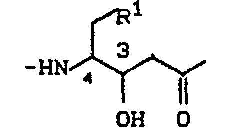 in der 3S,4S-Konfiguration, 3R,4S-Konfiguration sowie als 3RS-4S-Isomerengemisch vorliegt,
J - für eine direkte Bindung steht oder
   - für Leucyl (Leu) oder Isoleucyl (Ile) bevorzugt jeweils in der L-Form steht,
L - für eine direkte Bindung steht oder
   - für Phenylalanyl (Phe), Histidyl (His) oder 4-Nitrophenylalanyl [Phe(4NO₂)], bevorzugt jeweils in der L-Form steht,
M - für eine direkte Bindung steht
   und
Q - für Hydroxy, Methoxy, Ethoxy, n-Propoxy, Isopropoxy, tert.-Butoxy oder Benzyloxy steht, oder - für Amino, Benzylamino, 1-Phenylethylamino, 2-Phenylethylamino, α-Pyridylmethylamino, β-Pyridylmethylamino, γ-Pyridylmethylamino, 4-Benzylcyclohexylmethylamino, 4-Benzylpiperidino, 4-Benzylpiperazino, N-Dimethylaminoethylpiperazino, 1-Adamantylamino, 2-Adamantylamino oder 3-Chinuclidinamino steht

und deren physiologisch unbedenklichen Salze.

Insbesondere zu nennen sind folgende erfindungsgemäße Verbindungen als optische Antipoden oder Isomerengemische :

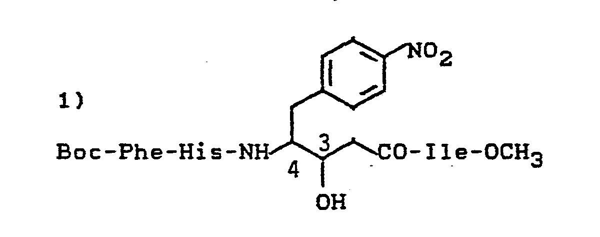

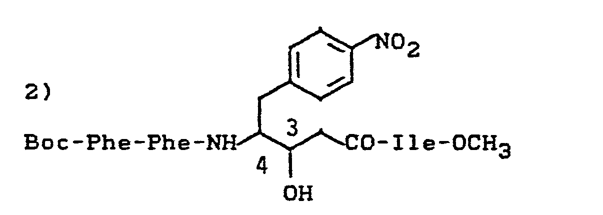

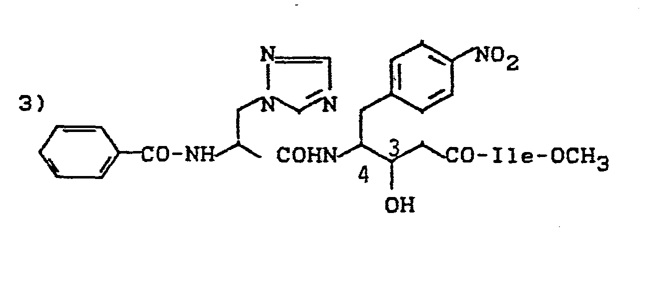

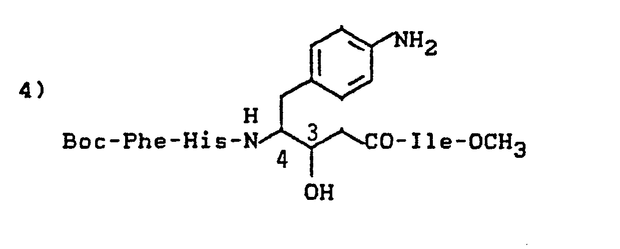

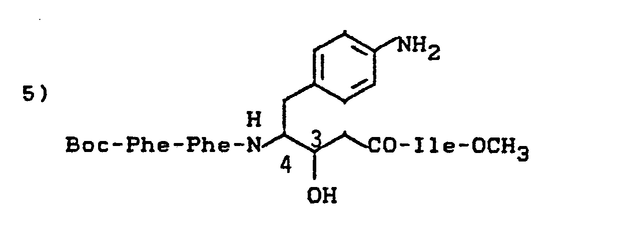

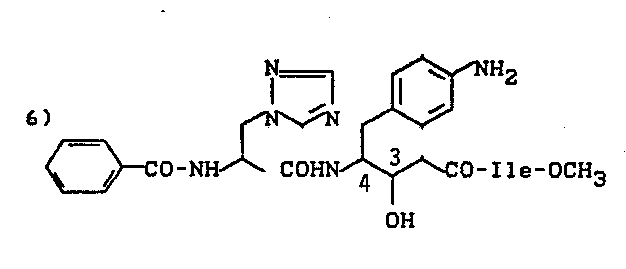

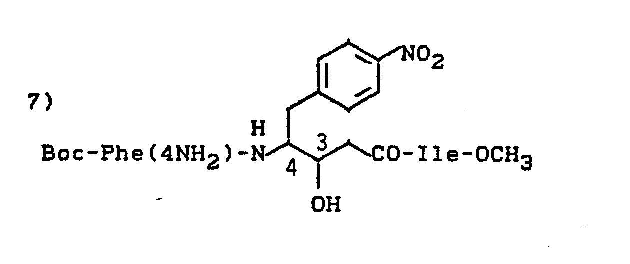

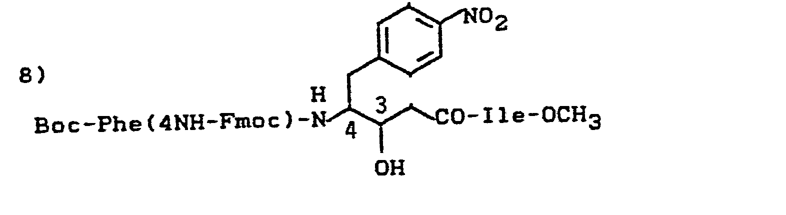

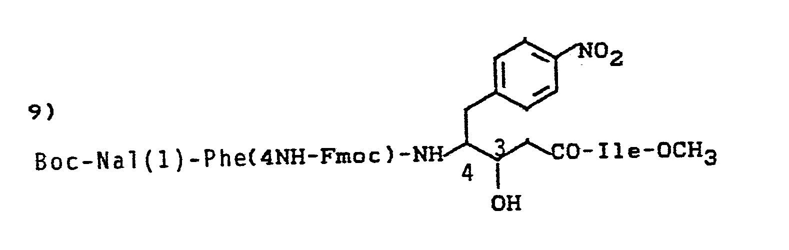

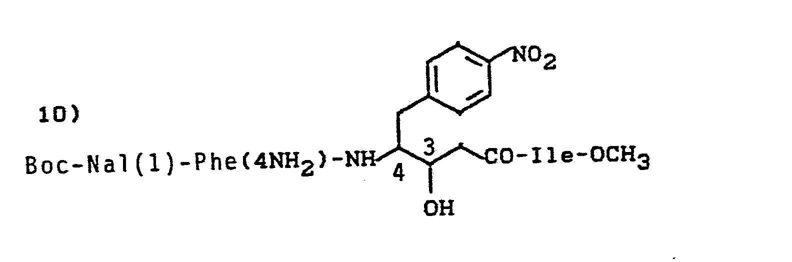

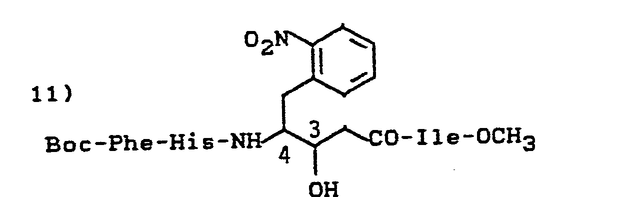

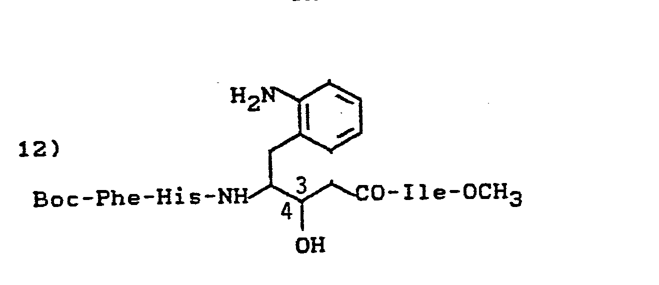

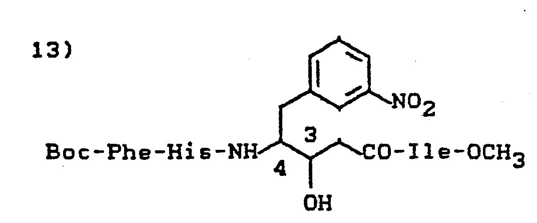

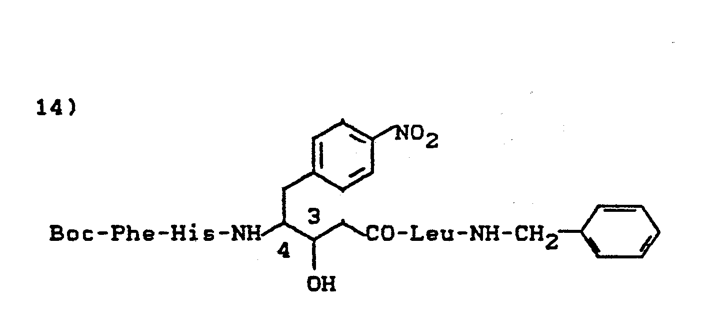

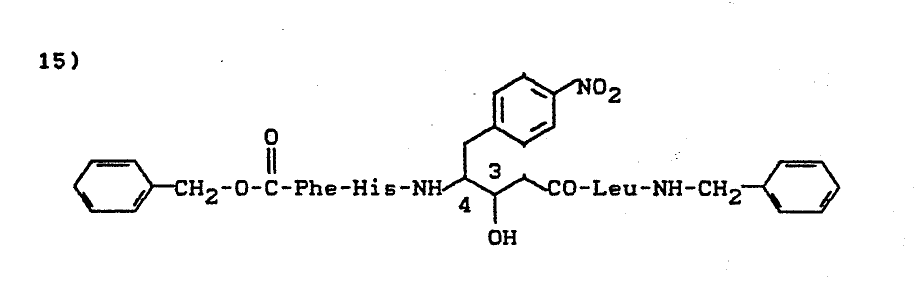

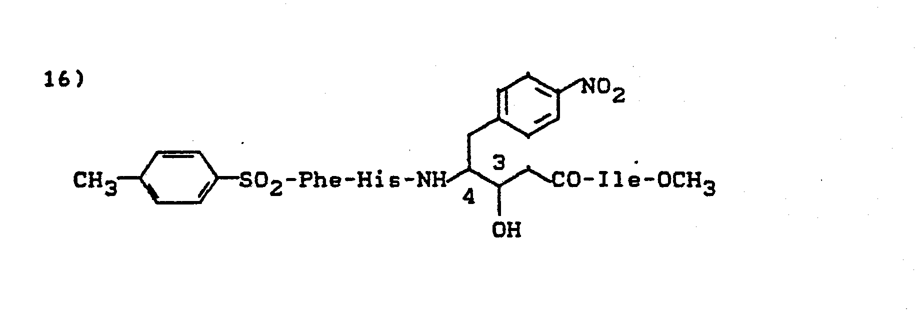

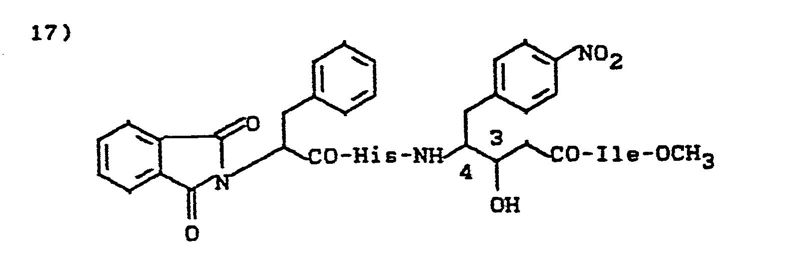

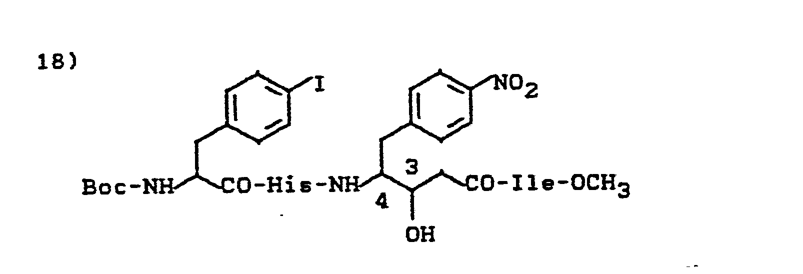

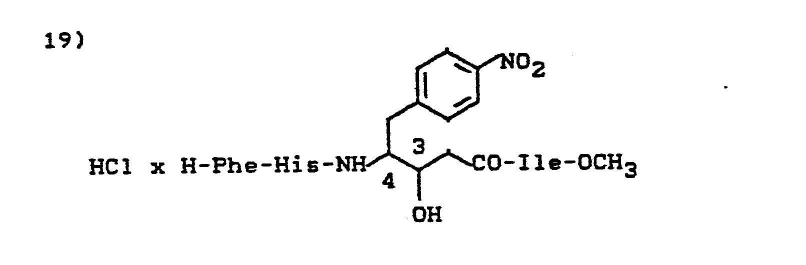

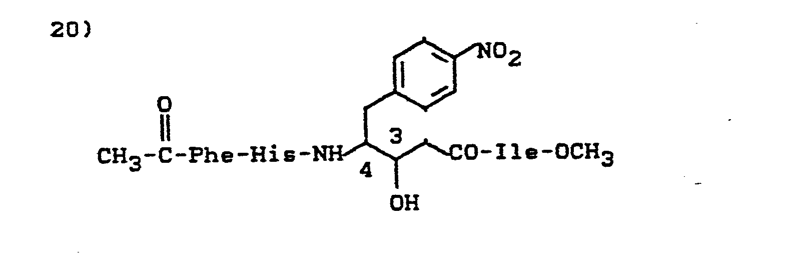

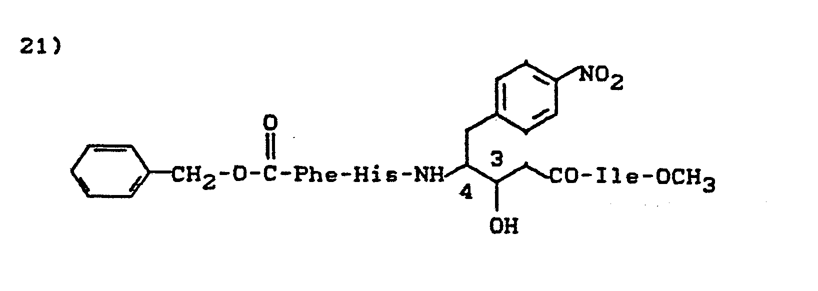

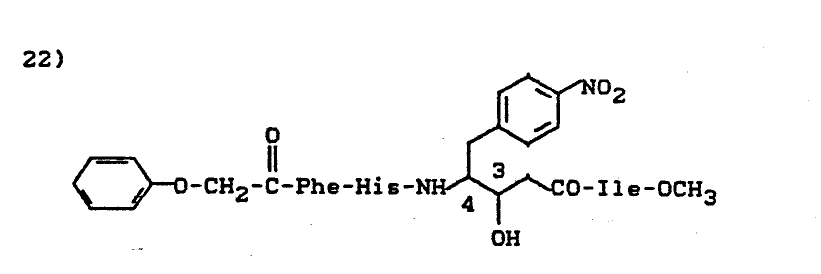

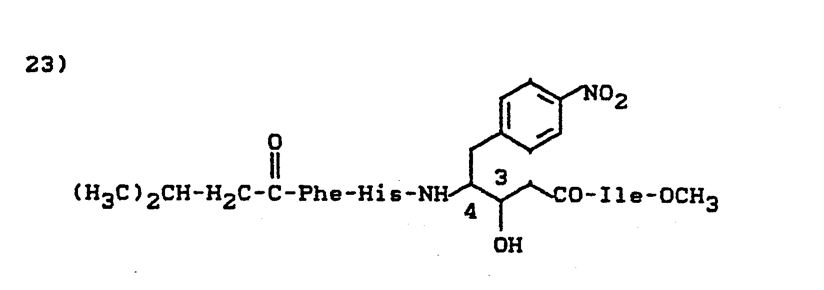

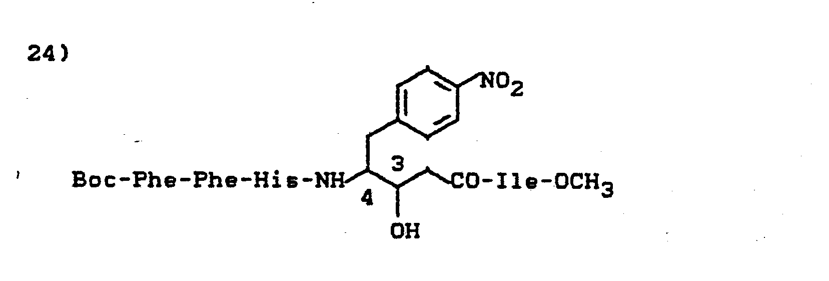

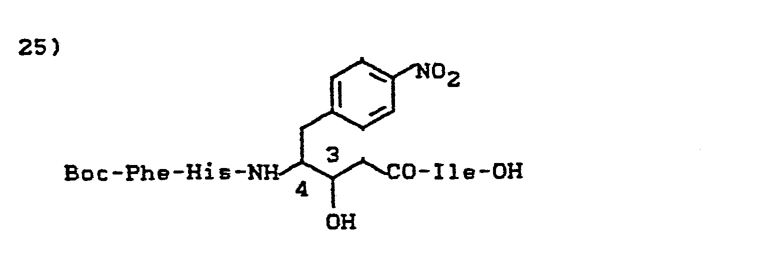

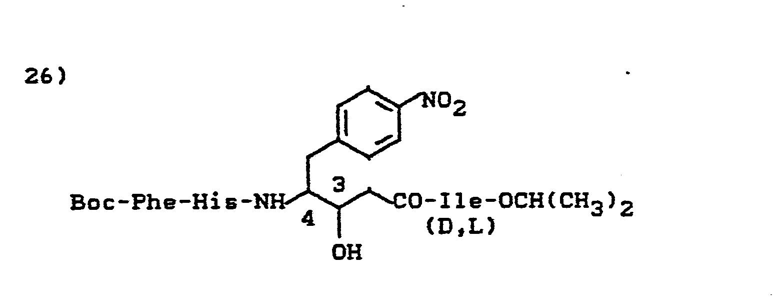

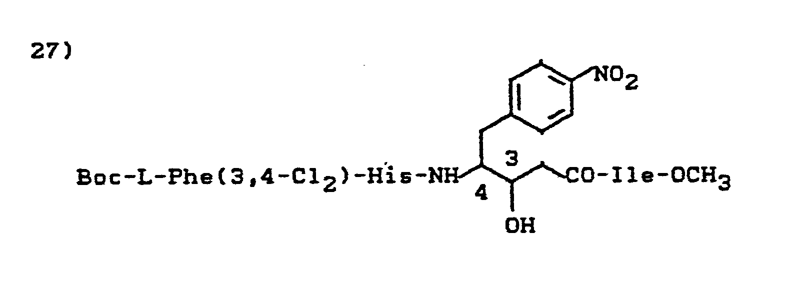

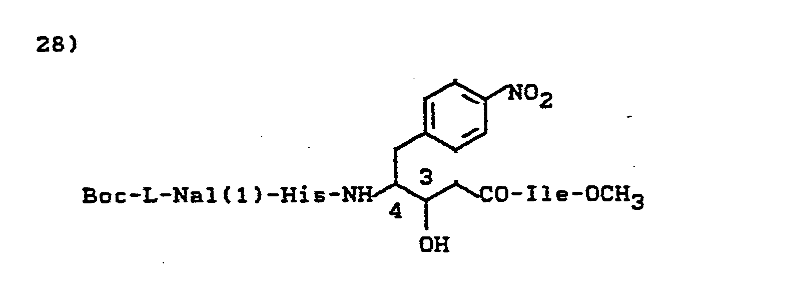

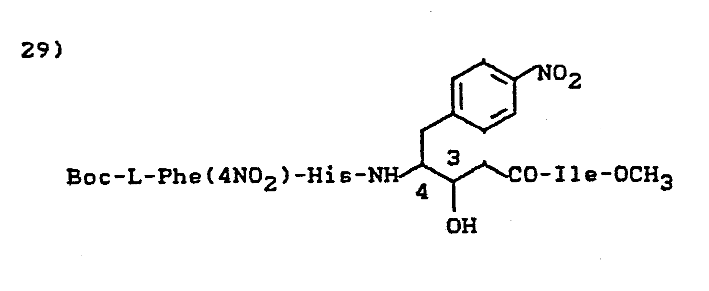

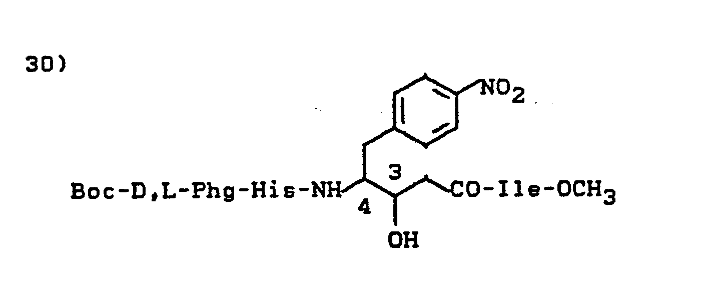

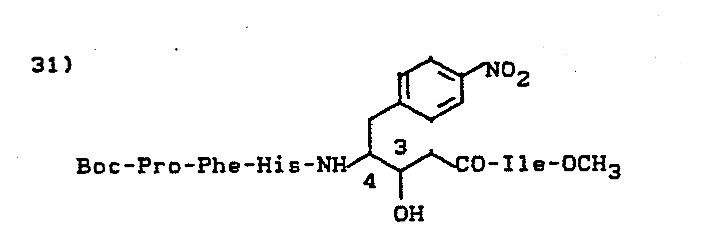

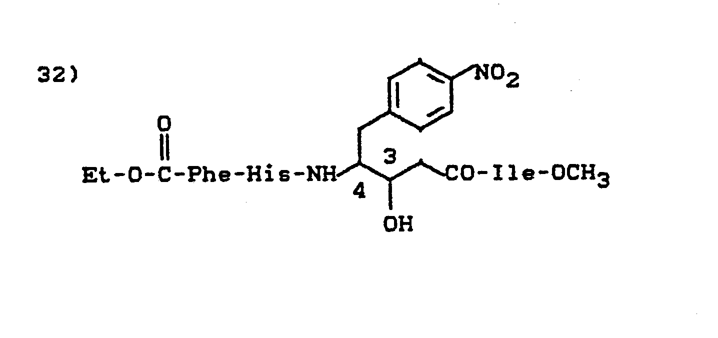

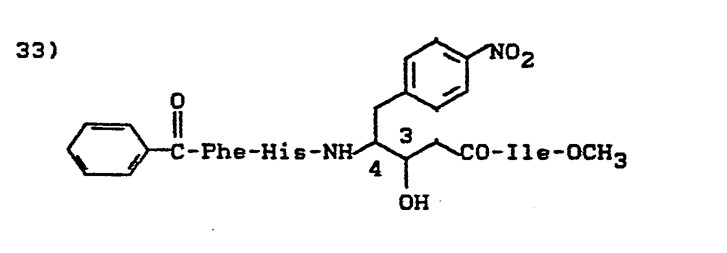

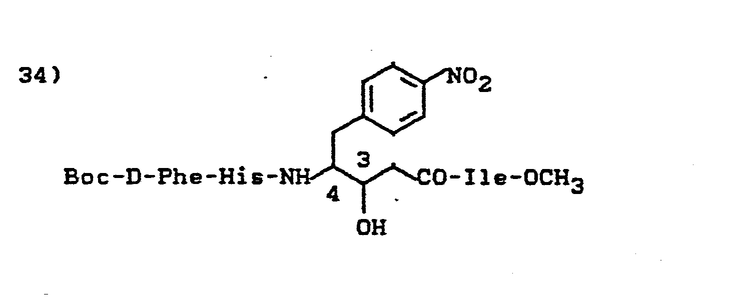

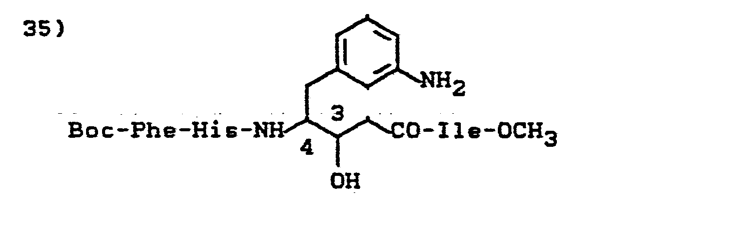

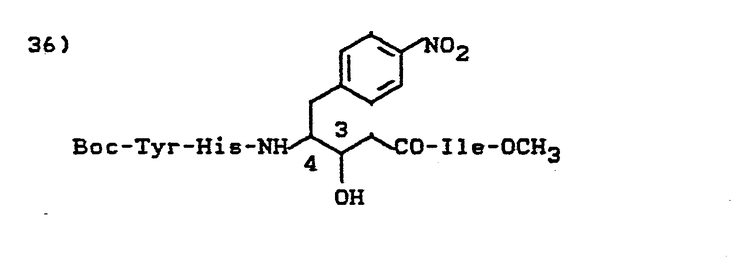

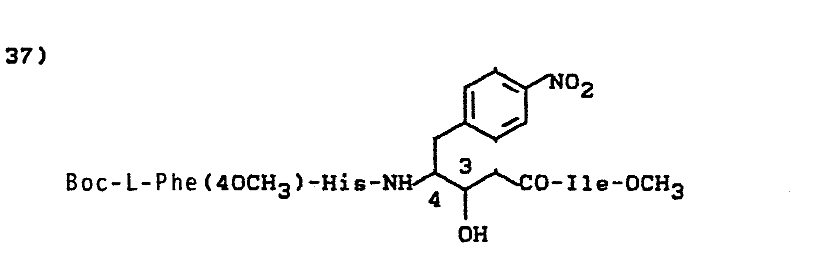

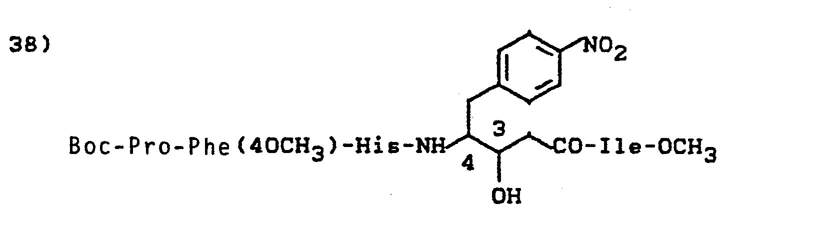

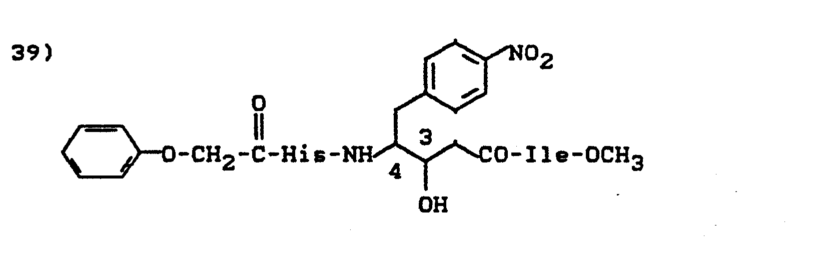

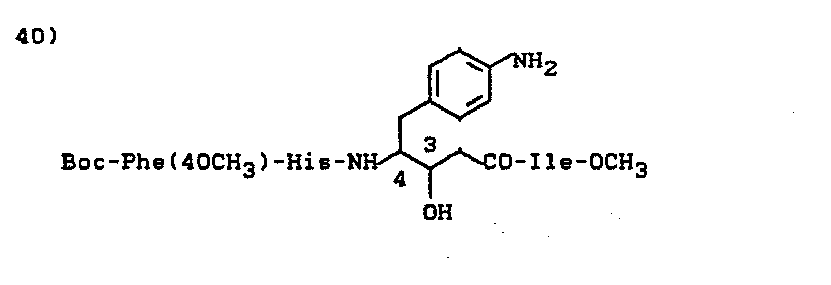

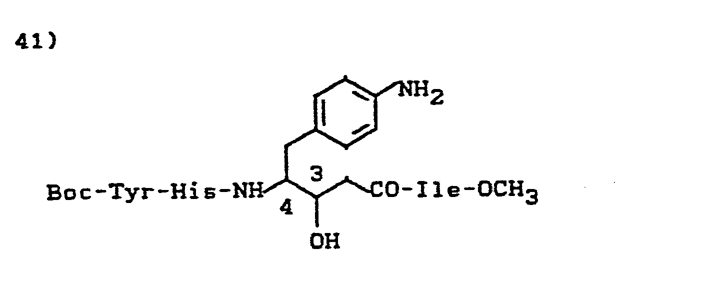

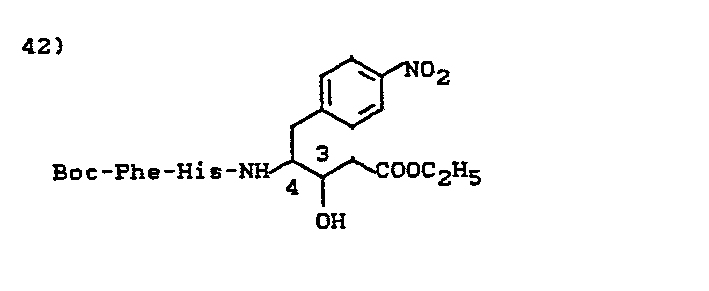

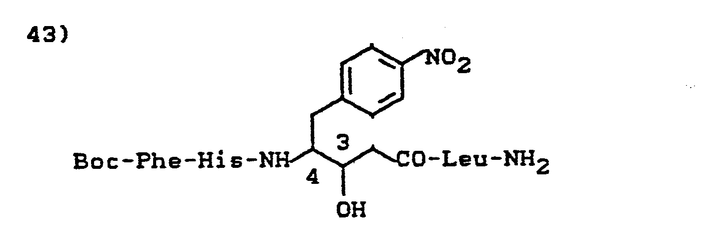

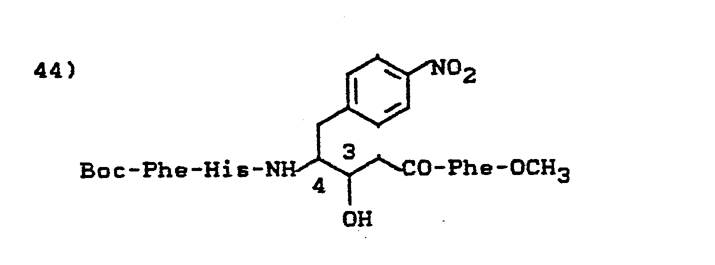

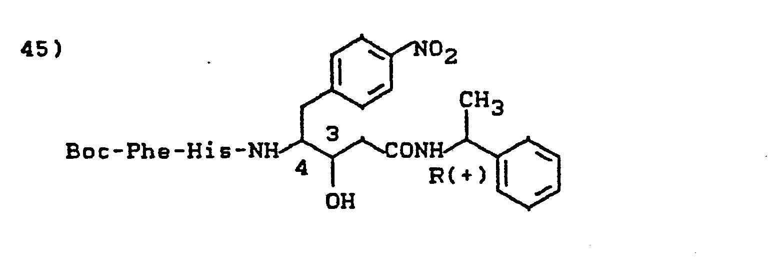

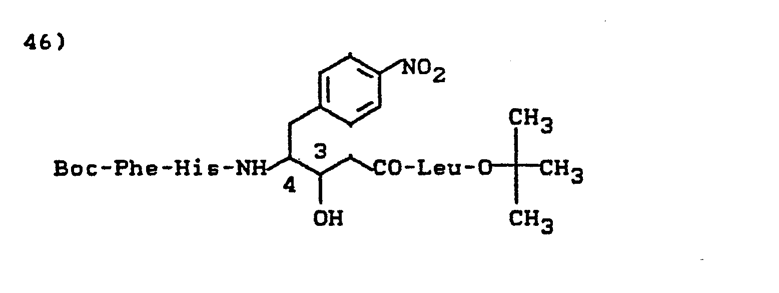

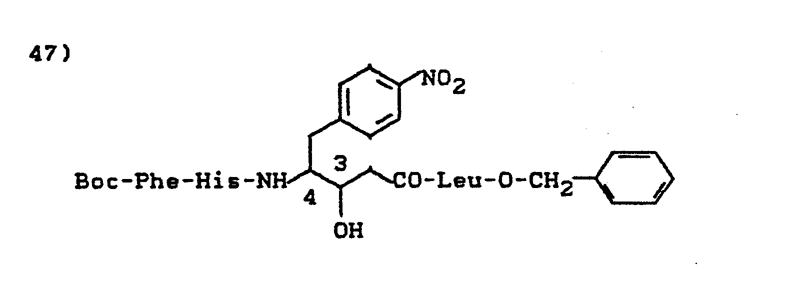

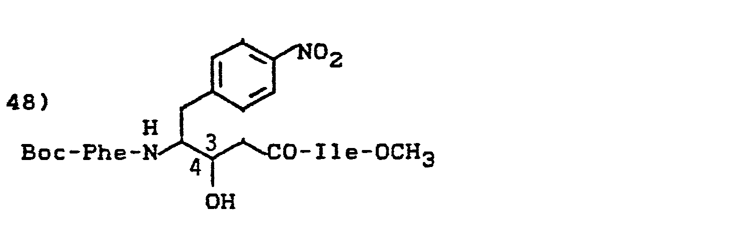

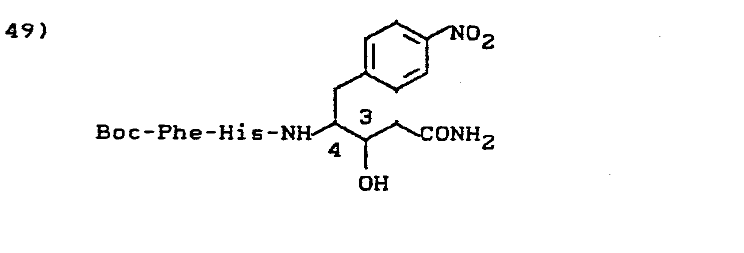

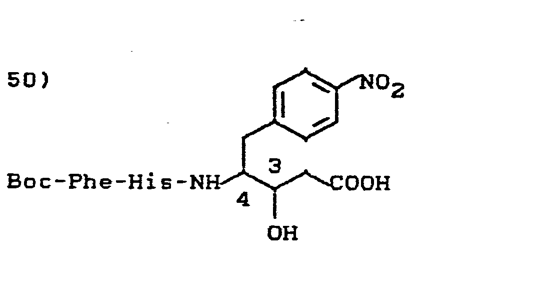

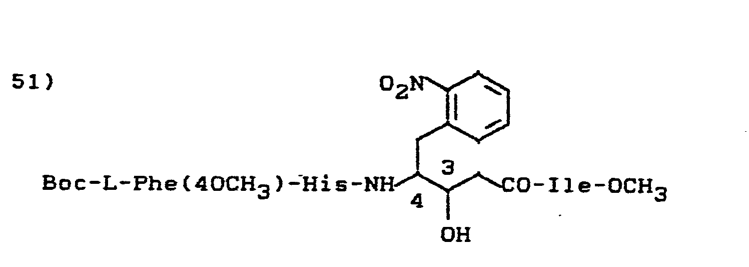

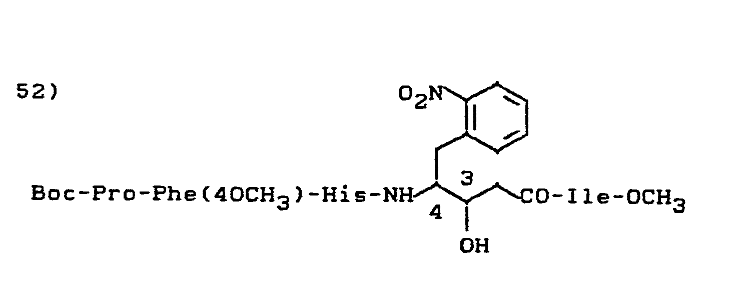

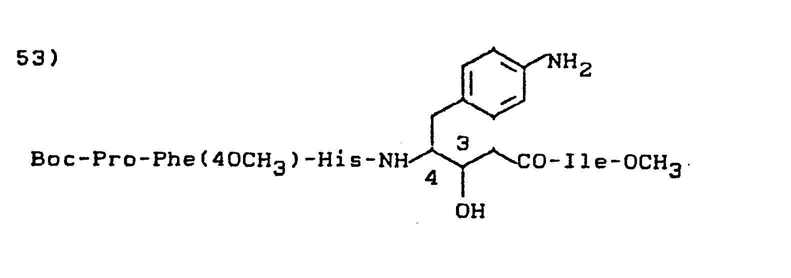

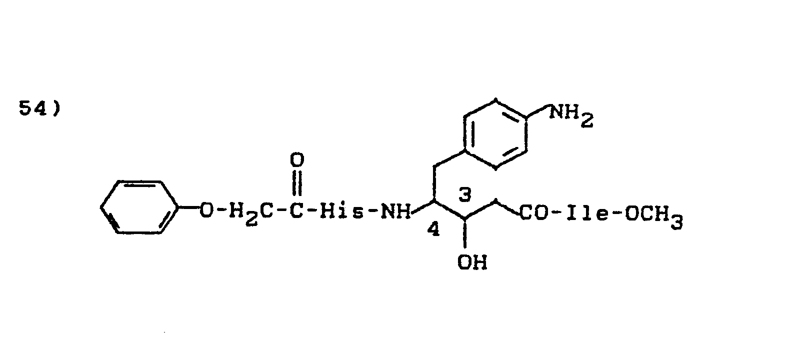

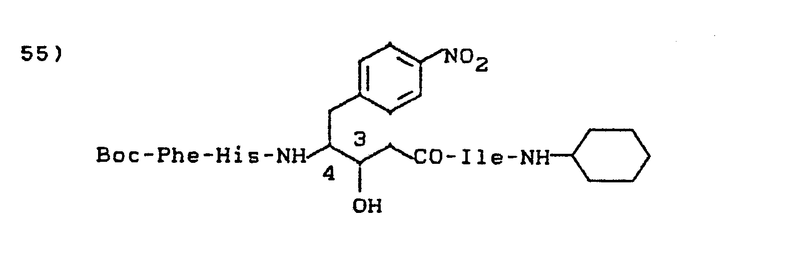

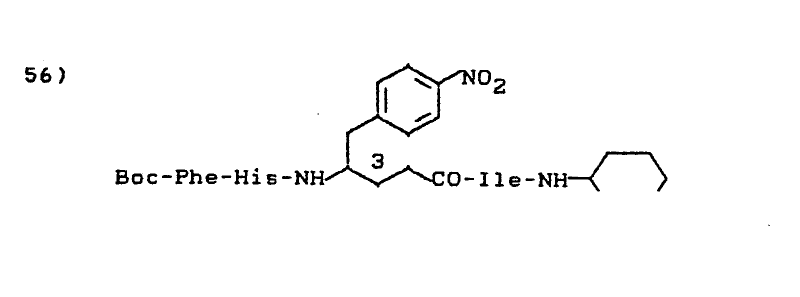

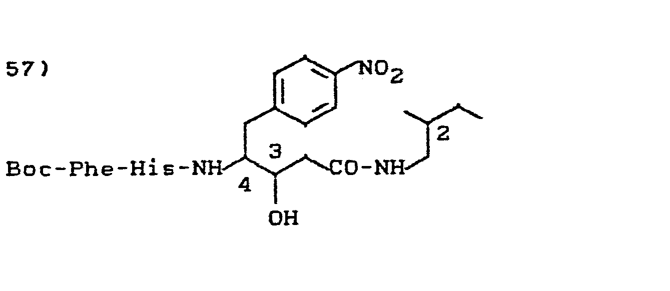

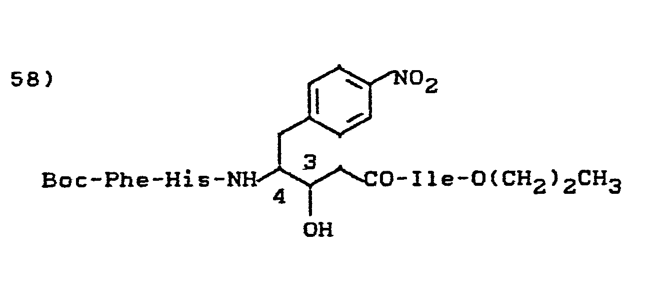

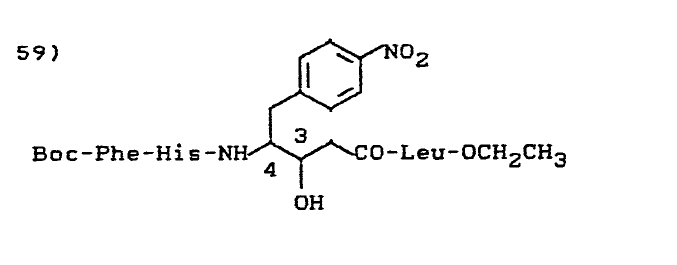

Die Erfindung betrifft auch ein Verfahren zur Herstellung der erfindungsgemäßen Peptide der allgemeinen Formel (I), bestehend aus einer bis acht Aminosäuren, dadurch gekennzeichnet, daß man
die Peptidbindungen der Verbindungen der Formel (I) durch Umsetzung eines entsprechenden Bruchstücks, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, herstellt, und diesen Vorgang gegebenenfalls so oft mit entsprechenden Bruchstücken wiederholt, bis man die gewünschten Peptide der allgemeinen Formel (I) hergestellt hat, anschließend gegebenenfalls Schutzgruppen abspaltet oder gegen andere Schutzgruppen austauscht.

Hierbei können zusätzliche reaktive Gruppen, wie z.B. Amino- oder Hydroxygruppen, in den Seitenketten der Bruchstücke gegebenenfalls durch übliche Schutzgruppen geschützt werden.

Durch das erfindungsgemäße Verfahren ist es möglich, a) die erfindungsgemäßen Peptide systematisch aus einzelnen Aminosäuren, vom C-Terminus (an den die Gruppe 0 angeknüpft ist) bis zum N-Terminus (an den die Gruppe A angeknüpft ist) oder umgekehrt schrittweise aufzubauen, oder
b) entsprechende Di-, Tri- oder Oligopeptidbausteine separat nach dem beschriebenen Verfahren zu synthetisieren und mit unterschiedlich großen Bausteinen in einem Schritt zu den gewünschten Verbindungen der Formel (I) zu kombinieren.

Aktivierte Carboxylgruppen sind hierbei bevorzugt:

Carbonsäureazide (erhältlich z.B. durch Umsetzung von geschützten oder ungeschützten Carbonsäurehydraziden mit salpetiger Säure, deren Salzen oder Alkylnitriten (z.B. Isoamylnitrit),
oder ungesättigte Ester, insbesondere Vinylester (erhältlich z.B. durch Umsetzung eines entsprechenden Esters mit Vinylacetat), Carbamoylvinylester (erhältlich z.B. durch Umsetzung einer entsprechenden Säure mit einem Isoxazoliumreagenz), Alkoxyvinylester (erhältlich z.B. durch Umsetzung der entsprechenden Säuren mit Alkoxyacetylenen, bevorzugt Ethoxyacetylen),
oder Amidinoester z.B. N,N'- bzw. N,N-disubstituierte Amidinoester (erhältlich z.B. durch Umsetzung der entsprechenden Säure mit einem N,N'-disubsituierten Carbodiimid (bevorzugt Dicyclohexylcarbodiimid, Diisopropylcarbodiimid oder N-(3-Dimethylaminopropyl)-N'-ethyl- carbodiimidhydrochlorid) oder mit einem N,N-disubstituierten Cyanamid), oder Arylester, insbesondere durch elektronenziehende Substituenten substituierte Phenylester, z.B. 4-Nitrophenyl-, 4-Methylsulfonylphenyl-, 2,4,5-Trichlorphenyl-, 2,3,4,5,6-Pentachlorphenyl-, 4-Phenyldiazophenylester (erhältlich z.B. durch Umsetzung der entsprechenden Säure mit einem entsprechend substituierten Phenol, gegebenenfalls in Anwesenheit eines Kondensationsmittels wie z.B. N,N'-Dicyclohexylcarbodiimid, Diisopropylcarbodiimid, N-(3-Dimethylaminopropyl)-N'-ethylcarbodiimid-Hydrochlorid, Isobutylchloroformat, Propanphosphonsäureanhydrid, Benzotriazolyloxytris(dimethyl- amino)phosphoniumhexafluorphosphat),
oder Cyanmethylester (erhältlich z.B. durch Umsetzung der entsprechenden Säure mit Chloracetonitril in Gegenwart einer Base),
oder Thioester, insbesondere Nitrophenylthioester (erhältlich z.B. durch Umsetzung der entsprechenden Säure mit Nitrothiophenolen, gegebenenfalls in Gegenwart von Kondensationsmitteln wie N,N'-Dicyclohexylcarbodiimid, Diisopropylcarbodiimid, N-(3-Dimethylaminopropyl)-N'- ethylcarbodiimid-Hydrochlorid, Isobutylchloroformat, Propanphosphonsäureanhydridt Benzotriazolyloxytris-(dimethylamino)phosphoniumhexafluorphosphat),
oder Amino- bzw. Amidoester (erhältlich z.B. durch Umsetzung der entsprechenden Säure mit einer N-Hydroxyamino- bzw. N-Hydroxyamido-Verbindung, insbesondere N-Hydroxy-succinimidₜ N-Hydroxypiperidin, N-Hydroxy-phthalimid, N-Hydroxy-5-norbornen-2,3-dicarbonsäureimid oder 1-Hydroxybenzotriazol, gegebenenfalls in Anwesenheit von Kondensationsmitteln wie N,N'-Dicyclohexylcarbodiimid, Diisopropylcarbodiimid oder N-(3-Dimethylaminopropyl)-N'-ethylcarbodiimid-Hydrochlorid, Isobutylchloroformat oder Propanphosphonsäureanhydrid),
oder Anhydride von Säuren, bevorzugt symmetrische oder unsymmetrische Anhydride der entsprechenden Säuren, insbesondere Anhydride mit anorganischen Säuren (erhältlich z.B. durch Umsetzung der entsprechenden Säure mit Thionylchlorid, Phosphorpentoxid oder Oxalylchlorid),
oder Anhydride mit Kohlensäurehalbderivaten z.B. Kohlensäureniederalkylhalbester (erhältlich z.B. durch Umsetzung der entsprechenden Säure mit Halogenameisensäureniedrigalkylestern, z.B.Chlorameisensäuremethylester, -ethylester, -propylester, -isopropylester, -butylester oder -isobutylester oder mit l-Niedrigalkoxycarbonyl-2- niedrigalkoxy-1,2-dihydro-chinolin, z.B. 1-Methoxycarbonyl-2-ethoxy-1,2-dihydrochinolin),
oder Anhydride mit Dihalogenphosphorsäuren (erhältlich z.B. durch Umsetzung der entsprechenden Säure mit Phosphoroxychlorid),
oder Anhydride mit Phosphorsäurederivaten oder Phosphorigsäurederivaten, (z.B. Propanphosphonsäureanhydrid, H. Wissmann und H.J. Kleiner, Angew. Chem. Int. Ed. 19, 133 (1980))
oder Anhydride mit organischen Carbonsäuren (erhältlich z.B. durch Umsetzung der entsprechenden Säuren mit einem gegebenenfalls substituierten Niederalkan- oder Phenylalkancarbonsäurehalogenid, insbesondere Phenylessigsäure, Pivalinsäure- oder Trifluoressigsäurechlorid),
oder Anhydride mit organischen Sulfonsäuren (erhältlich z.B. durch Umsetzung eines Alkalisalzes einer entsprechenden Säure mit einem Sulfonsäurehalogenid, insbesondere Methan-, Ethan-, Benzol- oder Toluolsulfonsäurechlorid),
oder symmetrische Anhydride (erhältlich z.B. durch Kondensation entsprechender Säuren, gegebenenfalls in Gegenwart von Kondensationsmitteln wie N,N'-Dicyclohexylcarbodiimid, Diisopropylcarbodiimid, N-(3-Dimethylaminopropyl)-N'-ethylcarbodiimid-Hydrochlorid, Isobutylchloroformat, Propanphosphonsäureanhydrid oder Benzotriazolyloxy-tris(dimethylamino)phosphonium- hexafluorphosphat.

Reaktionsfähige cyclische Amide sind insbesondere Amide mit fünfgliedrigen Heterocyclen mit 2 Stickstoffatomen und gegebenenfalls aromatischem Charakter, bevorzugt Amide mit Imidazolen oder Pyrazolen (erhältlich z.B. durch Umsetzung der entsprechenden Säuren mit N,N'-Carbonyldiimidazol oder - gegebenenfalls in Gegenwart von Kondensationsmitteln wie z.B. N,N'-Dicyclohexylcarbodiimid, N,N'-Diisopropylcarbodiimid, N-(3-Dimethylaminopropyl)-N'-ethylcarbodiimid-Hydrochlorid, Isobutylchloroformat, Propanphosphonsäureanhydrid, Benzotria- zolyloxy-tris(dimethylamino)phosphonium-hexafluorphosphat- mit z.B. 3,5-Dimethyl-pyrazol, 1,2,4-Triazol oder Tetrazol.

Ein komplementierendes Bruchstück mit freier Aminogruppe ist Ammoniak, ein primäres oder sekundäres Amin, oder eine Aminosäure oder ein Peptidrest, der nach den beschriebenen Methode A,B getrennt hergestellt wurde.

Die an der Reaktion teilnehmende Aminogruppe in einem komplementierenden Bruchstück einer erfindungsgemäßen Verbindung liegt bevorzugt in freier Form vor, insbesondere dann, wenn die damit zur Umsetzung gebrachte Carboxylgruppe in aktivierter Form eingesetzt wird. Sie kann jedoch ebenso selbst aktiviert sein. Eine solche reaktive Form ist beispielsweise ein Isocyanat oder ein Carbaminsäurehalogenid.

Ist das komplementierende Bruchstück mit einer freien Aminogruppe Ammoniak, ein mono- oder ein disubstituiertes Amin (bevorzugt beim Anknüpfen der Gruppe Q), so kann auch ein entsprechender Harnstoff eine reaktive Form dieses Amins sein.

Die Durchführung des erfindungsgemäßen Verfahrens erfolgt in an sich bekannter Weise, wobei die Reaktionsbedingungen entsprechend der Art der Carboxylgruppenaktivierung gewählt werden. Ublicherweise wird das Verfahren in Gegenwart geeigneter Löse- bzw. Verdünnungsmittel, gegebenenfalls in Anwesenheit eines Hilfsstoffes in einem Temperaturbereich von -8D°C bis 300°C, bevorzugt von -30°C bis 200°C bei normalem Druck durchgeführt. Ebenso ist es möglich, bei erhöhtem oder erniedrigtem Druck zu arbeiten.

Als Hilfsstoffe werden bevorzugt Kondensationsmittel eingesetzt, die auch Basen sein können, insbesondere wenn die Carboxylgrupppe als Anhydrid aktiviert vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert-Butyl-5-methyl-isoxazolium-perchlorat, oder Acylaminoverbindungen wie 2-Ethoxy-1-ethoxycarbonyl-1,2-dihydro- chinolin, oder Propanphosphonsäureanhydrid, oder Isobutylchloroformat, oder Benzotriazolyloxy-tris(dimethyl- amino)phosphonium-hexafluorophosphat, oder als Basen Alkalicarbonate z.B. Natrium-oder Kaliumcarbonat oder - hydrogencarbonat, oder organische Basen wie Trialkylamine z.B. Triethylamin, N-Ethylmorpholin oder N-Methylpiperidin eingesetzt.

Als Lösemittel eigenen sich die üblichen inerten Lösemittel, die sich unter den jeweils gewählten Reaktionsbedingungen, vor allem der jeweils gewählten Aktivierungmethode nicht verändern. Hierzu gehören bevorzugt Wasser oder organische Lösemittel wie Methanol, Ethanol, Propanol, Isopropanol, oder Ether wie Diethylether, Glykolmono- oder-dimethylether, Dioxan oder Tetrahydrofuran, oder Kohlenwasserstoffe wie Benzol, Toluol, Xylol, Cyclohexan oder Erdölfraktionen, oder Halogenkohlenwasserstoffe wie Methylenchlorid, Chloroform, Tetrachlorkohlenstoff, oder Aceton, Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden.

Salze der erfindungsgemäßen Verbindungen mit salzbildenden Gruppen können in an sich bekannter Weise hergestellt werden, zum Beispiel durch Umsetzung der erfindungsgemäßen Verbindungen die saure Gruppen enthalten, mit entsprechenden Basen oder durch Umsetzung der erfindungsgemäßen Verbindungen die basische Gruppen enthalten mit entsprechenden Säuren, jeweils bevorzugt mit den vorne genannten Basen bzw. Säuren.

Stereoisomerengemische, insbesondere Diastereomerengemische, können in an sich bekannter Weise, z.B. durch fraktionierte Kristallisation oder Chromatographie in die einzelnen Isomeren aufgetrennt werden.

Racemate können in an sich bekannter Weise, z.B. durch Überführung der optischen Antipoden in Diastereomere gespalten werden.

Die Herstellung der erfindungsgemäßen Verbindungen ist auch nach weiteren üblichen Varianten des beschriebenen Verfahrens durchgeführt worden (vgl. z.B. Houben-Weyls "Methoden der organischen Chemie" XV!1 und 2; M. Bodanszky, A. Bodanszky in "The Practice of Peptide Synthesis", Springer Verlag, Berlin, 1984; George R. Pettit in "Synthetic Peptides", Volume 4, Elsevier Scientific Publishing Company, Amsterdam-Oxford-New York, 1976; E. Gross und J. Meienhofer (Editors) in "The Peptides", Volt 1-3, Academic Press, New York-London-Toronto-Sydney-San Francisco, 1981; M. Bodanszky in "Principles of Peptide Synthesis", Springer Verlag, Berlin-Heidelberg-New York-Tokyo, 1984; R. Uhmann und K. Radscheit, Offenlegungsschrift, DE 3 411 244 A1)

oder auch nach der "Solid-Phase-Methode", wie sie beispielsweise von M. Bodanszky, A. Bodanszky in "The Practice of Peptide Synthesis", Springer-Verlag, Berlin, 1984, oder G. Barany, R.B. Merrifield in "Solid-Phase Peptide Synthesis" aus "The Peptides", Volt 2, S: 3-254, edited by E. Gross, J. Meienhofer, Academic Press, New York-London-Toronto-Sydney-San Francisco (1980) beschrieben wird.

Die als Ausgangsstoffe eingesetzten Aminosäuren in der Definition von B, D, G, J, L, M sind bekannt oder können nach bekannten Methoden erhalten werden bzw. sind natürliche Aminosäuren [Houben-Weyls "Methoden der organische Chemie" Band XV/1 und 2). Die Aminosäuren DL-, D- bzw. L-(1-Triazolyl-) alanin in geschützter und ungeschützter Form sind neu. Sie werden hergestellt aus N-Acetyldehydroalaninethylester [H. Hellmann, H. Teichmann und F. Ringens, Chem. Ber. 91, 2427-2431 (1958)], Triazol und Natrium, wie von Wieland in Chem. Ber. 90, 194 (1957) beschrieben.

Die als Ausgangsstoffe sowohl in ihrer Amino-geschützten Form als auch als Ester eingesetzten Aminosäuren der Formel (II) 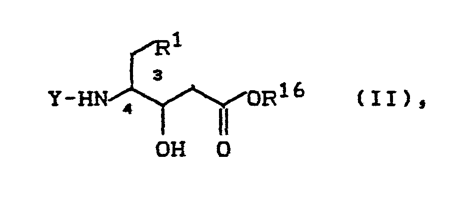 in welcher
R¹- für eine Gruppe der Formel 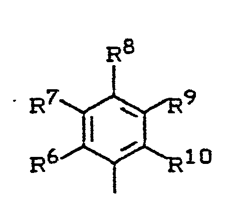 steht, worin
   R⁶,R⁷,R⁸,R⁹ und R^{10 -} gleich oder verschieden sind und
      - für Wasserstoff, C₁-C₆-Alkyl, C₁-C₆-Alkoxy, Halogen, Hydroxy, Cyano, Trifluormethyl, Trifluormethoxy, Trifluormethylthio, Nitro oder für eine Gruppe der Formel 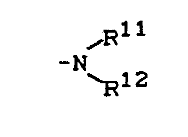 stehen, wobei
   R¹¹,R¹² - gleich oder verschieden sind und - für Wasserstoff, C₁-C₆-Alkyl, Aryl, Aralkyl, Phenylsulfonyl, Tolylsulfonyl, Cₗ-C₆-Alkylsulfonyl, Acetyl, Benzoyl oder für eine Aminoschutzgruppe stehen,

   mit der Maßgabe, daß mindestens einer der Substituenten R⁶,R⁷,R^{S},R⁹ oder R¹⁰ für _{N}iₜᵣₒ - oder die Gruppe NR¹¹R¹² stehen _{muß},
R¹⁶ für Wasserstoff oder für geradkettiges oder verzweig tes gegebenenfalls durch ein Sauerstoffatom in der Kette unterbrochenes C_{I}-C₁₂-Alkyl steht
   und
Y - für Wasserstoff, C₇-C₁₄-Aralkyl, C₁-C₈-Alkyl, Phenylsulfonyl, Tolylsulfonyl, C₁-C₈-Alkylsulfonyl oder für eine Aminoschutzgruppe steht,

sind neu und können hergestellt werden,
indem man

Aldehyde der allgemeinen Formel (III) 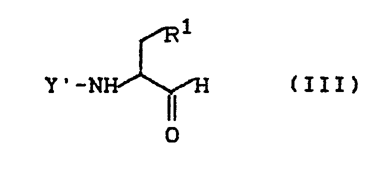 in welcher
R^{l-} die angegebene Bedeutung hat und
Y'- die für Y angegebene Bedeutung hat jedoch nicht für Wasserstoff steht,
in inerten organischen Lösemitteln, gegebenenfalls in Anwesenheit einer Base mit Essigsäureestern der allgemeinen Formel (IV) 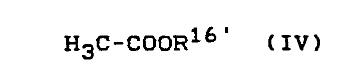 in welcher
_{R}'⁶'- für geradkettiges oder verzweigtes gegebenenfalls durch ein Sauerstoffatom in der Kette unterbrochenes Alkyl mit bis zu 12 Kohlenstoffatomen, bevorzugt mit bis zu 6 Kohlenstoffatomen, steht,
umsetzt,
das anfallende Stereoisomerengemisch gegebenfalls nach üblichen Methoden trennt und gegebenfalls anschließend Schutzgrupppen abspaltet.

Als Lösemittel können die üblichen organischen Lösemittel verwendet werden, die sich unter den Reaktionsbedingungen nicht verändern. Hierzu gehören bevorzugt Alkohole wie Methanol, Ethanol, Propanol, Isopropanol, oder Ether wie Diethylether, Dioxan, Tetrahydrofuran, Glykolmono- oder -diethylether, oder Kohlenwasserstoffe wie Benzol, Toluol, Xylol oder Erdölfraktionen, oder Dimethylformamid oder Hexamethylphosphorsäuretriamid. Ebenso ist es möglich, Gemische der genannten Lösemittel einzusetzen.

Als Basen eignen sich die üblichen anorganischen oder organischen Basen. Hierzu gehören bevorzugt Alkalimetalle wie z.B. Natrium, oder Alkalihydride wie z.B. Natriumhydrid oder Kaliumhydrid, oder Alkalialkoholate wie z.B. Natriummethanolat oder -ethanolat, oder Kaliummethanolat oder -ethanolat, oder Kalium-tert-butylat, oder Alkaliamide wie z.B. Natriumamid oder Lithiumdiisopropylamid, Lithiumhexamethyldisilazid, oder metallorganische Verbindungen wie z.B. n-, sec- oder tert-Butyllithium, oder Phenyllithium, oder Alkalihydroxide wie Natrium-oder Kaliumhydroxid.

Die Umsetzung kann bei Normaldruck, aber auch bei erhöhtem oder erniedrigtem Druck durchgeführt werden. Im allgemeinen arbeitet man bei Normaldruck.

Die Umsetzung wird in einem Temperaturbereich von -100°C bis 50° C, bevorzugt von -80°C bis 20°C durchgeführt.

Die Trennung des anfallenden Isomerengemisches erfolgt nach üblicher Methode wie es beispielsweise bei E.L. Eliel in "Stereochemistry of Carbon Compounds", McGraw Hill (1962) beschrieben ist, bevorzugt erfolgt die Trennung auf chromatographischem Weg, wie es beispielsweise von W.C. Still et al. in J. Org. Chem. 43, 2923 (1978) beschrieben wird.

Das Abspalten der Schutzgruppen erfolgt gegebenenfalls nach bekannten Methoden, je nach Art der vorhandenen Schutzgruppen, wie es beispielsweise in Houben-Weyls "Methoden der organischen Chemie" Band XV/1 und 2 beschrieben ist.

Bei der Umsetzung wird im allgemeinen der Essigester in einer Menge von 0,5 bis 5, bevorzugt von 1 bis 2 Mol bezogen auf ein Mol des Aldehyds eingesetzt, und die Base in einer Menge von 1 bis 5, bevorzugt 1 bis 1,6 Mol bezogen auf 1 Mol des Essigesters eingesetzt.

Die Herstellung der Aminosäuren der Formel (II) kann durch folgendes Schema verdeutlicht werden:

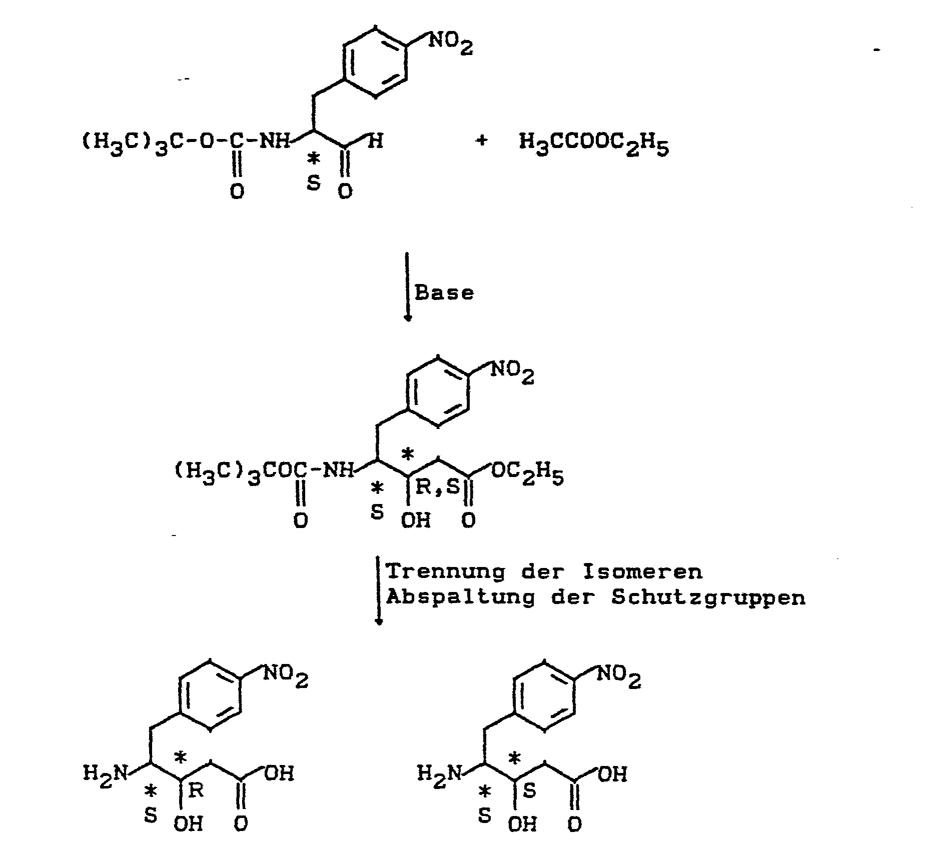

Bevorzugt wird bei der Herstellung der erfindungsgemäßen Aminosäuren der Formel (II) als Ausgangsprodukte die stereochemisch reinen 2S- oder 2R-Formen, besonders bevorzugt die 2S-Formen der Aldehyde (III) eingesetzt.

Die als Ausgangsstoffe eingesetzten Aldehyde der allgemeinen Formel (III) sind neu.

Sie werden hergestellt, indem man
[A] Alkohole der allgemeinen Formel (IV) 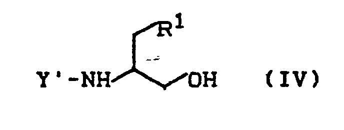 in welcher
   Y' und R¹ die angegebene Bedeutung haben

   gegebenenfalls in Anwesenheit einer Base und/oder eines Hilfsstoffs in inerten organischen Lösemitteln oxidiert,
   oder indem man

[B] Aminosäurederivate der allgemeinen Formel (V) 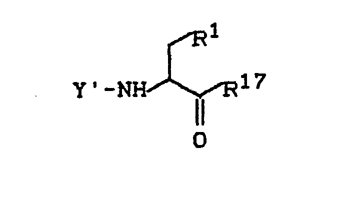 in welcher
   R¹ und Y' die angegebene Bedeutung haben
      und
   R¹⁷- für Hydroxy, für C₁-C₆-Alkoxy, für Di-C₁-C₄- alkylamino, 1-Imidazolyl oder für N-Methoxy-N-methylamino steht,

   gegebenenfalls in Anwesenheit eines Hilfsmittels reduziert.

Die erfindungsgemäßen Verfahren lassen sich beispielsweise durch folgendes Schema verdeutlichen:

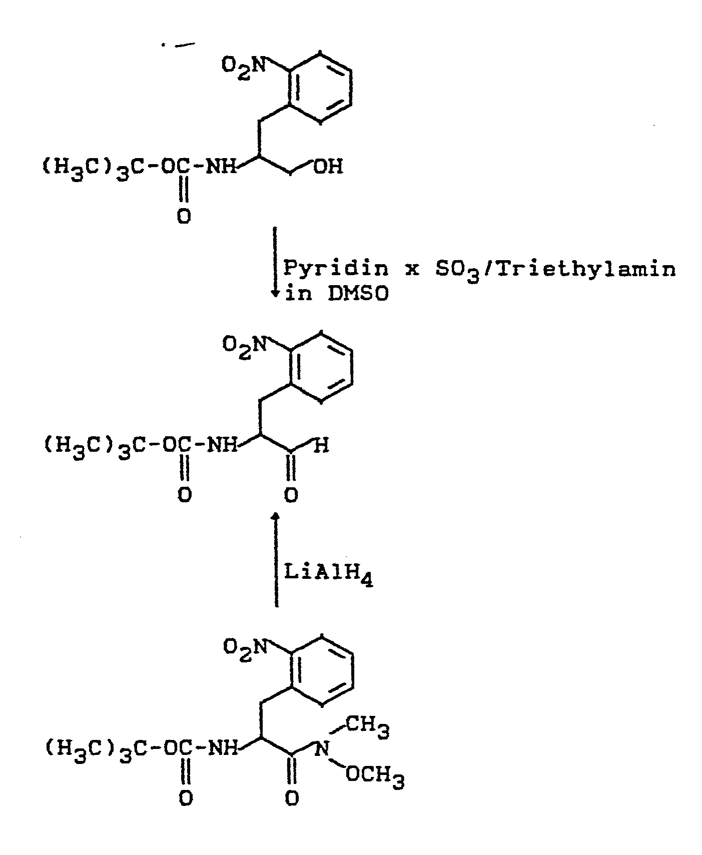

Die Oxidation in Verfahren A kann nach üblichen in der Literatur beschriebenen Methoden durchgeführt werden. Hierzu gehören beispielsweise:
die Oxidation mit Chrom-(VI)-Verbindungen, bevorzugt mit Pyridinium-Chlorochromat, Pyridiniumdichromat oder Pyridin/Cr0₃ in Halogenkohlenwasserstoffen wie Methylenchlorid oder Chloroform, oder Kohlenwasserstoffen wie Benzol, Toluol oder Hexan, oder Ethern wie Diethylether, Dioxan oder Tetrahydrofuran, bei Temperaturen von -80°C bis 100°C, bevorzugt -30°C bis 50°C wie von C.F. Stanfield, J.E. Parker, P. Kanellis in J. Org. Chem. 46, 4797 (1981) oder von E.J. Corey, G. Schmidt in Tetrahedron Letters 1979, 399 oder in US-Patentschrift 4.397.786 oder in Houben-Weyls "Methoden der organischen Chemie" Ergänzungsband 3, S. 287 ff. beschrieben, ferner die Oxidation mit Pyridin/S0₃ oder bevorzugt Pyridin/S0₃/Triethylamin in Halogenkohlenwasserstoffen wie Methylenchlorid oder Chloroform, oder Kohlenwasserstoffen wie Benzol, Toluol oder Xylol, oder Ethern wie Diethylether, Dioxan oder Tetrahydrofuran oder Hexamethylphosphorsäuretriamid oder bevorzugt Dimethylsulfoxid wie von J.R. Parika und W.E. von Doering in J. Am. Chem. Soc. 89, 5505 (1967) oder Y. Hamada, T. Shioiri in Chem. Pharm. Bull 30, 1921 (1982) beschrieben oder
die Oxidation mit Dimethylsulfoxid in Halogenkohlenwasserstoffen wie Methylenchlorid oder Chloroform, oder Ethern wie Diethylether, Dioxan oder Tetrahydrofuran, oder Dimethylsulfoxid im Überschuß, gegebenenfalls in Anwesenheit von Dicyclohexylcarbodiimid, N-(3-Dimethylaminopropyl)-N'-ethylcarbodiimid-Hydrochlorid, Trifluoracetanhydrid, Oxalylchlorid oder Acetanhydrid, gegebenenfalls in Gegenwart von Basen wie Triethylamin bei Temperaturen von -80°C bis 100°C, bevorzugt von -30°C bis 50°C wie in Houben-Weyls "Methoden der organischen Chemie" Ergänzungsband 3 Seite 275 ff beschrieben wird.

Die Reduktion der Carbonsäurederivate nach Verfahren B kann nach üblichen, in der Literatur beschriebenen Verfahren durchgeführt werden. Hierzu gehören bevorzugt die Reduktion mit Hydriden wie Lithiumaluminiumhydrid, Diisobutylaluminiumhydrid, BH₃, Diboran, Natriumborhydrid, Natriumcyanoborhydrid, Tributylzinnhydrid, Triethylsilan oder Dimethylphenylsilan in inerten Lösemitteln wie Ethern z.B. Diethylether, Dioxan oder Tetrahydrofuran, oder Halogenkohlenwasserstoffen wie Methylenchlorid oder Chloroform, oder Kohlenwasserstoffen wie Benzol, Toluol oder Xylol, gegebenenfalls in Anwesenheit von Säuren wie Essigsäure, Dichloressigsäure, Trifluoressigsäure oder Methan-, Benzol- oder Toluolsulfonsäure, in einem Temperaturbereich von -80°C bis 100°C, bevorzugt von -30°C bis 50°C, oder die Reduktion mit Wasserstoff, gegebenenfalls mit Katalysatoren wie Raney-Nickel, Palladium, Palladium/Tierkohle oder Platin, gegebenenfalls in Anwesenheit von Säuren wie Salzsäure oder Essigsäure, Chloressigsäure, Dichloressigsäure, Trifluoressigsäure, Methan-, Benzol- oder Toluolsulfonsäure, in inerten Lösungsmitteln wie Alkohlen z.B. Methanol, Ethanol, Propanol, Isopropanol oder Ethern wie Diethylether, Dioxan,

Tetrahydrofuran, oder Kohlenwasserstoffen wie Benzol, Toluol oder Xylol, oder Essigsäure oder Trifluoressigsäure, mit erhöhtem oder erniedrigtem Wasserstoffdruck (von 0,5 bis 100 bar) bei Temperaturen von -80°C bis 100°C, bevorzugt von -30°C bis 50°C wie beispielsweise von E. Adams in J. Biol. Chem. 217, 317 (1955) oder von K. Balemovic et al., in J. Org. Chem. 18, 297 (1953) bzw. 21, 115 (1956) oder von W. Ried und P. Paender in Justus Liebigs Ann. Chem. 640, 111 (1961) oder von H. Umezawa et al., in J. Antibiotics (Tokyo) 25, 515 (1972) oder von H. Seki, K. Koga und S. Yomada in Chem. Pharm. Bull. 23, 3081 (1975), von D.H. Rich, E.T. Sun, H.S. Bopacci in J. Org. Chem. 43, 3624 (1978) oder von R. Streulmann, K. Klostermeyer in Liebigs Ann. Chem. 1975. 2245, von K.E. Rittle, C.F. Hommiek, G. S. Penticello, B.E. Evans in J. Org. Chem. 47, 3016 (1982) oder in US-Patentschrift 4.397.786 beschrieben wird.

Die als Ausgangsstoffe eingesetzten Alkohole (IV) bzw. Aminosäurederivate (V) sind teilweise bekannt oder können nach bekannten Methoden hergestellt werden [C. Freeman et al, J. org. Chem. 1981, 46, 4797-4798:
a) Fehrentz, J.A. et al (1983) Synthesis, 676-678,
b) Ito, A et al., Chem. Pharm. Bull, 23, 3081-3087 (1975)].

### In vitro Test

Die inhibitorische Stärke der erfindungsgemäßen Peptide gegen endogenes Renin vom Humanplasma wird in vitro bestimmt. Gepooltes Humanplasma wird unter Zusatz von Ethylendiamintetraessigsäure (EDTA) als Antikoagulanz erhalten und bei -20°C gelagert. Die Plasmareninaktivität (PRA) wird als Bildungsrate von Angiotensin I aus endogenem Angiotensinogen und Renin nach Inkubation bei 37°C bestimmt. Die Reaktionslösung enthält 150 µl Plasma, 3 µl 6,6Xige 8-Hydroxychinolinsulfatlösung, 3 µl 10%ige Dimercaprollösung und 144 gl Natriumphosphatpuffer (0,2 M; 0,1% EDTA; pH 5,6) mit oder ohne den erfindungsgemäßen Stoffen in verschiedenen Konzentrationen. Das pro Zeiteinheit gebildete Angiotensin I wird mit einem Radioimmunoassay (Sorin Biomedica, Italien) bestimmt. Die prozentuale Inhibition der Plasmareninaktivität wird berechnet durch Vergleich der hier gebildeten Menge an Angiotensin I mit oder ohne die erfindungsgemäßen Stoffe.

Der Konzentrationsbereich, in dem die erfindungsgemäßen Stoffe eine 50Xige Inhibition der Plasmareninaktivität zeigen, liegt zwischen 10 ⁴ bis 10⁻⁹ M.

### In vivo Test

Es war nach dem derzeitigen Stand der Technik nicht zu erwarten, daß die erfindungsgemäßen Verbidungen oral blutdrucksenkend wirksam sind. Im Rahmen der hier vorliegenden Erfindung ist es überraschend, daß die erfindungsgemäßen Peptide, wie z.B. die erfindungsgemäße Verbindung des Beispiels 12 sowohl in vitro die Reninaktivität hemmen als auch oral blutdrucksenkend sind. Die unerwartete antihypertensive Wirkung wird beispielhaft nach oraler Gabe einer Dosis von 100 mg/kg des Beispiels 12 an wachen, spontan hypertensiven Ratten beobachet. [siehe Tabelle 1].

Dabei wird der Blutdruck der wachen Tiere mit der üblichen indirekten Meßmethode am Schwanz, unter Verwendung einer aufblasbaren Okklusionsmanschette und eines Infratonpulsabnehmers gemessen [vgl, H. Breuninger: Methoden zur unblutigen Messung des Blutdrucks an Kleintieren, Arzneimittelforschung 6, 222-225 (1956)]. Die Substanz wird als Suspension in 1%iger Tylose mittels Schlundsonde appliziert. Der systolische Blutdruck wird vor und 1, 2, 4 und 6 Stunden nach Gabe gemessen.

Auch bei Inactin^{®} narkotisierten, natriumdepletierten spontan hypertensiven Ratten (250 mg/l Furosemid im Trinkwaser über 14 Tage) senkt die Verbindung nach intragastraler Gabe den arteriellen Mitteldruck,

### Tabelle 1

Antihypertensive Wirkung von Beispiel 12 bei spontan hypertensiven Ratten
Einfluß einer oralen Dosis von 100 mg/kg auf den systolischen Blutdruck (mmHg).

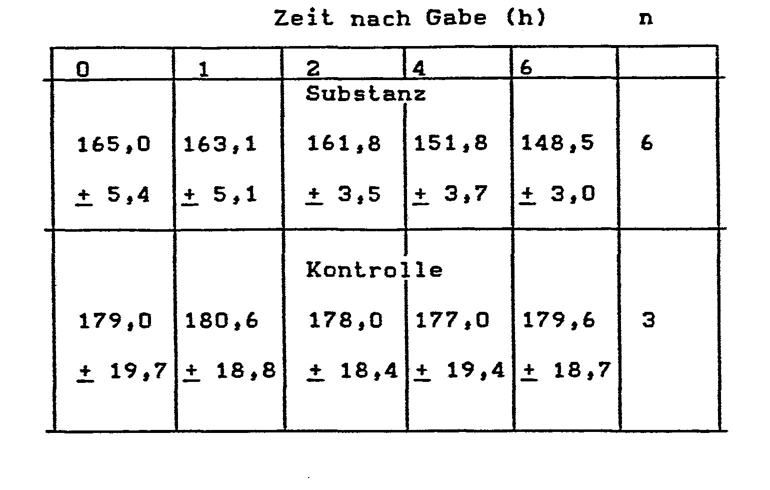

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln undloder Trägerstoffen, gegebenenfalls unter Verwendung von Emulgiermitteln undloder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. ErdnußlSesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-FettalkoholEther (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,1 bis 5 mg/kg Körpergewicht zur Erzielung wirksamer Ergebnisse zu verabreichen, und bei oraler Applikation beträgt die Dosierung etwa 0,1 bis 200 mg/kg, vorzugsweise 1 bis 100 mg/kg Körpergewicht. Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen

Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Erklärungen zum Experimentellen Teil:

### DC-Svsteme:

### Stationäre Phase

Merck DC-Fertigplatten Kieselgel 60 F-254, 5 x 10 cm, Schichtdicke 0,25 mm, Art.-Nr. 5719.

Mobile Phasen (im Test als "DC-System")

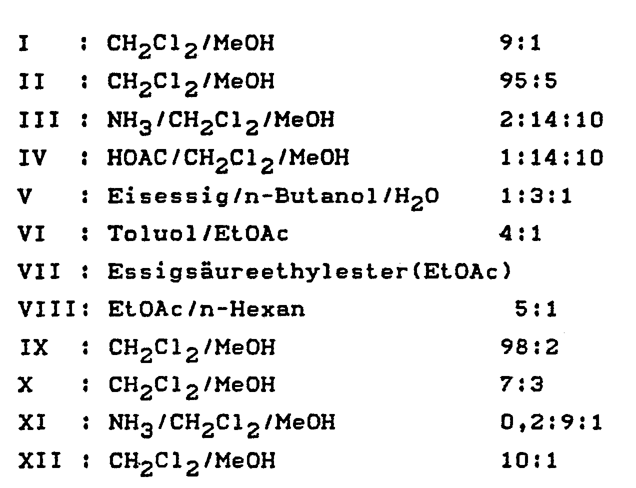

### HPLC-Systeme:

HPLC-System I : Säule Merck LiChrosorb® RP-8, 250-4, 10 µm, Kat.-No. 50318
HPLC-System II: Säule Merck LiChrosorb® RP-18, 250-4, 10 µm, Kat.-No. 50334
Eluens bei System 1 und II
A: pH 7,00 Phosphatpuffer, Merck, Art.-Nr. 9439/H₂O 1:50
B: Acetonitril
A/B wie 1/1, Fluß: 2 ml/min, isokratisch,
Detektion: 254 nm

### Verzeichnis der benutzten Abkürzungen

### 1. allgemeine analytische Methoden

DC Dünnschichtchromatographie
PDC präparative Dickschichtchromatographie
GC Gaschromatographie
HPLC Hochdruckflüssigkeitschromtographie
SC Säulenchromatographie
NMR Kernspinresonanzspektroskopie (Protonen)
MS Massenspektrometrie (Elektronenstoßionisation)
(+)FAB-MS Fast-atomic-bombardement-Massenspektrometrie, positive Ionen, Matrixsubstanz: m-Nitrobenzylalkohol
MS-DCI Massenspektrometrie, chemische Ionisation

### 2. Aminosäuren

Im allgemeinen erfolgt die Bezeichnung der Konfiguration durch das Vorausstellen eines L bzw. D vor der Aminosäureabkürzung, im Fall des Racemats durch ein D,L-wobei zur Vereinfachung bei L-Aminosäuren die Konfigurationsbezeichnung unterbleiben kann und dann nur im Fall der D-Form bzw. des D,L-Gemisches explizierte Bezeichnung erfolgt.

### a) natürliche Aminosäuren

Ala L-Alanin
Arg L-Arginin
Asn L-Asparagin
Asp L-Asparaginsäure
Cys L-Cystein
Gln L-Gluthamin
Glu L-Gluthaminsäure
Gly L-Glycin
His L-Histidin
Ile L-Isoleucin
Leu L-Leucin
Lys L-Lysin
Met L-Methionin
Orn L-Ornithin
Phe L-Phenylalanin
Ser L-Serin
Sar L-Sarcosin (N-Methylglycin)
Thr L-Threonin
Trp L-Tryptophan
Tyr L-Tyrosin
Val L-Valin

### b) unnatürliche Aminosäuren

D- oder L-Nal(1) β-(1-Naphthyl)-D- oder -L-alanin
D- oder L-Nal(2) β-(2-Naphthyl)-D- oder -L-alanin
D- oder L-Phe(2N0₂) ß-(2-Nitrophenyl)-D- oder -L-alanin
D- oder L-Phe(3N0₂) β-(3-Nitrophenyl)-D- oder -L-alanin
D- oder L-Phe(4N0₂) ß-(4-Nitrophenyl)-D- oder -L-alanin
D- oder L-Phe(2NH₂) β-(2-Aminophenyl)-D- oder -L-alanin
D- oder L-Phe(3NH₂) ß-(3-Aminophenyl)-D- oder -L-alanin
D- oder L-Phe(4NH₂) ß-(4-Aminophenyl)-D- oder -L-alanin
D- oder L-Phe(3,4-Cl₂) ß-(3,4-Dichlorphenyl)-D- oder -L-alanin
D- oder L-Phg D- oder L-Phenylglycin
D- oder L-Pyr(2) ß-(2-Pyridyl)-D- oder -L-alanin
D- oder L-Pyr(3) β-(3-Pyridyl)-D- oder -L-alanin
D- oder L-Pyr(4) ß-(4-Pyridyl)-D- oder -L-alanin
D- oder L-Trz(1) β-(1-Triazolyl)-D- oder -L-alanin
D- oder L-Phe (4I) ß-(4-Iodophenyl)-D- oder -L-alanin
D- oder L-Phe(40CH₃) β-(4-Methaxyphenyl)-D- oder -L-alanin

### 3. Aktivierunasaruppen

HOBT 1-Hydroxybenzotriazol
HOSU N-Hydroxysuccinimid

### 4. Kupplungsreagenzien

DCC Dicyclohexylcarbodiimid
DPPA Diphenylphosphorylazid
PPA n-Propanphosphonsäureanhydrid
BOP Benzotriazolyloxy-tris(dimethylamino)phos- phoniumhexafluorphosphat

### 5. Reagentien

NEM N-Ethylmorpholin
NMM N-Methylmorpholin
TEA Triethylamin
TFA Trifluoressigsäure

### 6. Lösemittel

HOAc Essigsäure
DMF Dimethylformamid
EtOAc Essigsäureethylester
MeOH Methanol
EtOH Ethanol
THF Tetrahydrofuran
DMSO Dimethylsulfoxid
HMPT Hexamethylphosphorsäuretriamid

### 7. Schutzgruppen

Boc Tert-Butoxycarbonyl
Z Benzyloxycarbonyl
DNP Dinitrophenyl
Fmoc 9-Fluorenylmethoxycarbonyl
OEt Ethylester
OMe Methylester

### 8. Sonstige

DCU N,N'-Dicyclohexylharnstoff

### Herstellunasbeispiele

### Beispiel 1

### L-(4-Nitrophenyl)alanin

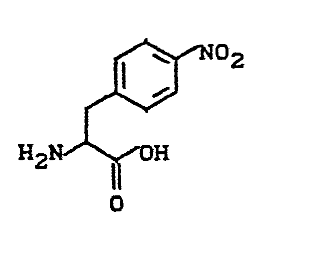

250 g (1,5 mol) L-Phenylalanin werden unter Eiskühlung in 750 ml konzentrierter Schwefelsäure gelöst. Man tropft unter Trockeneis/Aceton-Kühlung 146,8 ml 65%ige Salpetersäure so zu, daß eine Temperatur von 5°C nicht überschritten wird. Es wird noch 15 Minuten nachgerührt, auf 1 kg Wassereis gegossen und 500 g festes Natriumhydroxid vorsichtig und in kleinen Portionen zu der Reaktionsmischung gegeben. Mit etwa 1 1 konzentrierter Ammoniaklösung (25%) wird auf pH 7 gestellt. Man saugt das ausgefallene Rohprodukt nach dem Erkalten ab und kristallisiert aus 7,5 1 Wasser um.

Ausbeute: 188,8 g (73,4% der Theorie)

Fp.: 245° C

¹H-NMR (NaOD, 200 MHz): δ = 8,2 (d, 2H); 7,6 (d, 2H); 3,6 (m, 1H); 3,1 (m, 2H).

DC-System III: R_{f} = 0,71
IV: R_{f} = 0,42

### Beispiel 2

### L-(4-Nitrophenyl)alanin-methylester-Hydrochlorid

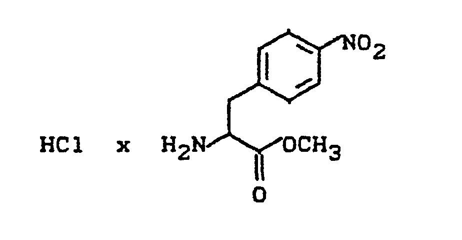

21 ml Thionylchlorid werden bei -5°C zu 160 ml Methanol p.a. getropft. Anschließend trägt man bei einer Temperatur von 5°C 105,1 g (0,5 mol) L-(4-Nitrophenyl)alanin in Portionen ein und läßt über Nacht am Rückfluß rühren. Man gibt nocheinmal 100 ml Methanol p.a. dazu, kühlt auf -5°C ab und tropft weitere 23 ml Thionylchlorid hinzu. Man rührt weitere 4 Stunden am Rückfluß und engt am Rotationsverdampfer zur Trockene ein. Das Rohprodukt wird aus 600 ml Methanol umkristallisiert, abgesaugt und mit Diethylether nachgewaschen.

Ausbeute: 93 g (71,7% der Theorie)

Fp.: 232°C Zers.

¹H-NMR (D₂0, 200 MHz): δ = 8,3 (d, 2H); 7,6 (d, 2H);. 4,6 (m, 1H); 3,9 (s, 3H); 3,5 (m, 2H)

DC-System II: R_{f} = 0,54
IV: R_{f} = 0,93

### Beispiel 3

### N-tert-Butoxycarbonyl-L-(4-nitrophenyl)alanin-methylester

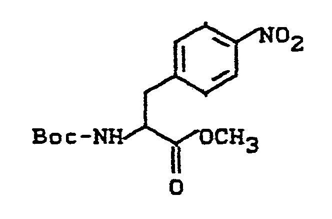

81,4 g (0,31 mol) L-(4-Nitropheny)alanin-methylester--Hydrochlorid werden in 630 ml wasserfreiem Dioxan gelöst, unter Eiskühlung tropfenweise zuerst mit 43,7 ml Triethylamin und dann mit 76,0 g Pyrokohlensäure-ditert-butylester versetzt. Man läßt 2 h bei Raumtemperatur rühren, stellt den pH mit Triethylamin auf 8,5 nach und läßt über Nacht bei Raumtemperatur weiterreagieren. Die Reaktionsmischung wird mit verdünnter Salzsäure vorsichtig unter Kühlung auf pH 3 gebracht und das Rohprodukt durch Zugabe von Wasser ausgefällt. Nach dem Trocknen über P₂O₅ kristallisiert man aus Tetrahydrofuran/n-Hexan um.

Ausbeute: 75,9 g (74,6X der Theorie)

Fp.: 94-96°C

¹H-NMR (CDCl₃, ₂₀₀ MHZ): δ= ₈,₁₅ (_{d}, _{2H}); ₇,₃ (_{d}, _{2H}); 5,1 (d, 1H); 4,65 (m, 1H); 3,75 (s, 3H); 3,2 (m, 2H); 1,4 (s, 9H).

### Beispiel 4

### N-tert-Butoxycarbonyl-L-(4-nitrophenyl)alaninol

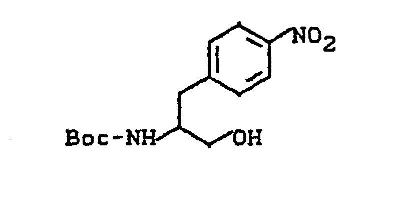

40 g (0,12 mol)N-tert-Butoxycarbonyl-L-(4-nitrophenyl)alanin-methylester werden in 400 ml THF p.a. gelöst, 9,4 g (200 mol-X) NaBH₄ dazugegeben und die - Mischung 15 Minuten unter leichtem Rückfluß (50-55°C) gerührt. Dann werden bei dieser Temperatur 99 ml Methanol p.a. binnen 1 Stunde vorsichtig zugetropft. Man läßt über Nacht bei Raumtemperatur rühren, gibt 247 ml Wasser hinzu und zieht die Lösungsmittel am Rotationsverdampfer ab. Zum Rückstand werden 400 ml einer gesättigten Natriumchloridlösung gegeben und das Rohprodukt 3 mal mit je 600 ml Ether extrahiert. Die vereinigten organischen Phasen werden mit gesättigter Natriumchloridlösung gewaschen, mit MgS0₄ getrocknet und im Vakuum zur Trockene eingeengt.

Ausbeute: 35,5 g (97,4% der Theorie)

Fp.: 132°C.

¹H-NMR (CDC1₃, 300 MHz): δ= 8,15 (d, 2H); 7,40 (d, 2H); 4,85 (d, 1H); 3,9 (s, breit, 1H); 3,62 (m, 2H); 2,97 (m, 2H); 2,29 (m, 1H); 1,30 (s, 9H).

DC-System II: R_{f} = 0,51

### Beispiel 5

### N-tert-Butoxycarbonyl-L-(4-nitrophenyl)alaninal

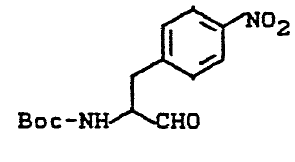

### Variante A:

Zu einer Lösung von 20 g (67,5 mmol) N-tert-Butoxycarbonyl-L-(p-nitrophenyl)alaninol und 28,4 ml Triethylamin in 210 ml Dimethylsulfoxid wird bei Raumtemperatur eine Lösung von 32,3 ml Pyridiniumsulfat in 210 ml Dimethylsulfoxid getropft. Man läßt 15 Minuten nachrühren, gießt auf 800 ml Eiswasser und extrahiert 3 mal mit je 500 ml Diethylether. Die vereinigten organischen Phasen werden nacheinander mit 2 mal mit 10%iger Citronensäure, 2 mal mit Wasser und 2 mal mit gesättigter Natriumhydrogencarbonatlösung gewaschen, sodann mit Magnesiumsulfat getrocknet, am Rotationsverdampfer bei einer Badtemperatur vom max. 30°C eingeengt und am Hochvakuum getrocknet (Öl, färbt gelb mit 2,4-Dinitrophenylhydrazin).

Ausbeute: 18.8 g (94,7X der Theorie)

¹H-NMR (CDC1₃, 200 MHz): δ= 9,66 (s, 1H); 8,18 (m, 2H); 7,37 (d, 2H); 5,13 (d, 1H); 4,45 (m, 1H); 3,25 (m, 2H); 1,40 (s, 9H).

DC-System II: R_{f} = 0,46

MS (DCI): m/z 295 (M+1), m/z 312 (M+NH₄⁺)

### Variante B:

Eine auf 0°C gekühlte Lösung von 19 g (54 mmol) N-tert-Butoxycarbonyl-L-(4-nitrophenyl)analyl-(N'-methoxy)me- thylamid (Beispiel 62) in 250 ml Tetrahydrofuran wird mit 2,55 g (125 mol-X) gepulvertem Lithiumaluminiumhydrid versetzt. Nach 30 minütigem Rühren ist die Reaktion laut DC-Kontrolle vollständig. Der Ansatz wird vorsichtig (Wasserstoffgasentwicklung!) mit einer Lösung von 13 g Natriumhydrogensulfat Hydrat in 200 ml Wasser versetzt, kurz nachgerührt und sodann mit 2 mal 300 ml Diethylether extrahiert. Die vereinigten Phasen werden mit 1 N Salzsäure, gesättigter Natriumhydrogencarbonat-und Natriumchloridlösung gewaschen, mit Natriumsulfat getrocknet und am Vakuum eingeengt.

Ausbeute: 15,9 g (100X der Theorie)

Analytische Daten wie bereits unter Variante A angegeben.

### Beispiel 6

### N-tert-Butoxycarbonyl-4S-amino-3R,S-hydroxy-5-(4-nitrophenyl)pentansäure-ethylester

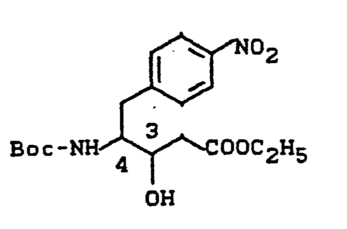

Zu 15 ml (160 mol-X) Diisopropylamin in 50 ml Tetrahydrofuran p.a. werden bei -20⁰C unter Stickstoff 72,3 ml (160 mol-X) n-Butyllithium getropft und 15 min bei dieser Temperatur nachgerührt. Man kühlt auf -80°C ab (Kühlbad: Aceton, Trockeneis und flüssiger Stickstoff) und gibt 10,5 ml (160 mol-%) Essigester bei einer Temperatur von unter -75°C zu. Nach 10 Minuten wird eine vorgekühlte Lösung von 18,6 g (63 mmol) N-tert-Butoxycarbanyl-L-(4-nitrophenyl)alaninal (Beispiel 5) in 70 ml Tetra-hydrofuran so zugetropft, daß die Temperatur der Reaktionsmischung unter -75°C bleibt. Man läßt 15 Minuten nachrühren und gibt dann 50 ml 2N HC1 hinzu. Die Mischung wird auf 10°C erwärmt und mit 2N HC1 auf pH 2,5 gestellt. Bei Raumtemperatur extrahiert man die Mischung 3 mal mit je 600 ml Diethylether, wäscht die vereinigten organischen Phasen 2 mal mit je 100 ml gesättigter Natriumchloridlösung, trocknet mit Magnesiumsulfat und engt am Rotationsverdampfer zur Trockene ein.

Rohausbeute: 21,2 g (83,3% der Theorie).

### Beispiel 7 und Beispiel 8

### N-tert-Butoxycarbonyl-4S-amino-3S-bzw. 3R-hydroxy-5-(4-nitrophenyl)pentansäure-ethylester

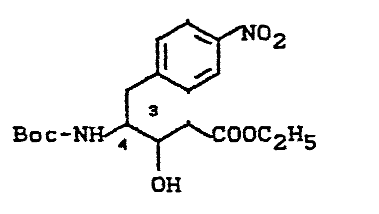

Die Trennung der beiden diastereomeren Produkte erfolgt durch Säulenchromatographie des Gemisches aus Bsp. 6 an Merck Kieselgel 60, Art 7734, Korngröße 0,063-0,2 mm, mit dem Laufmittelsystem ToluollEssigester 8:2 (Verhältnis stationäre Phase / zu trennendes Gemisch: 75:1).

Zuerst eluiert man das 3S,4S-Isomere (Bsp. 7) dann das 3R,4S-Isomere (Bsp. 8).

Bsp.-Nr, 7_{:} ¹H-NMR (CDCl₃, 300 MHz) = 8,15 (d, 2H); 7,42 (d, 2); 4,99 (d, 1H); 4,14 (q, 2H); 3,95 (d, 1H); 3,79 (m, 1H); 3,63 (s (breit) 1H); 3,04 (m, 2H); 2,60 (m, 1H); 2,39 (m, 1H); 1,39 (s, 9H); 1,26 (t, 3H).

DC-System VI: R_{f} = 0,21

HPLC-Wert, System II: Rₜ = 5,31 min

Fp.: 114 -116°C

Bsp.-Nr, 8: ¹H-NMR (CDCl₃, 200 MHz) δ = 8,15 (d, 2H); 7,42 (d, 2H); 4,62 (d, 1H); 4,20 (q, 2H); 3,90 (m, 2H); 3,60 (s, (breit) 1H); 3,19 (m, 1H); 2,90 (m, 1H); 2,65 (m, 2H); 1,40-1,25 (m, 12H)

DC-System VI : R_{f} = 0,13

HPLC-Wert, System II. Rₜ = 4,66 min

Fp.: 171-174°C

### Beispiel 9

### N-tert-Butoxycarbonyl-4S-amino-3S-hydroxy-5-(4-nitrophenyl)pentansäure

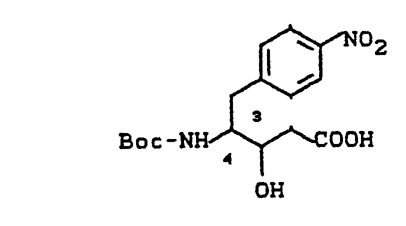

3,6 g (9,4 mmol) N-tert-Butoxycarbonyl-4S-amino-3S-hydroxy-5-(4-nitrophenyl)pentansäure-ethylester werden in 50 ml Dioxan/Wasser 1:1 suspendiert. Unter Rühren wird Dioxan bis zum vollständigen Lösen zugegben, mit 1N Natriumhydroxid auf pH 12-13 gestellt und dieser pH-Wert durch Zugabe von 1N Natriumhydroxid unter pH-Meter-Kontrolle gehalten. Laut DC war nach 4 Stunden noch keine vollständige Abreaktion erfolgt. Es werden 10 ml Ethanol zugegeben, der pH-Wert auf 13,5 gestellt und der Ansatz sodann über Nacht gerührt. Die Reaktionsmischung wird mit IN Salzsäure auf pH 6,5 gestellt, das Dioxan am Rotationsverdampfer abgezogen und mit NatriumbicarbonatLösung erneut alkalisch (pH 11) gemacht. Die wässrige Phase wird einmal mit Diethylether gewaschen, mit 1N Salzsäure auf pH 3 gestellt und mit Essigester extrahiert. Die organische Phase wird mit Natriumsulfat getrocknet und am Rotationsverdampfer zur Trockene eingeengt.

Ausbeute: 2,2 g (66% der Theorie)

Fp.: 168°C (Zers.)

¹H-NMR (CDCl₃ + DMSO, 300 MHz): δ = 8.11 (d, 2H); 7,44 (d, 2H); 5,55 (d, 1H); 3,96 (m, 1H); 3,81 (m, 1H); 2,99 (m, 2H); 2,33-2,48 (m, 2H); 1,37 (s, 9H).

(+)FAB-MS: m/z 355 (M+H); m/z 299; m/z 255

DC-System I: R_{f} = 0,24
V: R_{f} = 0,75

### Beispiel 10

### N-tert-Butoxycarbonyl-4S-amino-3S-hydroxy-5-(4-nitrophenyl)pentanoyl-L-isoleucin-methylester

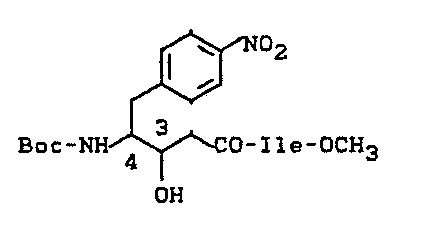

Zu einer Suspension von 0,73 g (110 mol-X) Isoleucin-methylester-Hydrochlorid in 30 ml Methylenchlorid gibt man 0,49 ml N-Methylmorpholin (120 mol-X). Die Lösung wird unter Rühren auf 0°C abgekühlt und es werden 1,3 g (3,7 mmol) N-tert-Butoxycarbonyl-4S-amino-3S-hydroxy-5-(4-nitrophenyl) pentansäure, 0,6 g (120 mol-X) N-Hydroxysuccinimid und 0,83 g (110 mol-X) DCC (gelöst in 15 ml Methylenchlorid), hinzugegeben.

Man läßt über Nacht bei 5°C rühren, saugt nochmals vom DCU ab, kühlt das Filtrat auf Trockeneis und saugt nochmals vom DCU ab. Das Filtrat wird je 2 x mit gesättigter Natriumhydrogencarbonat Lösung, kalter 0,1 N Salzsäure und gesättigter Natriumchlorid Lösung gewaschen. Die organische Phase wird mit Magnesiumsulfat getrocknet und am Rotationsverdampfer zur Trockene eingeengt.

Ausbeute: 1,8 g (100% der Theorie)

Fp.: 73-77°C

¹H-NMR (CDC1₃, 200 MHz): δ= 8,15 (d, 2H); 7,45 (d, 2H); 6,63 (d, 1H); 5,10 (d, 1H); 4,55 (m, 1H); 3,90 (m, 1H); 3,73 (s, 3H); 3,05 (m, 2H); 2,55 (m, 1H); 2,30 (m, 1H); 1,90 (m, 1H); 1,40 (s, 9H); 1,37 (m, 2H); 0,92 (m, 6H).

(+)FAB-M5: m/z 482 (M+H), m/z 504 (M+Na)

DC-System I: R_{f} = 0,57

### Beispiel 11

### 4S-amino-3S-hydroxy-5-(4-nitrophenyl)pentanoyl-L-isoleucin-methylester-Hydrochlorid

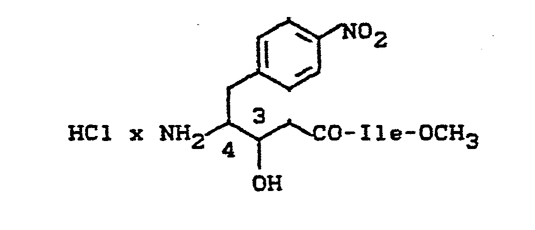

1,1 g (2,3 mmol) N-tert-Butoxycarbonyl-4S-amino-3S-hydroxy-5-(4-nitrophenyl)pentanoyl-L-isoleucin-methylester werden mit 1,15 ml 4N HC1 in Dioxan versetzt und bei Raumtemperatur gerührt. Nach 1/2 h engt man am Rotationsverdampfer zur Trockene ein und trocknet den Rückstand über Nacht am Hochvakuum.

Ausbeute: 1,1 g (114% der Theorie) (hygroskopisch)
(+)FAB-MS: m/z 382 (M+H)
DC-System I : R_{f =} 0,29
DC-System IV: R_{f} = 0,87
DC-System V : R_{f} = 0,79
DC-System XI: R_{f} = 0,51
HPLC-Wert, System II: Rₜ = 5,38 min

### BeisPiel 12

### N-tert-Butoxycarbonyl-L-phenylalanyl-L-histidyl-4S-amino-3S-hydroxy-5-(4-nitrophenyl)pentanoyl-L-isoleucin methylester

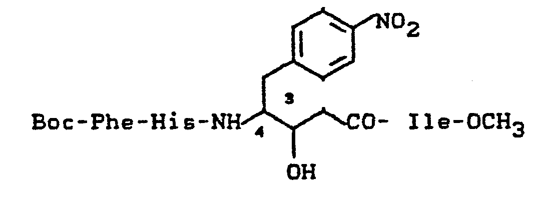

0,6 g (1,4 mmol) 4S-Amino-3S-hydroxy-5-(4-nitrophenyl)-pentanoyl-L-isoleucin-methylester-Hydrochlorid und 0,174 ml (110 mol-X) N-Methylmorpholin werden in 15 ml CH₂C1₂ suspendiert und es wird unter Rühren auf 0°C abgekühlt, dann gibt man 0,29 g (150 mol-X) HOBT, 0,39 g (130 mol-%) DCC und 0,69 g N-tert-Butoxycarbonyl-L-phenylalaninyl-L-histidin hinzu. Man läßt die Reaktionsmischung über Nacht auf Raumtemperatur erwärmen, filtriert das ausgefallene DCU ab, kühlt das Filtrat auf Trockeneis und saugt vom nachgefällten DCU ab. Das Filtrat wird 2 x mit gesättigter Natriumchloridlösung gewaschen. Die organische Phase wird mit Natriumsulfat getrocknet und das Lösungsmittel am Rotationsverdampfer abgezogen. Das erhaltene Rohprodukt (0,9 g) wird über 75 g Kieselgel, Merck Silicagel 60, Korngröße 0,063 - 0,200 mm, Art. 7734 mit dem Laufmittelsystem Methylenchlorid/Methanol 95/5 chromatographiert. Die das Produkt enthaltenden, sauberen Fraktionen werden vereinigt und zur Trockene eingeengt.

Ausbeute: 319 mg (29% der Theorie)

DC-System I: R_{f =} 0,55

HPLC-Wert System I: Rₜ = 6,29 min

(+)FAB-MS: m/z 766 (M+H).

### Beispiel 13

### N-tert-Butoxycarbonyl-L-phenylalanyl-L-phenylalanyl-4-S-amino-3S-hydroxy-5-(4-nitrophenyl)pentanoyl-L-isoleucin-methylester

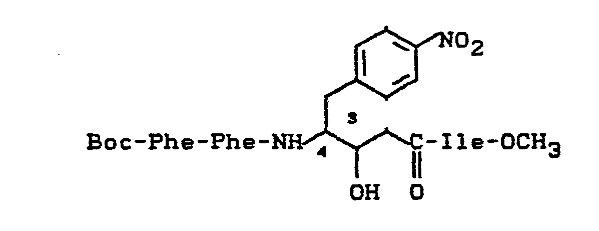

Die Titelverbindung wird analog Beispiel 12 hergestellt.Ausgehend von 0,3 g (9,72 mmol) des Hydrochlorids (Beispiel 11.) werden nach Chromatographie 190 mg erhalten.

Ausbeute: 190 mg (33% der Theorie)

DC-System I: R_{f} = 0.68

HPLC-Werte: System I : Rₜ = 12,62 min
System II: Rₜ = 21,90 min

(+)FAB-MS: m/z 776 (M+H)

### Beispiel 14

### N-tert-Butoxycarbonyl-4S-amino-3R-hydroxy-5-(4-nitrophenyl)pentansäure

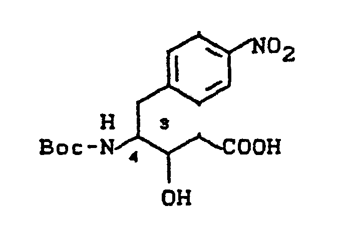

Die Titelverbindung wird analog Beispiel 9.) aus 3g (7,8 mmol) N-tert-Butoxycarbonyl-4S-amino-3R-hydroxy-5-(4-nitrophenyl)pentansäure-ethylester hergestellt.

Ausbeute: 1,7 g (61% der Theorie)

Fp.: 198-204°C Zersetzung

DC-System II: R_{f =} 0,24

¹H-NMR (CDC1₃ + DMSO, 300 MHz): δ = 8,10 (d, 2H); 7,4 (d, 2H); 5,50 (d, 1H); 4,01 (m, 1H); 3,74 (m, 1H); 3,15 (m, 1H); 2,85 (m, 1H); 2,40-2,60 (m, 2H); 1,27 (s 9H).

(+)FAB-MS: m/z 355 (M+H); m/z 299; m/z 255

### Beisipiel 15

### N-tert-Butoxycarbonyl-4S-amino-3R-hydroxy-5-(4-nitrophenyl)pentanoyl-L-isoleucin-methylester

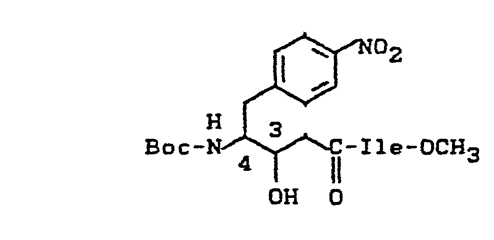

Die Titelverbindung wird analog Beispiel 10.) aus 1,3 g (3,4 mmol) Beispiel 14.) und Isoleucin-methylester Hydrochlorid hergestellt.

Ausbeute: 1,4 g (85,5% der Theorie)

DC-System I: R_{f} = 0,61

(+) FAB-MS: m/z 482 (M+H)

### Beispiel 16

### 4S-Amino-3R-hydroxy-5-(4-nitrophenyl)pentanoyl-L-isoleucin-methylester-Hydrochlorid

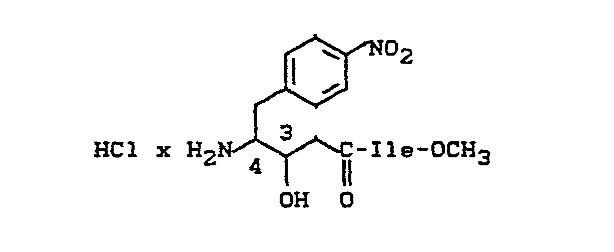

Die Titelverbindang wird analog Beispiel 11.) aus 1,18 g (2,4 mmol) Beispiel 15.) hergestellt.

Ausbeute: 1,23 g (120X der Theorie) hygroskopisch Fp.: - (+)FAB-M_{S}: m/z 382 (M+H)

### Beispiel 17

### N-tert-Butoxycarbonyl-L-phenylalanyl-L-histidyl-4S-amino-3R-hydroxy-5-(4-nitrophenyl)pentanoyl-L-isoleucin methylester

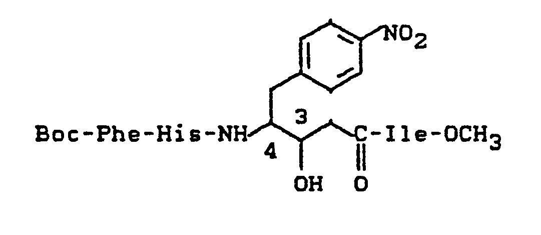

Die Titelverbindung wird analog Beispiel 12.) aus 0,61 g (1,2 mmol) Beispiel 16.) hergestellt.

Ausbeute: 684 mg (74% der Theorie)

Fp.: - (Amorph)

DC-System I: R_{f} = 0,56

HPLC-Wert; System II: Rₜ = 5,94 min

(+)FAB-MS: m/z 766 (M+H)

### Beispiel 18

### N-tert-Butoxycarbonyl-L-(4-aminophenyl)alanin

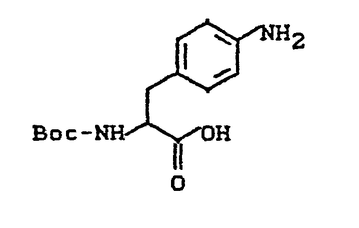

36 g (110 mmol) N-tert-Butoxycarbonyl-L-(4-nitrophenyl)alanin (A.V. Schally et al., J. Med. Chem. 16, 828 (1973)) werden in 500 ml Methanol gelöst, mit 5 g Palladiumkohle (10%ig) versetzt und 3 Tage bei Normaldruck hydriert. Der Katalysator wird abfiltriert und das Lösungsmittel am Vakuum abgezogen. Nach Umkristallisieren aus Diethylether wird die Titelverbindung reinst erhalten.

Ausbeute: 12,8 g (40X der Theorie)

Fp.: 145° C

¹H-NMR (DMSO, 200 MHz): δ = 6,90 (m, 2H); 6,40 (m, 2H); 4,03 (m, 1H); 3,37 (breit, 2H); 2,75 (dd, 1H); 2,65 (dd, 1H); 1,30 (s, 9H).

### Beisipiel 19

### N-tert-Butoxycarbonyl-L-[4-(N-9-fluorenylmethoxycarbo- yl)aminophenyl]alanin

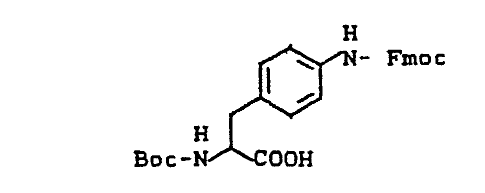

8,4 g (30 mmol) N-tert-Butoxycarbonyl-L-(4-aminophenyl)alanin werden unter Zusatz von 3,5 g Natriumcarbonat in 35 ml Wasser gelöst. Dazu wird eine Suspension von 9-Fluorenylmethoxycarbonyl-O-hydroxysuccinimidester (Fmoc-OSU) gegeben und die Reaktionsmischung wird 3 Tage bei Raumtemperatur gerührt. Der entstehende voluminöse Niederschlag wird abgesaugt und in 1 1 Wasser suspendiert. Die wäßrige Phase wird mit Salzsäure auf pH 4 gestellt und 2 mal mit je 300 ml Methylenchlorid extrahiert.Die vereinigten organischen Phasen werden über Natriumsulfat getrocknet, filtriert und am Rotationsverdampfer eingeengt. Das amorphe Rohprodukt (14,9 g) wird aus 800 ml Wasser/Isopropanol 7:3 umkristallisiert.

Ausbeute: 10,5 g (77% der Theorie)

Fp.: 115"C

DC-Wert System X: R_{f} = 0,80

¹H-NMR (DMSO, 300 MHz): 6 = 12,55 (b, 1H); 9,66 (s, 1H); 7,90 (m, 2H); 7,23 (m, 2H); 6,80-7,50 (m, 10H); 4,44 (m, 2H); 4,29 (m, 1H); 4,06 (m, 1H); 2,96 (dd, 1H); 2,78 (dd, 1H); 1,29 (s, 9H).

(+)FAB-MS: m/z 503 (M+H); m/z 447; m/z 403.

### Beispiel 20

### N-tert-Butoxycarbonyl-L-{4-(N-9-fluorenylmethoxycarbonyl)aminophenyl]alaninyl-4S-amino-3S-hydroxy-5-(4-nitrophenyl)pentanoyl-L-isoleucin-methylester

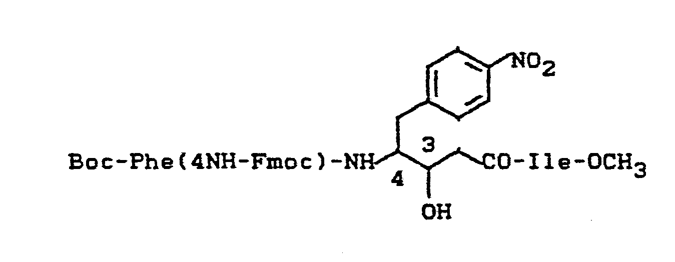

Die Titelverbindung wird mit der DCC/HOBT-Methode (wie Bsp. 12) aus 0,48 g (1 mmol) Beispiel 11.) und 0,60 g (1,2 mmol) Beispiel 19.) hergestellt.

Nach Chromatographie an Kieselgel mit CH₂Cl₂/MeOH 9:1 wird das Produkt rein erhalten.

Ausbeute: 620 mg (70% der Theorie)

Fp.: amorph

DC-System I: R_{f} = 0,7

(+)FAB-MS: m/z 866 (M+H); m/z 888 (M+Na); mlz 787, m/z 766.

### Beispiel 21

### N-tert-Butoxycarbonyl-L-(4-aminophenyl)alanyl-4S-amino-3S-hydroxy-5-(4-nitrophenyl)pentanoyl-L-isoleucin- methylester

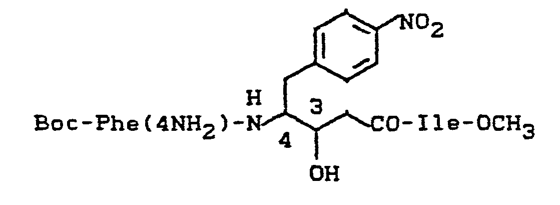

Die Titelverbindung wird analog Beispiel 24.) durch Abspaltung der Fmoc-Schutzgruppe aus 55 mg (0,063 mmol) Beispiel 20.) erhalten.

Ausbeute: 20,6 mg (50% der Theorie)

DC-System I : R_{f} = 0,52

DC-System II: R_{f} = 0,11

HPLC-Wert; System I: Rₜ = 4.94 min

(+)FAB-MS: m/z 777 ((M+C₇H₅NO₃)-H₂O))

### Beispiel 22

### L-4-(N(-9-Fluorenylmethoxycarbonyl)aminophenyl]alanyl-4S-amino-3S-hydroxy-5-(4-nitrophenyl)pentanoyl-L-isoleucin-methylester-Hydrochlorid

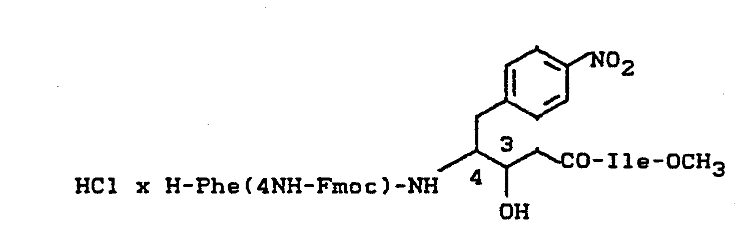

Die Titelverbindung wird aus 544 mg (0,62 mmol) Beispiel 20.) durch saure Abspaltung der α-Aminoschutzgruppe wie in Beispiel 11.) beschrieben erhalten.

Ausbeute: 440 mg (87% der Theorie)

Fp.: - (amorph, hygroskopisch)

DC-System I: R_{f} = 0,68

(+)FAB-MS: m/z 766 (M+H)

### Beispiel 23

### N-tert-Butoxycarbonyl-L-phenylalanyl-L-4-{N-(9-fluorenylmethoxycarbonyl)aminophenyl)alanyl-4S-amino-3S-hydroxy-5-(4-nitrophenyl)pentanoyl-L-isoleucin- methylester

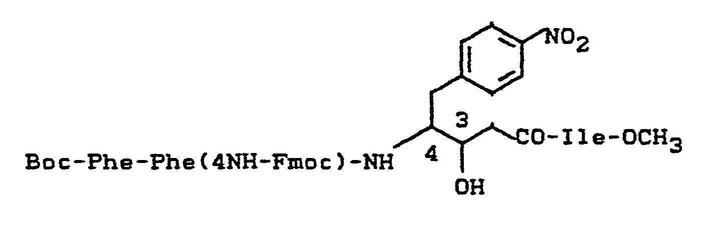

Die Titelverbindung wird aus 257 mg (0,32 mmol) Beispiel 22.) und N-tert-Butoxycarbonyl-L-Phenylalanin nach der DCC/HOBT Methode (analog Beispiel 12.)dargestellt. Aus dem Rohprodukt wird die Substanz durch Chromatographie an Kieselgel mit dem Laufmittelgemisch CH₂Cl₂/MeOH 98:2 in analytisch reiner Form erhalten.

Ausbeute: 75 mg (23X der Theorie)

Fp.: - (amorph)

DC-System IX: R_{f} = 0,27

(+)FAB-MS: m/z 1035 (M+Na); m/z 913; m/z 938

### Beispiel 24

### N-tert-Butoxycarbonyl-L-phenylalanyl-L-(4-aminophenyl)alanyl-4S-amino-3S-hydroxy-5-(4-nitrophenyl)pentanoyl-L-isoleucin-methylester

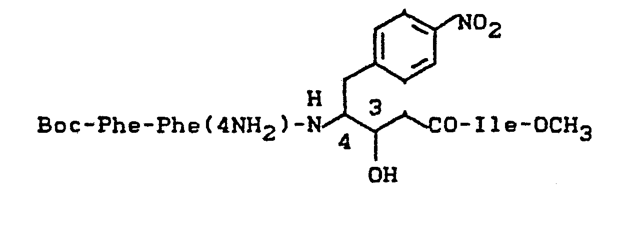

55 mg (0,68 mmol) der Fmoc-geschützte Verbindung des Beispiels 23.) wird 4 h in einer Mischung aus 1,5 ml Piperidin und 6 ml THF bei Raumtemperatur gerührt. Die Lösungsmittel werden im Vakuum abgezogen und das Rohprodukt durch Chromatographie an Kieselgel mit CH₂Cl₂/CH₃OH 9:1 gereinigt.

Ausbeute: 30,9 mg (57% der Theorie)

Fp.: - (amorph)

DC-System I : R_{f} = 0,65

DC-System II: R_{f} = 0,38

(+)FAB-MS: m/z 924 ((M+ C₇H₅NO₃)-H₂O)

### Beispiel 25

### N-tert-Butoxycarbonyl-L-(1-naphthyl)alanyl-L-{4-N(9-fluorenylmethoxycarbonyl)aminophenyl}alanyl-4S-amino-3S-hydroxy-5-(4-nitrophenyl)pentanoyl-L-isoleucin-methylester

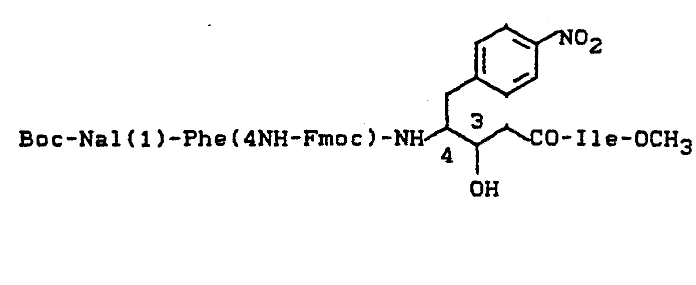

Die Titelverbindung wird nach der Propanphosphonsäureanhydrid-Methode analog Beispiel 37) und 47.)aus 139 mg (0,137 mmol) Beispiel 22.) und tert-Butoxycarbonyl-L-(l-naphthyl)alanin hergestellt. Das Rohprodukt (90 mg) wird aus EtherlHexan kristallisiert.

Ausbeute: 75 mg (40% der Theorie)

Fp.: - (amorph)

DC-System I: R_{f} = 0,5

HPLC-Wert; System I: Rₜ = 11,51 min.

(+)FAB-MS: m/z 1063 (M+H); m/z 963

### Beispiel 26

### N-tert-Butoxycarbonyl-L-(1-naphthyl)alanyl-L-(4-aminophenyl)alanyl-4S-amino-3S-hydroxy-5-(4-nitrophenyl)pentanoyl-L-isoleucin-methylester

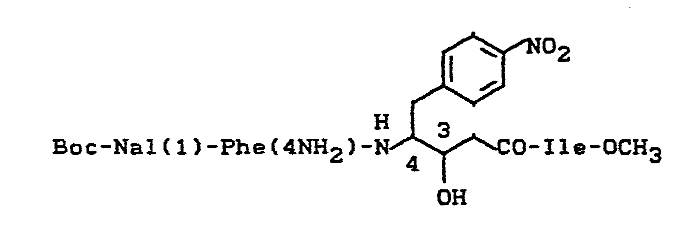

Die Titelverbindung wird aus 52 mg (0,05 mmol) Beispiel 25.) analog Beispiel 24.) durch Abspalten der Fmoc-Schutzgruppe dargestellt. Das Rohprodukt wird aus Ether/Hexan kristallisiert.

Ausbeute: 23 mg (56% der Theorie)

Fp.: - (amorph)

DC-System I: R_{f =} 0,60

(+)FAB-MS: m/z 841 (M+H); m/z 741

### Beispiel 27

### N-tert-Butoxycarbonyl-L-phenylalanyl-L-histidyl-4S-amino-3S-hydroxy-5-(4-aminophenyl)pentanoyl-L-isoleucin- methylester

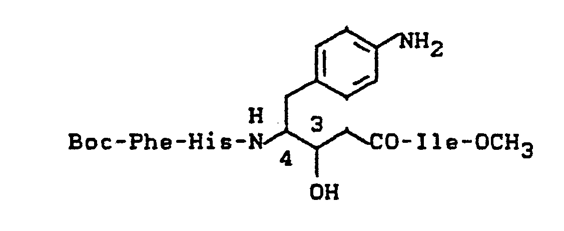

66mg (0,086 mmol) der entsprechende Nitroverbindung (Beispiel 12.) werden in 7,5 ml EssigesterlMethanol 1:2 gelöst. Dazu gibt man 62 mg 10%iger Palladiumkohle und hydriert die gerührte Suspension 1 h unter Normaldruck. Man filtriert den Katalysator ab und engt im Vakuum zur Trockene ein.

Ausbeute: 57 mg (90% der Theorie)

Fp.: - (amorph)

DC-System I: R_{f} = 0,47

HPLC-Wert; System I: Rₜ = 3,98 min

(+)FAB-MS: mlz 736 (M+H); m/z 869 ((M+C₇H₅NO₃)-H₂O),

### Beispiel 28

### N-tert-Butoxycarbonyl-L-phenylalanyl-L-phenylalanyl-4S-amino-3S-hydroxy-5-(4-aminophenyl)pentanoyl-L-isoleucin-methylester

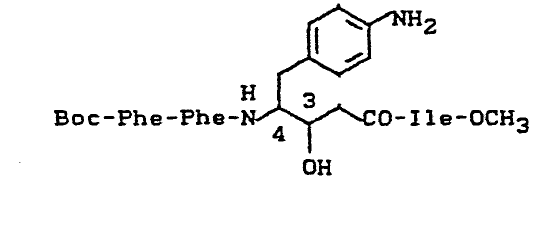

Die Titelverbindung wird aus 52 mg (0,067 mmol) Beispiel 13.) durch katalytische Hydrierung (wie in Beispiel 27.) beschrieben) dargestellt.

Ausbeute: 43 mg (86X der Theorie)

Fp.: - (amorph)

DC-System I: R_{f} = 0,53

HPLC-Wert; System I: Rₜ = 7,32 min

(+)FAB-MS: m/z 869 ((M ⁺ C₇H₅NO₃)-H₂O), m/z 769

### Beispiel 29

### N-tert-Butoxycarbonyl-L-phenylalanyl-L-histidyl-4S-amino-3R-hydroxy-5-(4-aminophenyl)pentanoyl-L-isoleucin-methylester

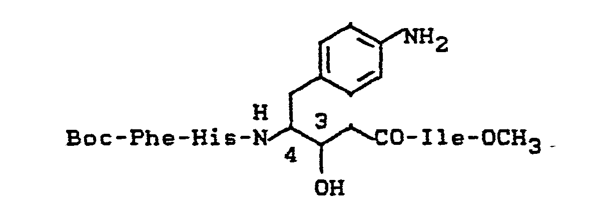

Die Titelverbindung wird aus 177 mg (0,23 mmol) Beispiel 17.) durch katalytische Hydrierung (wie in Beispiel 27.) beschrieben) dargestellt.

Ausbeute: 151,6 mg (90% der Theorie)

Fp.: - (amorph)

DC-System: I: R_{f} = 0,48

HPLC-Wert; System I: Rₜ = 3,94 min

### BeisPiel 30

### N-tert-Butoxycarbonyl-L-phenylalanyl-4S-amino-3S-hydroxy-5-(4-nitrophenyl)pentanoyl-L-isoleucin-methylester

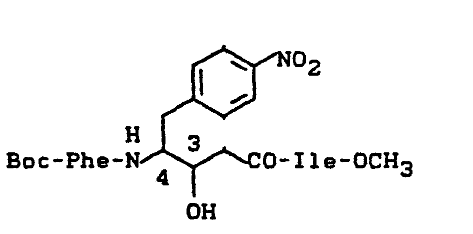

Die Titelverbindung wird nach der Propanphosphonsäureanhydrid-Methode (wie in Beispiel 37.) und 47.) beschrieben) aus 0,6 g (1,4 mmol) Beispiel 11.) und 420 mg (1,6 mmol) N-tert-Butoxycarbonyl-L-Phenylalanin hergestellt.

Ausbeute: 690 mg (78% der Theorie)

Fp.: - (amorph)

DC-System I : R_{f =} 0,86

HPLC-Wert; System II: Rₜ = 10,55 min.

(+)FAB-MS: m/z 629 (M+H); m/z 573; m/z 529

### Beispiel 31

### L-(2-Nitrophenyl)alanin-methylester-Hydrochlorid

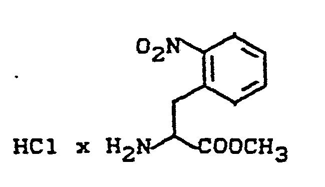

Die Titelverbindung wird analog Beispiel 2.) aus 30,3 g (0,12 mol) L-(2-Nitrophenyl)alanin-hydrochlorid (Alvie L. Davies et al., J. Med. Chem. 15, 325-327 (1972)) hergestellt.

Ausbeute: 15,6 g (50X der Theorie)

DC-System I: R_{f} = 0,60

DC System VII: R_{f} = 0,76

¹H-NMR (DMSO, 250 MHz): δ = ₈,₉₅ (b, 3H); 8,04 (m, 1H); 7,50-7,75 (m, 3H); 4,20 (m, 1H); 3,60 (s, 3H); 3,48 (m, 2H).

### Beispiel 32

### N-tert-Butoxycarbonyl-L-(2-nitrophenyl)alanin-methylester

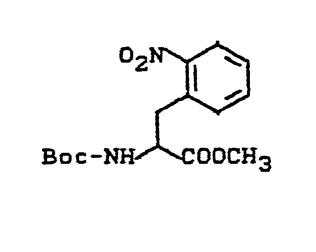

Die Titelverbindung wird analog Beispiel 3.) aus 10,5 g (0,04 mol) Beispiel 31.) hergestellt.

Ausbeute: 11.2 g (87,6% der Theorie)

DC-System I : R_{f} = 0,72

DC-System II: R_{f} = 0,75

¹H-NMR (CDC1₃, 250 MHz):δ = 7,90 (m, 1H); 7,30-7,75 (m, 3H); 5,20 (d, 1H); 4,65 (m, 1H); 3,72 (s, 3H); 3,52 (m, 1H); 3,26 (m, 1H); 1,45 (s, 9H).

### Beispiel 33

### N-tert-Butoxycarbonyl-L-(2-nitrophenyl)alaninol

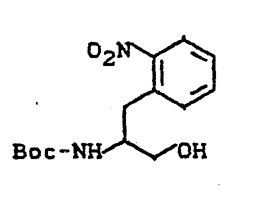

Die Titelverbindung wird analog Beispiel 4.) aus 17,9 g (55 mmol) Beispiel 32.) hergestellt.

Ausbeute: 14,3 g (87,7% der Theorie)

DC-System II: R_{f} = 0,32

¹H-NMR (200 MHz, CDCl₃) δ = 7,90 (m, 1H); 7,35-7,62 (m, 3H); 5,12 (d, 1H); 4,04 (m, 1H); 3,60 (m, 2H); 2,95-3,30 (m, 2H); 2,67 (breit, 1H); 1,33 (s, 9H).

(+)FAB-MS: m/z 297 (M+H)

### Beispiel 34

### N-tert-Butoxycarbonyl-L-(2-nitrophenyl)alaninal

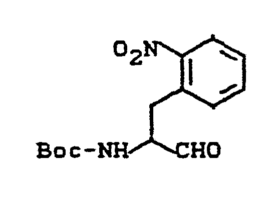

Die Titelverbindung wird analog Beispiel 5.) aus 13,5 g (0,045 mol) Beispiel 33.) hergestellt.

Ausbeute: 11,3 g (85% der Theorie)

¹H-NMR (200 MHz, CDCl₃):δ = 9,69 (s, 1H); 8,04 (m, 1H); 7,35-7,62 (m, 3H); 5,40 (d, 1H); 4,59 (m, IH); 3,63 (dd, 1H); 3,12 (dd, 1H); 1,35 (s, 9H).

### Beispiel 35.

### N-tert-Butoxycarbonyl-4S-amino-3RS-hydroxy-5-(2-nitrophenyl)pentansäure-ethylester

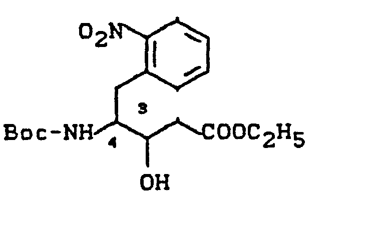

Die Titelverbindung wird analog Beispiel 6.) aus 11,3 g (38,4 mmol) N-tert-Butoxycarbonyl-L-(2-nitrophenyl)-alaninal (Beispiel 34.) und dem Lithiumsalz des Essigsäureethylesters hergestellt. Rohausbeute: 12,3 g (83,3 % der Theorie) Die entstehenden Stereoisomeren, N-tert-Butoxycarbonyl-4S-amino-3R bzw. 3S-Hydroxy-5-(0-2-nitrophenyl)pentansäure-ethylester werden durch Säulenchromatographie analog Beispiel 7.) und 8.) nicht vollständig getrennt, so daß die folgende Verseifung analog Beispiel 9.) mit dem Diastereomerengemisch durchgeführt wird.

DC-System VI: R_{f =} 0,12-0,18

HPLC-Werte; System I: Diastereomer A, Rₜ = 4,22 min Diastereomer B, R_{t =} 4,44 min

¹H-NMR (200 MHz, CDCl₃):δ = 7,95 (m, 1H); 7,15-7,60 (m, 3H); 5,13 (d+d, 1H); 4,20 (m, 3H); 3,98 (m, 1H); 4,80 (breit, 1H); 2,50-3,40 (m, 4H); 1,20-1,35 (m, 12H).

(+)FAB-MS: m/z 383 (M+H); m/z 327; m/z 283

### Beispiel 36

### N-tert-Butoxycarbonyl-4S-amino-3RS-hydroxy-5-(2-nitrophenyl)pentansäure

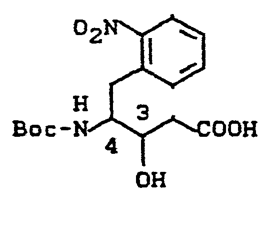

Die Titelverbindung wird analog Beispiel 9.) aus 2,25 g (5,9 mmol) N-tert-Butoxycarbonyl-4S-amino-3RS-hydroxy-5-(2-nitrophenyl)pentansäure-ethylester (Beispiel 35.) hergestellt.

Ausbeute: 0,88 g (42% der Theorie)

Fp.: - (amorph)

(+)FAB-MS: m/z 355 (M+H); m/z 299; m/z 255

### Beispiel 37

### N-tert-Butoxycarbonyl-4S-amino-3RS-hydroxy-5-(2-nitrophenyl)pentanoyl-L-isoleucin-methylester

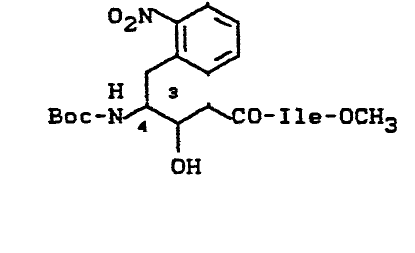

Zu einer Suspension von 0,53 g (3 mmol) Isoleucin-methylester-Hydrochlorid und 0,7 g (2 mmol) N-tert-Butoxycarbonyl-4S-amino-3RS-hydroxy-5-(2-nitrophenyl)pentansäure (Beispiel 36.) in 10 ml Methylenchlorid gibt man bei 0°C 1,1 g (=̂ 1,53 ml) Triethylamin. Man läßt 10 Minuten rühren, kühlt auf -20°C (Trockeneis/Aceton) und tropft bei dieser Temperatur 1,43 ml (=̂ 2,2 mmol) einer 50Xigen Lösung von Propanphosphonsäureanhydrid in Methylenchlorid. Man läßt den Ansatz 1 Stunde bei -20°C und über Nacht bei Raumtemperatur rühren. Die Reaktionsmischung wird nacheinander 3 mal mit je 10 ml 5%ige Natriumhydrogencarbonatlösung, 3 mal mit je 10 ml 1 N Salzsäure, 1 mal mit 10 ml 5%ige Natriumhydrogencarbonatlösung und schließlich 1 mal mit 10 ml gesättigter Kochsalzlösung ausgeschüttelt. Die organische Phase wird über Magnesiumsulfat getrocknet und im Vakuum eingeengt. Durch Trocknen am Hochvakuum wird das Produkt als amorpher Schaum erhalten.

Ausbeute: 0,75 g (78% der Theorie)

¹H-NMR (300 MHz, DMSO):δ= 8,13 (d, 1H); 7,89 (m, 1H); 7,37-7,68 (m, 3H); 6,54 (d) und 6,44 (d): zusammen 1H; 5,04 (d) und 4,88 (d): zusammen 1H; 4,23 (m, 1H); 3,85 (m, 2H); 3,61 (s) und 3,62 (s): zusammen 3H; 3,28 (m, 2H); 2,10 -2,85 (m, 2H); 1,73 (m, 1H); 1,08-1,46 (m, 2H); 1,18 (s) und 1,22 (s): zusammen 9H; 0,83 (m, 6H).

(+)FAB MS: m/z 482 (M+H); m/z 504 (M+Na); m/z 426; m/z 382.

HPLC-Werte; System I: Diastereomer A (3R,4S bzgl. des (2-nitrophenyl)pentanoylteils)
Rt ₌ 5,70
Diastereomer B (3S,4S bzgl. des (2-nitrophenyl)pentanoylteils)
Rt ⁼ 6,32

### Beispiel 38 und Beispiel 39

### N-tert-Butoxycarbonyl-4S-amino-3R-hydroxy-5-(2-nitrophenyl)pentanoyl-L-isoleucin-methylester (Bsp. 38) und N-tert-Butoxycarbonyl-(4S-amino-3S-hydroxy-5-(2-nitrophenyl pentanoyl-L-isoleucin-methylester (Beispiel 39)

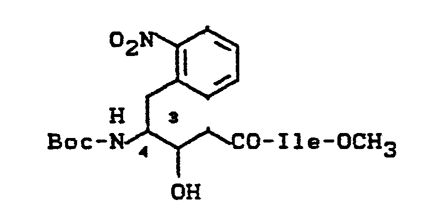

Durch isokratische präparative Mitteldruckchromatographie der Diastereomerenmischung aus Beispiel 37.) über eine Merck Lobar Fertigsäule RP8, Größe C (440-37), Lichroprep® RP-8 40-63 mm, Artikelnummer: 10629 mit dem Laufmittelgemisch Wasser/ Acetonitril 1:1 lassen sich die beiden Verbindungen Beispiel 38.) und 39.) isomerenrein erhalten.

HPLC-Werte; System I: Beispiel 38: Rₜ = 5,70
Beispiel 39: R_{t =} 6,32

### Beispiel 40 und Beispiel 41.

### N-tert-Butoxycarbonyl-L-phenylalanyl-L-histidyl-4S-amino-3RS-hydroxy-5-(2-nitrophenyl)pentanoyl-L-isoleucin-methylester (Bsp. 40) und

### N-tert-Butoxycarbonyl-L-phenylalanyl-L-histidyl-4S-amino-3S-hydroxy-5-(2-nitrophenyl)pentanoyl-L-isoleucin- methylester (Bsp. 41)

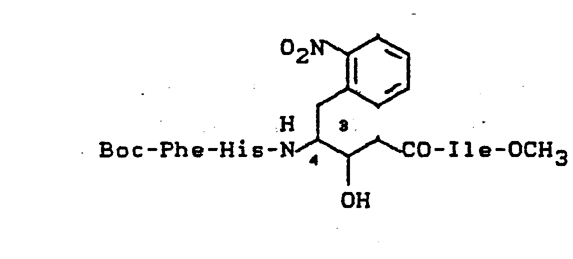

300 mg ( 0,63 mmol) N-tert-Butoxycarbonyl-4S-amino-3RS-hydroxy-5-(2-nitrophenyl)pentanoyl-L-isoleucin- methylester (Beisp. 37.) werden zum Abspalten der tert-Butoxycarbonyl-Schutzgruppe mit 10 ml 4N HCl in Dioxan gerührt, eingeengt und 1 Stunde am Hochvakuum getrocknet. Das erhaltene Dihydrochlorid wird dann wie unter Beispiel 12.) beschrieben, mit Boc-Phe-His-OH (Beispiel 48) nach der DCC/HOBT-Methode zur Titelverbindung gekoppelt Ausbeute: 480 mg

Durch Mitteldruckchromatographie des Rohproduktes an einer Merck-Lobar-Fertigsäule Größe B(310-25) Si0₂-LiChroprep® Si 60 (40-63 µm), Art-Nr. 10401, mit dem Laufmittelgemisch CH₂Cl₂/CH₃OH 9:1 erhält man die beiden Titelverbindungen in reiner Form.

### Beispiel 41.), eluiert zuerst bei obiger Chromatographie, Ausbeute: 91 mg

(+)FAB-MS: m/z 766 (M+H)

HPLC-Wert; System I: R_{t =} 5,47

Beispiel 40.) Ausbeute: 160 mg

(+)FAB-MS: m/z 766 (M+H)

HPLC-Wert; System I: Rₜ = 5,47 und - 5,20

### Beispiel 42

### N-tert-Butoxycarbonyl-L-phenylalanyl-L-histidyl-4S-amino-3RS-hydroxy-5-(2-aminophenyl)pentanoyl-L-isoleucin-methylester

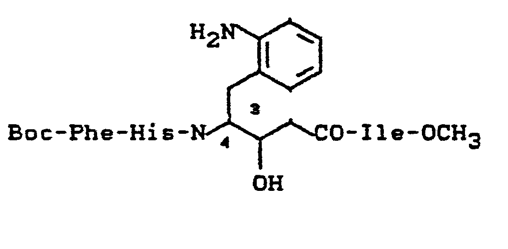

74 mg (0,lmmol) der entsprechende 2-Nitroverbindung (Beispiel 40.), wird in 10 ml Methanol gelöst, mit 55 mg 10%iger Palladiumkohle versetzt und eine Stunde bei Normaldruck hydriert. Nach der DC-Kontrolle wird der Katalysator abfiltriert und das Lösungsmittel eingeengt. Ausbeute: 47 mg (64% der Theorie)

Fp.: - (amorph, Diastereomerenmischung)

HPLC-Werte; System I: 4,09 min und 4,43 min

### Beispiel 43

### N-Benzoyl-D,L-(1-triazolyl)alanyl-4S-amino-3S-hydroxy-5-(4-nitrophenyl)pentanoyl-L-isoleucin-methylester

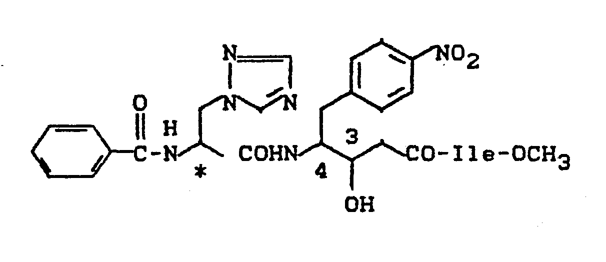

Die Titelverbindung wird aus 111mg (0,26 mmol) Beispiel 12.) und 80,6 mg (0,31 mmol) N-Benzoyl-DL-(1-triazolyl)-alanin nach der DCC/HOBT-Methode (wie in Beispiel 12.) beschrieben) hergestellt.

Rohausbeute: 240 mg

DC-System I: R_{f} = 0,57

HPLC-Wert; System I: Rₜ = 2,88 min und 3.00 min (+)FAB-MS: m/z 624 (M+H)

Die gesamte Rohausbeute aus Beispiel 43.) wird weiter zu Beispiel 44.) und Beispiel 45.) verarbeitet wobei Beispiel 44.) das Diastereomere von Beispiel 45.) darstellt, die exakten Konfigurationen am asym. Zentrum des (1-Triazolyl)alanins aber nicht bestimmt wurden.

### Beispiel 44 und Beispiel 45

### N-Benzoyl-D-(1-triazolyl)alaninyl-4S-amino-3S-hydroxy-5-(4-nitrophenyl)pentanoyl-L-isoleucin-methylester (Bsp. 44) und

### N-Benzoyl-L-(1-triazolyl)alaninyl-4S-amino-3S-hydroxy-5-(4-nitrophenyl)pentanoyl-L-isoleucin-methylester (Bsp.4S)

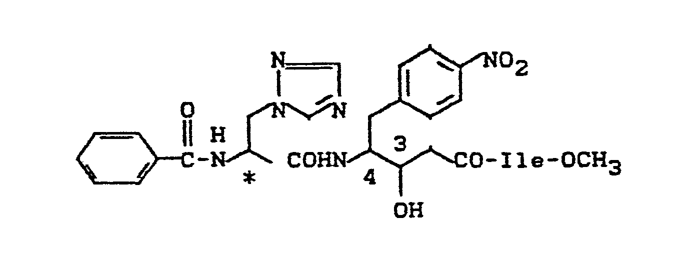

Die Rohausbeute aus Beispiel 43.) wird durch Mitteldruckchromatographie über eine Merck Lobar Fertigsäule, Lichroprep® RP-8 (40-63 µm), Größe B, 310-297 Art-Nr. 11804, mit dem Laufmittelgemisch Acetonitril/ Wasser 50:50 gereinigt. Die Fließgeschwindigkeit beträgt 15 ml/min, die Fraktionsgröße 15 ml. Die einzelnen Fraktionen werden je nach HPLC-Kontrolle vereinigt. Nach Lyophilisierung werden die Titelverbindungen reinst erhalten.

Ausbeute: Fraktion 13,14: 18,5 mg = Beispiel 44.)
Fraktion 15: 34,2 mg = D,L Mischung (=Beispiel 43.)
Fraktion 16,17: 31,7 mg = Beispiel 45.)

### Beispiel 44.):

Ausbeute: 18.5 mg

HPLC-Wert; System I: Rₜ = 2,87 min

(+)FAB-MS: m/z 624 (M+H)

### Beispiel 45.):

Ausbeute: 31,7 mg

HPLC-Wert; System I: Rₜ = 2,99 min

(+)FAB-MS: m/z 624 (M+H)

### Beispiel 46

### N-Benzoyl-D,L-(1-triazolyl)alanyl-4S-amino-3S-hydroxy-5-(4-aminophenyl)pentanoyl-L-isoleucin-methylester

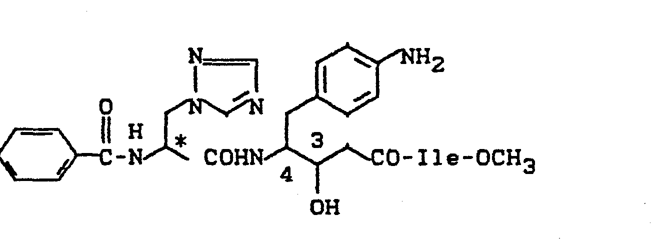

Die Titelverbindung wird durch katalytische Hydrierung (wie in Beispiel 27.) beschrieben) der entsprechenden Nitroverbindung (D,L-Gemisch aus Beispiel 44.) und 45.), Fraktion 15, 31,2 mg, 50 mmol) erhalten. Das Rohprodukt wird aus Essigester umkristallisiert.

Ausbeute: 27,1 mg (91% der Theorie)

Fp.: - (amorph)

DC-System I: R_{f =} 0,47 und R_{f} = 0,50

HPLC-Wert; System I: Rₜ = 2.01 min, ununterscheidbar. (+)FAB-MS: m/z 594 (M+H)

### Beispiel 47

### N-tert-Butoxycarbonyl-L-phenylalanyl-L-histidinmethylester

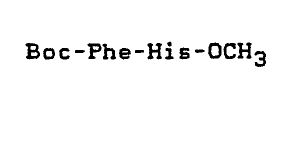

43,73 g (164 mmol) N-tert-Butoxycarbonyl-L-phenylalanin und 34,5 g (142 mmol) L-Histidinmethylester-dihydrochlorid werden in 400 ml Methylenchlorid vorgelegt. Unter Rühren gibt man bei -20°C (Aceton-Trockeneisbad) 100 g (1 mol) Triethylamin hinzu. Die gekühlte Mischung wird nun tropfenweise mit 111 ml (0,171 mol) Propanphosphonsäureanhydridlösung (50Xig in Methylenchlorid) versetzt. Über Nacht läßt man auf Raumtemperatur erwärmen. Das Lösungsmittel wird abgezogen und das Rohprodukt in 500 ml Essigester aufgenommen. Die organische Phase wird 4 mal mit je 200 ml gesättigter Natriumbicarbonatlösung, 3 mal mit 0,1 N Salzsäure und 1 mal mit gesättigter Kochsalzlösung gewaschen, über Magnesiumsulfat getrocknet und im Vakuum eingeengt.

Ausbeute: 29,2 g (49X der Theorie)

Fp.: - (amorph)

DC-System I: R_{f} = 0,50

HPLC-Wert; System I : Rₜ = 10,68 min
System II: Rₜ = 16,73 min

### Beispiel 48

### N-tert-Butoxycarbonyl-L-phenylalanyl-L-histidin

Boc-Phe-His-OH

Die Titelverbindung wird aus Beispiel 47.) durch alkalische Verseifung hergestellt. Hierzu werden 4,16 g (10 mmol) des Methylesters in 200 ml Dioxan und 20 ml IN NaOH gelöst. Man rührt 2,5 Tage bei Raumtemperatur, zieht am Rotationsverdampfer vom Dioxan ab, neutralisiert den Ansatz mit 20 ml 1 N HC1 und saugt den ausgefallenen Feststoff ab. Das Produkt wird mit Wasser nachgewaschen und über Phosphorpentoxid getrocknet. Ausbeute: 2,7 g (67X der Theorie)

Fp.: - (amorph)

DC-System I: R_{f =} 0,10

### Beispiel 49

### D,L-(1-Triazolyl)alanin

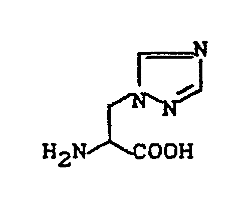

0,6 g (0.0026 mol) Natrium werden in 150 ml Methanol gelöst. Dazu gibt man 8,5 g (0,123 mol) Triazol. Zu der gerührten Miscchung werden 8,5 g (0,123 mol) N-Acetyldehydroalanin-ethylester (H. Hellmann et al., Chem. Ber. 91, 2427-2431 (1958)) gelöst in 150 ml Ethanol zugetropft. Man rührt noch eine Stunde am Rückfluß und dampft sodann die Lösungsmittel ab.

Zur Abspaltung der Schutzgruppen wird der Rückstand von N-Acetyl-D,L-(1-Triazolyl)alanin-ethylester 8 Stunden in halbkonzentrierter Salzsäure gekocht. Die Salzsäure wird abgedampft. Das Rohprodukt wird zum Entfernen der überschüssigen Salzsäure zuerst 3 mal mit Wasser und danach noch zweimal mit Toluol einrotiert. Das rohe salzsaure D,L-(l-Triazolyl)alanin wird in Wasser heiß gelöst. Der pH-Wert der Lösung wird mit Diethylamin auf pH 7 gestellt. Nach dem Erkalten wird die in Form weißer Kristalle ausgefallene Titelverbindung abgesaugt, mit Methanol nachgewaschen und getrocknet.

Ausbeute: 7,5 g (48 X der Theorie)

Fp.: 250°C (Zers.)

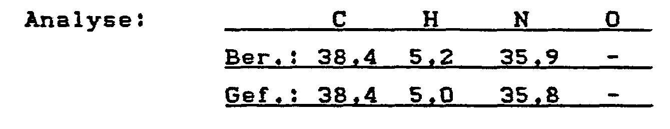

### Beispiel 50

### N-Benzoyl-D,L-(1-Triazolyl)alanin

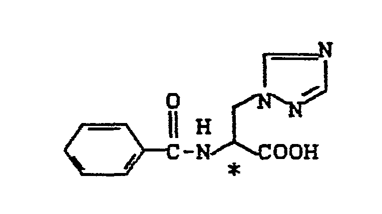

7,8 g (0,05 mol) D,L-(1-Triazolyl)alanin werden in einer Mischung von 2 g Natriumhydroxid und 23 ml Wasser gelöst. Bei einer Temperatur von unter 30°C (Eiskühlung) tropft man 7 g (0.05 mol) Benzoylchlorid an der gerührten Mischung, rührt bei dieser Temperatur noch 4 Stunden nach und läßt über Nacht bei Raumtemperatur stehen. Man saugt das ausgefallene Rohprodukt ab und wäscht mit kaltem Wasser nach. Der Rückstand wird mit Aceton ausgekocht, heiß abgesaugt, mit Aceton nachgewaschen und getrocknet.

Ausbeute: 3,3 g (25,4% der Theorie)

Fp.: 216-220⁰C

MS: m/z 260; m/z 215; m/z 191; m/z 147; m/z 105
(Basepeak); m/z 83; m/z 77; m/z 51.

### Beispiel 51

### L-(3-Nitrophenyl)alanin-methylester-Hydrochlorid

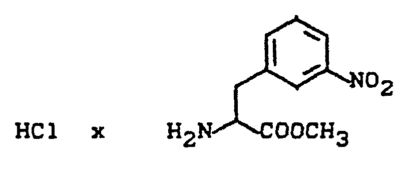

Die Titelverbindung wird analog Beispiel 2 aus 134 g (0.57 mol) L-(3-Nitrophenyl)alanin-hydrochlorid hergestellt. Ausbeute: 133,6 g (90% der Theorie)

DC-System I: R_{f} = 0,58

¹H-NMR (DMSO, 250 MHz): δ = 8,88 (b, 3H); 8,16 (m, 2H); 7,07 - 7,82 (m, 2H); 4,40 (m, 1H); 3,70 (s, 3H); 3,34 (m, 2H).

### Beispiel 52

### N-tert-Butoxycarbonyl-L-(3-nitrophenyl)alanin-methylester

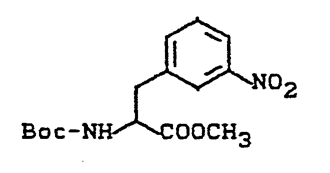

Die Titelverbindung wird analog Beispiel 3 aus 93,5 g (0,36 mol) L-(3-Nitrophenyl)-methylester-Hydrochlorid (Beispiel 51) hergestellt.

Ausbeute: 96,3 g (82,4% der Theorie)

DC-System I : R_{f} = 0,32

### Beispiel 53

### N-tert-Butoxycarbonyl-L-(3-nitrophenyl)alaninol

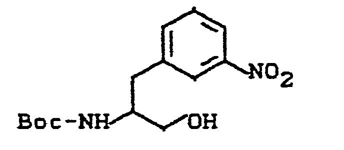

Die Titelverbindung wird analog Beispiel 4 aus 90 g (0,27 mol) N-tert-Butoxycarbonyl-L-(3-nitrophenyl)alanin-methylester (Beispiel 52) hergestellt.

Ausbeute: 60 g (73% der Theorie)

DC-System I : R_{f} = 0,68

DC-System II: R_{f} = 0,78

DC-System IV: R_{f} = 0,18

¹H-NMR (CDC1₃, 250 MHz): α = 8.05 (m, 2H); 7,55 (m, 1H); 7,41 (m, 1H); 4,98 (b, 1H); 3,84 (b, 1H); 3,59 (m, 2H); 2,92 (m, 2H); 1,33 (s, 9H).

### BeisPiel 54

### N-tert-Butoxycarbonyl-L-(3-nitrophenyl)alaninal

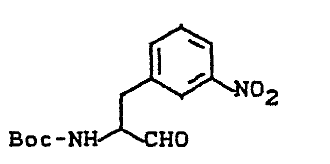

Die Titelverbindung wird analog Beispiel 5 aus 30 g (0,1 mol) N-tert-Butoxycarbonyl-L-(3-nitrophenyl)alaninol (Beispiel 53) hergestellt.

Ausbeute: 26,1 g (87,6% der Theorie)

DC-System II: R_{f} = 0,72

DC-System VI: R_{f} = 0,29

### Beispiel 55

### N-tert-Butoxycarbonyl-4S-amino-3RS-hydroxy-5-(3-nitrophenyl)pentansäure-ethylester

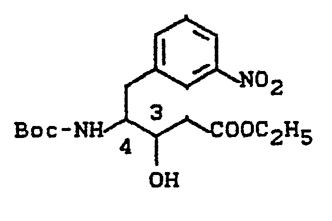

Die Titelverbindung wird analog Beispiel 6 aus 26,1 g (88,7 mmol) N-tert-Butoxycarbonyl-L-(3-nitrophenyl)-alaninal (Beispiel 54) und dem Lithiumsalz des Essigsäureethylesters hergestellt. Ausbeute: 27,3 g (80 % der Theorie) (+) FAB-MS: m/z 383 (M+H); m/z 327; m/z 283

### Beispiel 56 und Beispiel 57

N-tert-Butoxycarbonyl-4S-amino-3S-hydroxy-5-(3-nitrophenyl)pentansäure-ethylester und N-tert-Butoxycarbonyl-4S-amino-3R-hydroxy-5-(3-nitrophenyl)pentansäure-ethylester

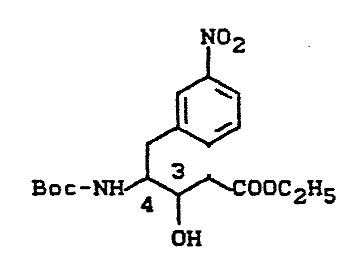

Die Trennung der beiden diasteromeren Produkte erfolgt durch Säulenchromatographie des Gemisches aus N-tert-Butoxycarbonyl-4S-amino-3RS-hydroxy-5-(3-nitrophenyl)pentansäure-ethylester (Beispiel 55) unter den in Beispiel 7 und 8 beschriebenen Bedingungen.

Zuerst eluiert das 3S,4S-Isomere (Beispiel 56) dann das 3R,4S-Isomere (Beispiel 57)

### Beispiel-Nr. 56:

¹H-NMR (CDCl₃, 250 MHz): δ = 8,09 (m, 2H); 7,6 (m, 1H); 7.46 (m, 1H); 4,98 (d, 1H); 4,16 (q, 2H); 3,98 (m, 1H); 3,77 (m, 1H); 3,61 (b, IH); 3,02 (m, 2H); 2,61 (m, 1H); 2,40 (m, 1H); 1,36 (s, 9H); 1,24 (t, 3H).

DC-System VI: R_{f} = 0,25

HPLC-Wert, System II: R_{f} = 5,31 min

### Beispiel-Nr. 57:

¹H-NMR (CDC1₃, 250 MHz): 6 = 8,09 (m, 2H); 7,59 (m, 1H); 7,46 (m, 1H); 4,66 (d, 1h); 4,18 (q, 2H); 4,01 (m, 1H); 3,84 (m, 1H); 3,59 (s, (breit) 1H); 3,13 (m, 1h); 2,88 (m, 1H); 2,46 - 2,78 (m, 2H); 1,29 (s, 9H); 1,27 (t, 3H).

DC-System VI: R_{f} = 0,22

HPLC-Wert; System II: R_{f} = 4,73 min

### Beispiel 58

### N-tert-Butoxycarbonyl-4S-amino-3RS-hydroxy-5-(3-nitrophenyl)pentansäure

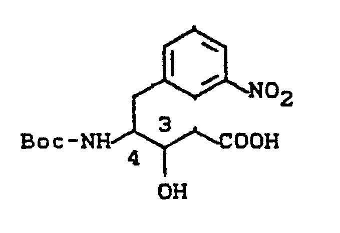

Die Titelverbindung wird analog Beispiel 9 aus 20,2 g (53 mmol) des Rohgemisches von N-tert-Butoxycarbonyl-4S-amino-3RS-hydroxy-5-(3-nitrophenyl)pentansäure-ethylester (Beispiel 55) hergestellt.

Ausbeute: 8,75 g (45% der Theorie)

DC-System I : R_{f} = 0,42

DC-System II: P_{f} = 0,20

### Beispiel 59

### N-tert-Butoxycarbonyl-4S-amino-3RS-hydroxy-5-(3-nitrophenyl)pentanoyl-L-isoleucin-methylester

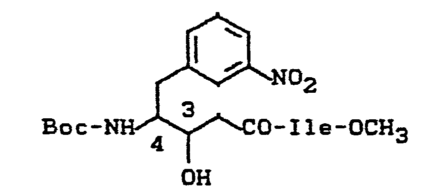

Die Titelverbindung wird analog Beispiel 37 aus 7,1 g (20 mmol) N-tert-Butoxycarbonyl-4S-amino-3RS-hydroxy-5-(3-nitrophenyl)pentansäure (Beispiel 58) und Isoleucin- methylester Hydrochlorid hergestellt.

Ausbeute: 9 g (96X der Theorie)

DC-System II: R_{f} = 0,61 (Isomer A)
R_{f} = 0,50 (Isomer B)

### Beispiel 60

### 4S-Amino-3RS-hydroxy-5-(3-nitrophenyl)pentanoyl-L-isoleucin-methylester-Hydrochlorid

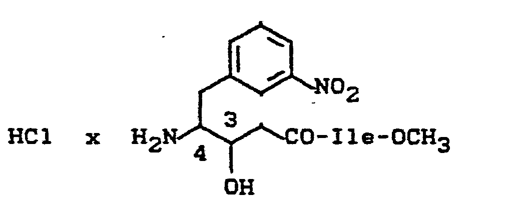

Die Titelverbindung wird analog Beispiel 11 aus 8,9 g (18,5 mmol) N-tert-Butoxycarbonyl-4S-amino-3RS-hydroxy-5-(3-nitrophenyl)pentanoyl-L-isoleucin-methylester (Beispiel 59) hergestellt.

Ausbeute: 7,7 g (100% der Theorie)

### Beispiel 61

### N-tert-Butoxycarbonyl-L-phenylalanyl-L-histidyl-4S-amino-3RS-hydroxy-5-(3-nitrophenyl)pentanoyl-L-isoleucin-methylester

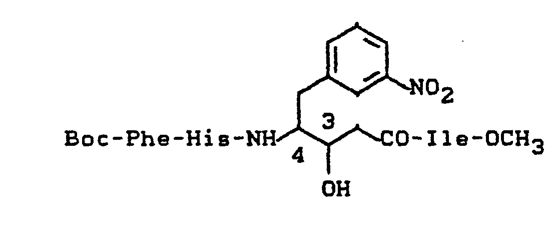

Die Titelverbindung wird aus 4S-Amino-3RS-hydroxy-5-(3-nitrophenyl)pentanoyl-L-isoleucin-methylester-Hydrochlorid (Beispiel 60) und Boc-Phe-His-OH (Beispiel 48) analog Beispiel 12 hergestellt.

(+)FAB-MS: m/z 766 (M + H)

DC-System I: R_{f} = 0,37

HPLC-Wert, System II: Rₜ = 7,00 min (Isomer A)
Rt ₌ 7,10 min (Isomer B)

### Beispiel 62

### N-tert-Butoxycarbonyl-L-(4-nitrophenyl)-alanyl-(N'- methoxy)methylamid

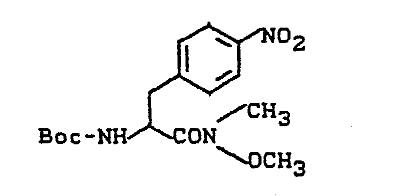

Die Titelverbindung wird nach der unter Beispiel 47 beschriebenen PPA-Kupplungsmethode aus 80 g (0,26 mol) N-tert-Butoxycarbonyl-L-(4-nitrophenyl)alanin (A.V. Schally et al., J. Med. Chem. 16, 828 (1973)) und 22,6 g (0,24 mol) N,0-Dimethylhydroxylamin Hydrochlorid (Ega) hergestellt.

Ausbeute: 78,2 g (95% der Theorie)

¹H-NMR (CDC1₃, 200 MHz): δ = 8,14 (d, 2H); 7,34 (d, 2H); 5,22 (d, 1H); 4,95 (m, 1H); 3,72 (s, 3H); 3,19 (s, 3H); 3,15 (m, 1H); 2,95 (m, 1H); 1,36 (s, 9H).

DC-System I : Rf = 0,76

DC-System II: R_{f} = 0,87

### Beispiel 63

### N-tert-Butoxycarbonyl-4S-amino-3RS-hydroxy-5-(4-nitrophenyl)pentansäure-(2-ethoxy)ethylester

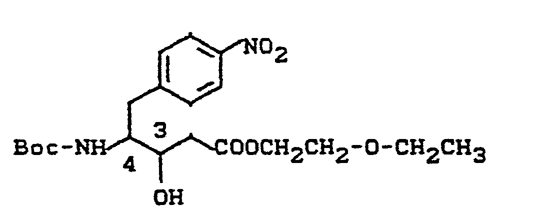

Die Titelverbindung wird analog Beispiel 6 aus 9 g (31 mmol) N-tert-Butoxycarbonyl-L-(4-nitrophenyl)alaninal (Beispiel 5) und dem Lithiumsalz des Essigsäure-(2-ethoxy) ethylesters hergestellt.

Rohausbeute: 12,2 g (93% der Theorie)

DC-System II: R_{f} = 0,50 (Isomeren nicht unterscheidbar)

HPLC-Wert, System II: R_{f m} 3,93 (Isomer A)
R_{f} = 4,35 (Isomer B)

im Verhältnis 1,1 : 1

### Beispiel 64

### N-tert-Butoxycarbonyl-4S-amino-3RS-hydroxy-5-(4-nitrophenyl)pentansäure

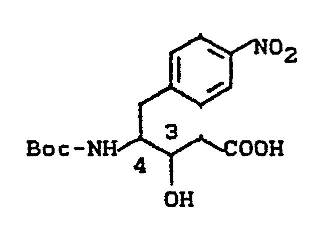

Die Titelverbindung wird analog Beispiel 9 aus 6,7 g (16 mmol) N-tert-Butoxycarbonyl-4S-amino-3RS-hydroxy-5-(4-nitrophenyl)pentansäure-ethoxyethylester (Beispiel 63) hergestellt.

Ausbeute: 4 g (71,5% der Theorie)

### Beispiel 65

### N-tert-Butoxycarbonyl-L-leucyl-benzyl-amid

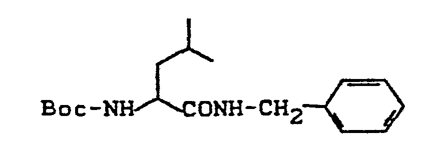

Die Titelverbindung wird nach der in Beispiel 37 bzw. 47 beschriebenen PPA-Kupplungsmethode aus 46 g (0,2 mol) N-tert-Butoxycarbonyl-L-leucin und 24 ml (0,22 mol) Benzylamin hergestellt.

Ausbeute: 32,1 g (53% der Theorie)

¹H-NMR (DMSO, 250 MHz): δ = 8,32 (m, 1H); 7,22 (m, SH); 6,88 (d, 1H); 4,29 (d, 2H); 4,02 (m, Ih); 1,30 - 1,70 (m, 3H); 1,38 (s, 9H); 0,86 (m, 6H).

DC-System I: R_{f} = 0,73

### Beispiel 66

### L-Leucyl-benzylamid Hydrochlorid

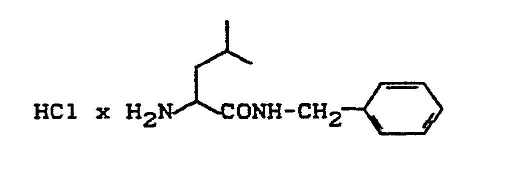

Die Titelverbindung wird aus 32 g (0,1 mol) N-tert-Butoxycarbonyl-L-leucyl-benzylamid (Beispiel 65) durch Abspaltung der tert-Butoxycarbonylschutzgruppe (analog Beispiel 11) hergestellt.

Ausbeute: 23,6 g (92% der Theorie)

DC-System XI: R_{f =} 0,75

### Beispiel 67

### N-tert-Butoxycarbonyl-4S-amino-3RS-hydroxy-5-(4-nitrophenyl)pentanoyl-L-leucyl-benzylamid

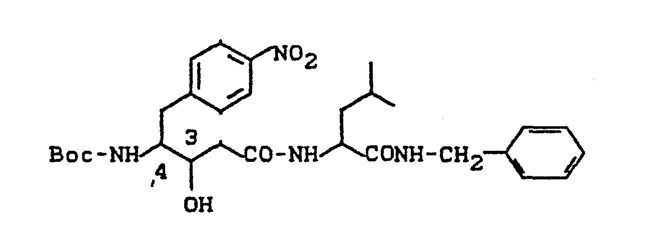

Die Titelverbindung wird nach der PPA-Methode (analog Beispiel 47) aus 6,1 g (17,3 mmol) N-tert-Butoxycarbonyl-4S-amino-3RS-hydroxy-5-(4-nitrophenyl)pentansäure (Beispiel 64) und 5,3 g (20,8 mmol) L-Leucyl-benzylamid Hydrochlorid (Beispiel 66) hergestellt.

Ausbeute: 4,2 g (44% der Theorie)

(+)FAB-MS: m/z 557 (M+H); m/z 579 (M + Na)

DC-System XI: R_{f} = 0,67

HPLC-Wert, System II: Rₜ = 6,7 min (Isomer A)
R_{t =} 7,7 min (Isomer B)

### Beispiel 68

### 4S-Amino-3RS-hydroxy-5-(4-nitrophenyl)pentanoyl-L-leucyl-benzylamid-Hydrochlorid

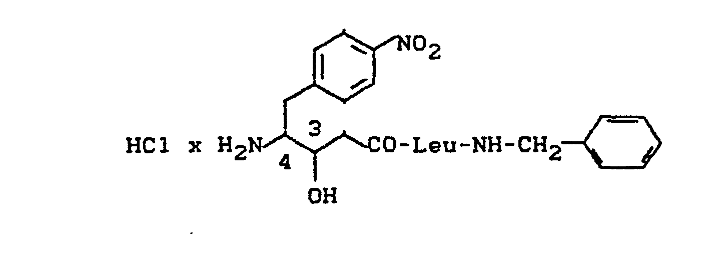

Die Titelverbindung wird aus 4,2 g (7,6 mmol) N-tert-Butoxycarbonyl-4S-amino-3RS-hydroxy-5-(4-nitrophenyl)-pentanoyl-L-leucyl-benzylamid (Beispiel 67) durch die analog Beispiel 11 durchgeführte Abspaltung der Butoxycarbonylschutzgruppe erhalten.

Ausbeute: 4 g (107X der Theorie, hygroskopisch) DC-System XI: R_{f =} 0,5

HPLC-Wert, System II: Rₜ = 7,61 min (Isomere nicht unterscheidbbar)

### Beispiel 69

### N-tert-Butoxycarbonyl-L-phenylalanyl-L-histidyl-4S-amino-3RS-hydroxy-5-(4-nitrophenyl)pentanoyl-L-leucyl-benzylamid

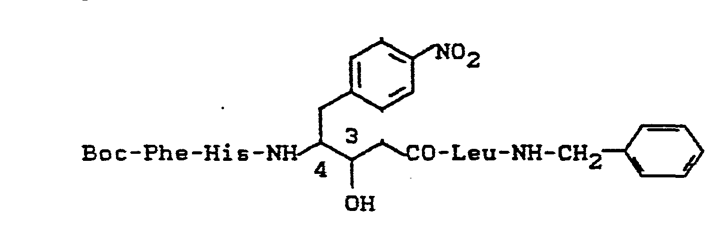

Die Titelverbindung wird analog Beispiel 12 aus N-tert-Butoxycarbonyl-L-phenylalanyl-L-histidin (Beispiel 48) und 4S-Amino-3RS-hydroxy-5-(4-nitrophenyl)pentanoyl-L-leucyl-benzylamid-Hydrochlorid (Beispiel 68) hergestellt. (+)FAB-MS: m/z 841 (M + H)

DC-System XI: R_{f =} 0,39

HPLC-Wert, System II: Rₜ = 8,5 min
beide Isomere (breiter Peak)

### Beispiel 70

### N-Benzyloxycarbonyl-L-phenylalanyl-L-histidyl-4S-amino-3RS-hydroxy-5-(4-nitrophenyl)pentanoyl-L-leucyl-benzyl- amid

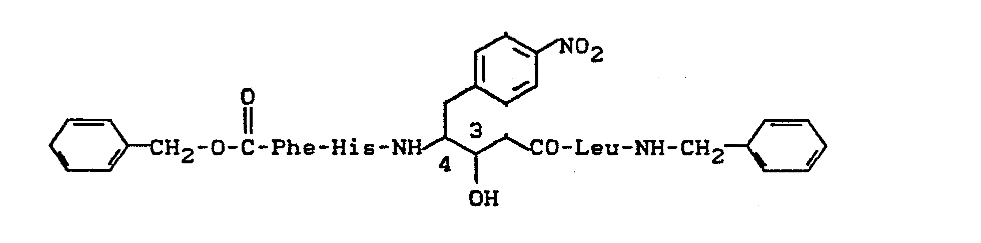

Die Titelverbindung wird analog Beispiel 12 aus N-Benzyl- oxycarbonyl-L-phenylalanyl-L-histidin (erhältlich durch Kupplung von Z-L-Phe und His-OCH₃ - analog Beispiel 47 - und nachfolgende Verseifung des Produktes (analog Beispiel 48)) und 4S-Amino-3RS-hydroxy-5-(4-nitrophenyl)-pentanoyl-L-leucyl-benzylamid-Hydrochlorid (Beispiel 68) hergestellt.

(+)FAB-MS: m/z 875 (M + H), m/z 897 (M + Na)

DC-System XI: R_{f} = 0,45

HPLC-Wert, System II: Rₜ = 10,3 min,
beide Isomeren (breiter Peak)

### Beispiel 71

### Nα, N_{T}-Di-tert-Butoxycarbonyl-L-histidyl-4S-amino-3S-hydroxy-5-(4-nitrophenyl)pentanoyl-L-isoleucin-methylester

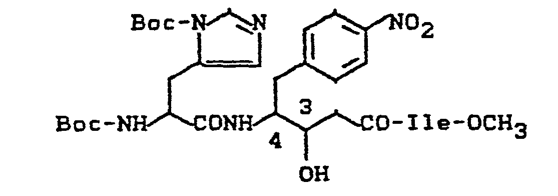

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und Di-tert-Butoxycarbonyl-L-histidin hergestellt.

(+)FAB-MS: m/z 719 (M + H); m/z 619

DC-System I : R_{f} = 0,64

HPLC-Wert, System II: Rₜ = 13,47 min

### Beispiel 72

### L-Histidyl-4S-amino-3S-hydroxy-5-(4-nitrophenyl)pentanoyl-L-isoleucin-methylester Dihydrochlorid

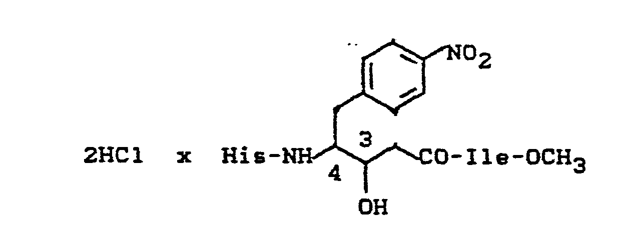

Die Titelverbindung wird analog Beispiel 11 durch saure Abspaltung der Schutzgruppen aus Nα, Nτ-Di-tert-Butoxycarbonyl-L-histidyl-4S-amino-3S-hydroxy-5-(4-nitrophenyl)pentanoyl-L-isoleucin-methylester (Beispiel 71) erhalten.

(+)FAB-MS: m/z 519 (M + H)

DC-System I : R_{f} = 0
IV: R_{f} = 0,36
XI: R_{f} = 0,21

### Beispiel 73

### N-p-Tolylsulfonyl-L-phenylalanyl-L-histidyl-4S-amino-3S-hydroxy-5-(4-nitrophenyl)pentanoyl-L-isoleucin-methylester

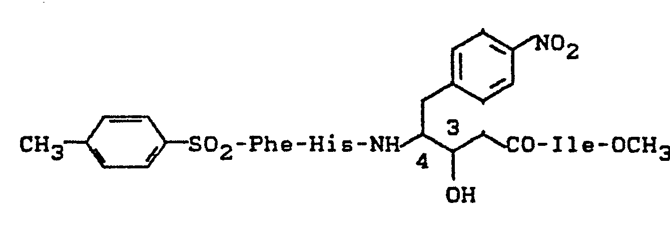

Die Titelverbindung wird ausgehend von L-Histidyl-4S-amino-3S-hydroxy-5-(4-nitrophenyl)pentanoyl-L-isoleucin-- methylester Dihydrochlorid (Beispiel 72) und N-Tosyl-L-phenylalanin nach der PPA-Kupplungsmethode (analog Beispiel 37 und 47) erhalten.

(+)FAB-MS: m/z 820 (M + H); m/z 842 (M + Na)

DC-System I : R_{f} = 0,53
IV: R_{f} = 0,98
XI: R_{f} = 0,49

HPLC-Wert, System II: Rₜ = 6,80 min

### Beispiel 74

### N-Phtalyl-L-phenylalanyl-L-histidyl-4S-amino-3S-hydroxy-5-(4-nitrophenyl)pentanoyl-L-isoleucin-methylester

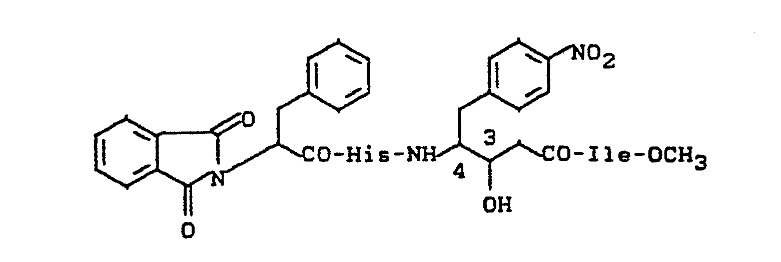

Die Titelverbindung wird ausgehend von L-Histidyl-4S-amino-3S-hydroxy-5-(4-nitrophenyl)pentanoyl-L-isoleucin- methylester Dihydrochlorid (Beispiel 72) und N-Phtalyl-L-phenylalanin nach der PPA-Kupplungsmethode (analog Beispiel 37 und 47) erhalten.

(+)FAB-MS: m/z 796 (M + H)

DC-System I : R_{f} = 0,51
IV: R_{f} = 0,98
XI: R_{f} = 0,46

HPLC-Wert, System II: R_{f} = 6,51 min

### Beispiel 75

### N-tert-Butoxycarbonyl-L-(4-Iodophenyl)alanyl-L-histidyl-4S-amino-3S-hydroxy-5-(4-nitrophenyl)pentanoyl-L-isoleucin-methylester

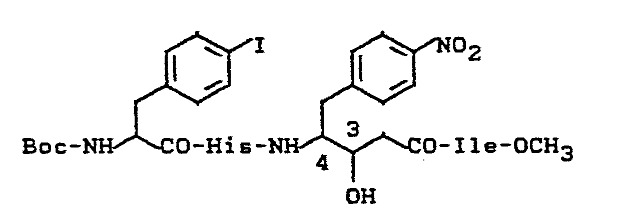

Die Titelverbindung wird ausgehend von L-Histidyl-4S-amino-3S-hydroxy-5-(4-nitrophenyl)pentanoyl-L-isoleucin- methylester Dihydrochlorid und N-tert-Butoxycarbonyl-L-(4-iodophenyl)alanin nach der PPA-Kupplungsmethode (analog Beispiel 37 und 47) erhalten.

(+) FAB-MS: m/z 892 (M + H); m/z 914 (M + Na)

DC-System XI: R_{f} = 0,48

HPLC-Wert, System II: Rₜ = 13,64 min

### Beispiel 76

### L-Phenylalanyl-L-histidyl-4S-amino-3S-hydroxy-5-(4-nitrophenyl)pentanoyl-L-isoleucin-methylester-Dihydrochlorid

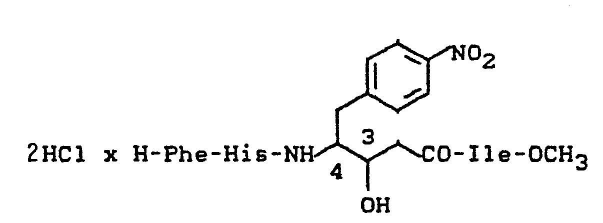

Die Titelverbindung wird durch Abspaltung der Butoxycarbonylschutzgruppe - analog Beispiel 11 - aus N-tert-Butoxycarbonyl-L-phenylalanyl-L-histidyl-4S-amino-3S-hydroxy-5-(4-nitrophenyl)pentanoyl-L-isoleucin-methylester (Beispiel 12) erhalten.

(+) FAB-MS: m/z 666 (M + H)

DC-System I : R_{f =} 0,52

DC-System XI: R_{f} = 0,42

HPLC-Wert, System II: Rₜ = 4,39 min

### Beispiel 77

### N-Acetyl-L-phenylalanyl-L-histidyl-4S-amino-3S-hydroxy-5-(4-nitrophenyl)pentanoyl-L-isoleucin-methylester

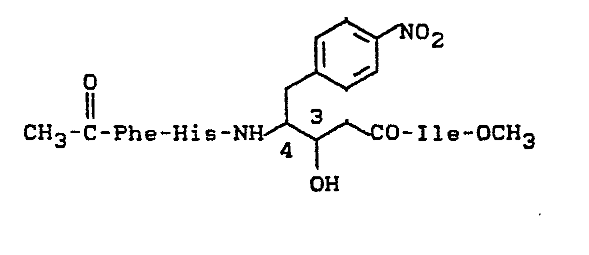

480 mg (0,65 mmol) des L-Phenylalanyl-L-histidyl-4S-amino- 3S-hydroxy-5-(4-nitrophenyl)pentanoyl-L-isoleucin-methyl- j ester-Dihydrochlorids (Beispiel 76) werden in 10 ml CH₂Cl₂ gelöst, mit 115 µl (1,43 mmol) Pyridin, 200 µl (1,43 mmol) Triethylamin und schließlich 68 µl (0,715 mmol) Acetanhydrid versetzt und über Nacht bei Raumtemperatur gerührt. Nach Zusatz von 10 ml CH₂C1₂ wird der Ansatz mit 0,1 N HCl bis pH 6,5 gebracht, mit Natriumhydrogencarbonatlösung und Wasser gewaschen. Die organische Phase wird mit Natriumsulfat getrocknet und eingeengt. Das nach dem Kristallisieren aus Ether erhaltene Rohprodukt (296,3 mg = 64X der Theorie) wird durch präparative Dickschichtchromatographie (PDC) an Merck 20 x 20 cm PSC-Fertigplatten, Kieselgel 60 F₂₅₄, Schichthöhe 2 mm, Kat.-Nr. 5717 mit dem Laufmittelsystem XI (CH₂Cl₂/MeOH/NH₃ = 9/1/0,2) aufgetrennt. Die die Substanzen enthaltende Zone wird herauspräpariert und mit CH₂Cl₂/MeOH 9/1 extrahiert. Nach dem Einengen des Filtrates erhält man 92,9 mg der Titelverbindung.

Ausbeute: 92,9 mg (20% der Theorie)

(+)FAB-MS: m/z 708 (M + H)

DC-System I : R_{f} = 0,34

DC-System XI: R_{f} = 0,53

HPLC-Wert, System II: Rₜ = 2,60 min

### Beispiel 78

### N-Benzyloxycarbonyl-L-phenylalanyl-L-histidyl-4S-amino-3S-hydroxy-5-(4-nitrophenyl)pentanoyl-L-isoleucin-methylester

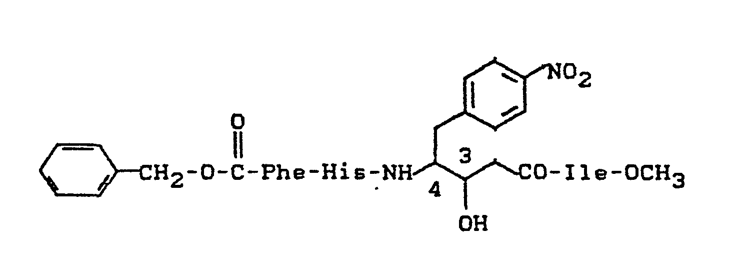

480 mg (0,65 mmol) des L-Phenylalanyl-L-histidyl-4S-amino-3S-hydroxy-5-(4-nitrophenyl)pentanoyl-L-isoleucin-methylester-Dihydrochlorids (Beispiel 76) werden in 10 ml THF durch Zugabe von 200 µl (1,43 mmol) Triethylamin gelöst. Nach Addition von 243 mg (0,975 mol) N-(Benzyloxycarbonyl- oxy)succinimid wird die Mischung über Nacht bei Raumtemperatur gerührt. Nach Zugabe von 49 mg (0,65 mmol) Glycin wird noch eine weitere Stunde gerührt, dann verdünnt man den Ansatz mit 10 ml CH₂C1₂ und arbeitet analog Beispiel. 77 auf. Nach PDC des Rohproduktes (289,9 mg = 56X der Theorie) erhält man 81 mg der Titelverbindung.

Ausbeute: 81,1 mg (15% der Theorie)

(+)FAB-MS: m/z 800 (M + H); m/z 822 (M + Na)

DC-System I: R_{f} = 0,62

DC-System XI: R_{f} = 0,53

HPLC-Wert, System II: Rₜ = 7,38 min

### Beispiel 79

### N-Phenoxyacetyl-L-phenylalanyl-L-histidyl-4S-amino-3S-hydroxy-5-(4-nitrophenyl)pentanoyl-L-isoleucin- methylester

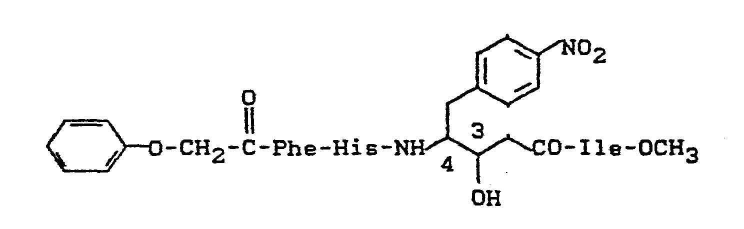

Die Titelverbindung wird aus L-Phenylalanyl-L-histidyl-4S-amino-3S-hydroxy-5-(4-nitrophenyl)pentanoyl-L-isoleucin- methylester-Dihydrochlorid (Bespiel 76) durch DCC/HOBT-Kupplung (wie in Beispiel 12 beschrieben) mit Phenoxyessigsäure erhalten. Nach PDC - analog Beispiel 77 - fällt die Verbindung analysenrein an.

(+)FAB-MS: m/z 800 (M + H)

DC-System I : R_{f} = 0,32

DC-System XI: R_{f} = 0,51

HPLC-Wert, System II: Rₜ = 6,59 min

### Beispiel 80

### N-Isovaleroyl-L-phenylalanyl-L-histidyl-4S-amino-3S-hydroxy-5-(4-nitrophenyl)pentanoyl-L-isoleucin-methylester

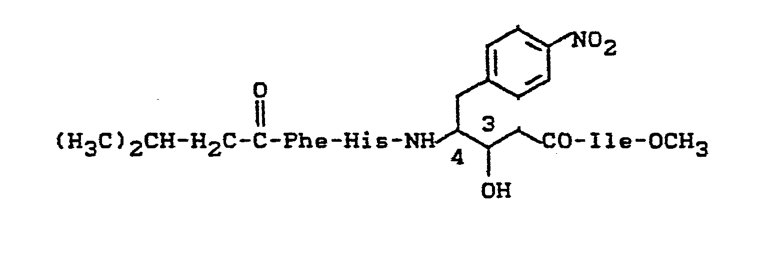

Die Titelverbindung wird aus L-Phenylalanyl-L-histidyl-4S-amino-3S-hydroxy-5-(4-nitrophenyl)pentanoyl-L-isoleucin- methylester-Dihydrochlorid (Beispiel 76) und Isovaleriansäure durch Kupplung mit Propanphosphonsäureanhydrid (PPA, analog Beispiel 37 und 47) erhalten.

(+)FAB-MS: m/z 750 (M + H)

DC-System I : R_{f} = 0,40

DC-System XI: R_{f} = 0,51

HPLC-Wert, System II: R_{t =} 5,01 min

### Beispiel 81

### N-tert-Butoxycarbonyl-L-phenylalanyl-L-phenylalanyl-L-histidyl-4S-amino-3S-hydroxy-5-(4-nitrophenyl)pentanoyl-L-isoleucin-methylester

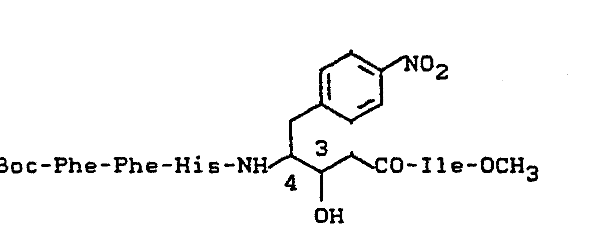

Die Titelverbindung wird aus L-Phenylalanyl-L-histidyl-4S-amino-35-hydroxy-5-(4-nitrophenyl)pentanoyl-L-isoleucin- methylester-Dihydrochlorid (Beispiel 76) und N-tert-Butoxycarbonyl-L-phenylalanin nach der in Beispiel 47 beschriebenen PPA-Kupplungsmethode erhalten.

(+)FAB-MS: mlz 913 (M + H); m/z 935 (M + Na)

DC-System I : R_{f} = 0,35

DC-System XI: R_{f =} 0,54

HPLC-Wert, System II: Rₜ = 12,70 min

### Beispiel 82

### N-tert-Butoxycarbonyl-L-phenylalanyl-L-histidyl-4S-amino-3S-hydroxy-5-(4-nitrophenyl)pentanoyl-L-isoleucin

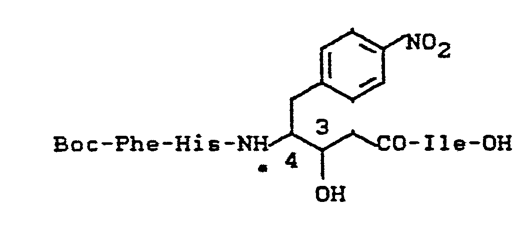

Die Titelverbindung wird aus N-tert-Butoxycarbonyl-L- - phenylalanyl-L-histidyl-4S-amino-3S-hydroxy-5-(4-nitrophenyl)pentanoyl-L-isoleucin methylester (Beispiel 12) durch alkalische Verseifung (wie in Beispiel 48 beschrieben) hergestellt.

(+)FAB-MS: m/z 752 (M + H); m/z 774 (M + Na)

DC-System I : R_{f} = 0,25

DC-System II: R_{f} = 0,05

### Beispiel 83

### N-tert-Butoxycarbonyl-L-phenylalanyl-L-histidyl-4S-amino-3S-hydroxy-5-(4-nitrophenyl)pentanoyl-D,L-isoleucin-isopropylester

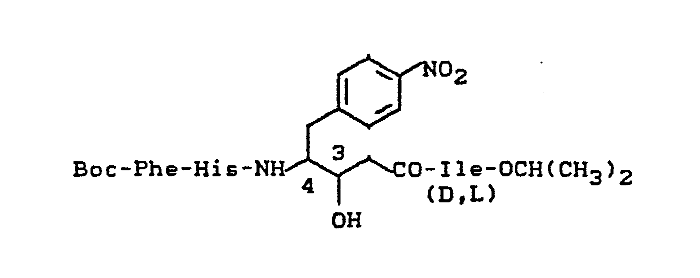

350 mg (0,5 mmol) N-tert-Butoxycarbonyl-L-phenylalanyl-L-histidyl-4S-amino-3S-hydroxy-5-(4-nitrophenyl)pentanoyl-L-isoleucin (Beispiel 82) werden in 10 ml Methylenchlorid gelöst. Dazu gibt man 105 mg DCC (110 mol-X), 33 µl Isopropanol (110 mol-X) und schließlich 60 mg (100 mol-X) Dimethylaminopyridin zu und läßt den Ansatz 2 Tage bei Raumtemperatur rühren. Die Mischung wird mit 10 ml Methylenchlorid verdünnt und nacheinander mit Natriumhydrogencarbonat-, Natriumchlorid-, und Natriumhydrogensulfatlösung gewaschen. Das nach dem Einengen der organischen Phase erhaltene Rohprodukt wird durch isokratische Hochdruckflüssigkeitschromatographie an einer Merck LiChrosorb RP-8 Säule, 7 um, Kat-Nr. 51 441 mit dem Laufmittelgemisch Acetonitril / 10 mmol Ammoniumacetat (mit NH₃ auf pH 8,2 eingestellt) 50150 aufgetrennt. Die die beiden Isomeren enthaltenden Fraktionen werden zusammengefaßt und lyophilisiert.

(+)FAB-MS: m/z 794 (M + H)

DC-System I : R_{f =} 0,54

DC-System II: R_{f =} 0,47

HPLC-Wert, System II: Rₜ = 12,58 (Isomer A)
R_{t =} 13,86 (Isomer B)

### Beispiel 84

### N-tert-Butoxycarbonyl-L-(3,4-Dichlorphenyl)alanyl-L-histidyl-4S-amino-3S-hydroxy-5-(4-nitrophenyl)pentanoyl-L-isoleucin-methylester

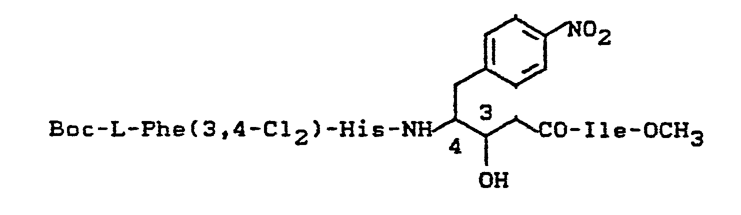

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und N-tert-Butoxycarbonyl-L-(3,4-dichlorphenyl)alanyl-L-histidin [erhältlich durch PPA-Kupplung von N-tert-Butoxycarbonyl-L-(3,4-di- chlorophenyl)alanin und Histidinmethylester (analog Beispiel 47) und nachfolgender Verseifung (analog Beispiel 48) des Produktes] hergestellt.

(+)FAB-MS: m/z 834 (M + H)

DC-System XI: R_{f =} 0,58

HPLC-Wert, System II: Rₜ = 14,55 min

### Beispiel 85

### N-tert-Butoxycarbonyl-L-(1-naphthyl)alanyl-L-histidyl-4S-amino-3S-hydroxy-5-(4-nitrophenyl)pentanoyl-L-isoleucin- methylester

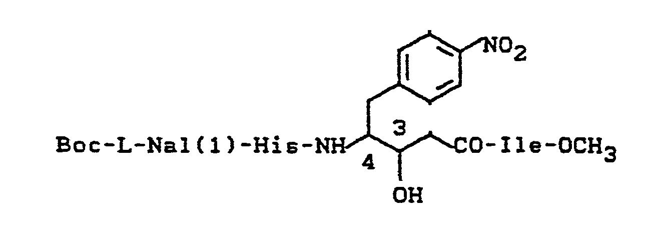

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und N-tert-Butoxycarbonyl-L-(1-naphthyl)alanyl-L-histidin [erhältlich durch PPA-Kupplung von N-tert-Butoxycarbonyl-L-(1-naphthyl)alanin und Histidinmethylester (analog Beispiel 47) und nachfolgender Verseifung (analog Beispiel 48) des Produktes] hergestellt.

(+)FAB-MS: m/z 816 (M + H)

DC-System XI: R_{f =} 0,56

HPLC-Wert, System II: Rₜ = 11,92 min

### Beispiel 86

### N-tert-Butoxycarbonyl-L-(4-nitrophenyl)alanyl-L-histidyl-4S-amino-3S-hydroxy-5-(4-nitrophenyl)pentanoyl-L-isoleucin-methylester

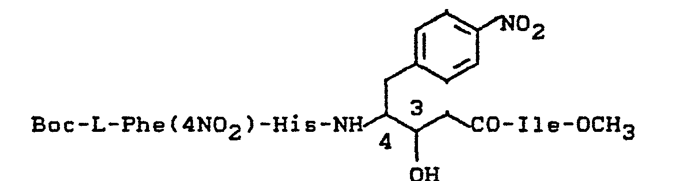

Die Titelverbindung wird analog Beispiel 12 aus 4S-Amino-3S-hydroxy-5-(4-nitrophenyl)pentanoyl-L-isoleucin-methylester Hydrochlorid (Beispiel 11) und N-tert-Butoxycarbonyl-L-(4-nitro phenyl)alanyl-L-histidin [erhältlich durch PPA-Kupplung von N-tert-Butoxycarbonyl-L-(4-nitrophenyl)alanin und Histidinmethylester (analog Beispiel 47) und nachfolgender Verseifung (analog Beispiel 48) des Produktes] hergestellt.

(+)FAB-MS: mlz 811 (M + H)

DC-System XI: R_{f} = 0,48

HPLC-Wert, System II: R_{t =} 6,71 min

### Beispiel 87

### N-tert-Butoxycarbonyl-D,L-phenylglycyl-L-histidyl-4S-amino-3S-hydroxy-5-(4-nitrophenyl)pentanoyl-L-isoleucin- methylester

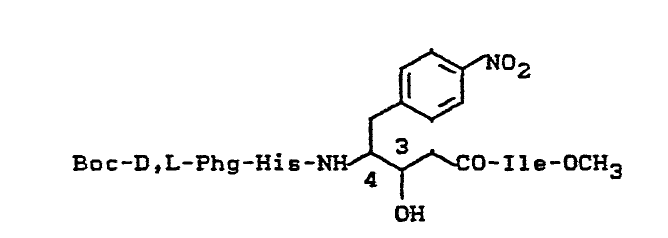

Die Titelverbindung wird analog Beispiel 12 aus 4S-Amino-3S-hydroxy-5-(4-nitrophenyl)pentanoyl-L-isoleucin-methylester Hydrochlorid (Beispiel 11) und N-tert-Butoxycarbonyl-D,L-(phenyl)glycyl-L-histidin [erhältlich durch PPA-Kupplung von N-tert-Butoxycarbonyl-D,L-phenylglycin und Histidinmethylester (analog Beispiel 47) und nachfolgender Verseifung (analog Beispiel 48) des Produktes] hergestellt.

(+)FAB-MS: mIz 752 (M + H); m/z 766 (M + Na)

DC-System XI: R_{f} = 0,52 (Isomer A) und 0,50 (Isomer B)

HPLC-Wert, System II: Rₜ = 5,46 min, ununterscheidbar

### Beispiel 88

### N-tert-Butoxycarbonyl-L-prolyl-L-phenylalanyl-L-histidyl-4S-amino-3S-hydroxy-5-(4-nitrophenyl)pentanoyl-L-isoleucin-methylester

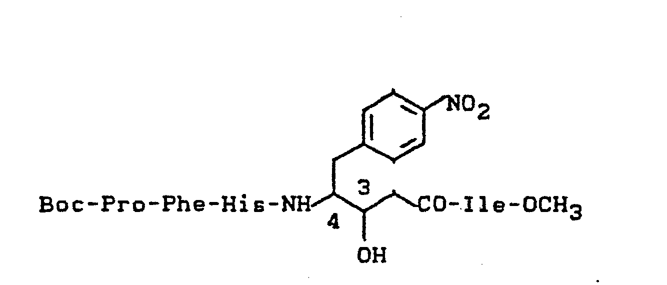

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und N-tert-Butoxycarbonyl-L-prolyl-L-phenylalanyl-L-histidin [erhältlich durch Abspalten der Schutzgruppe aus Beispiel 47 (analog Beispiel 11), nachfolgender Umsetzung des Produktes mit N-tert-Butoxycarbonyl-L-prolin (analog Beispiel 47) und anschließende Verseifung des Kupplungsproduktes (analog Beispiel 48)] hergestellt.

(+)FAB-MS: m/z 863 (M + H)

DC-System XI: R_{f} = 0,52

HPLC-Wert, System II: Rₜ = 7,87 min

### Beispiel 89

### N-Ethoxycarbonyl-L-phenylalanyl-L-histidyl-4S-amino-3S-hydroxy-5-(4-nitrophenyl)pentanoyl-L-isoleucin-methylester

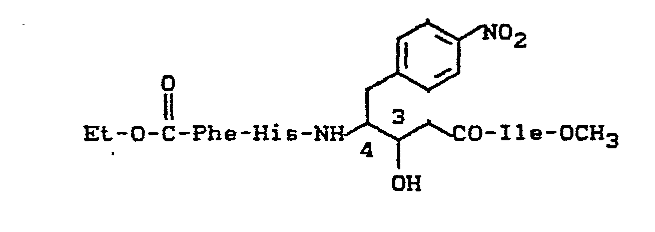

Die Titelverbindung wird analog Beispiel 12 aus 4S-Amino-3S-hydroxy-5-(4-nitrophenyl)pentanoyl-L-isoleucin-methylester Hydrochlorid (Beispiel 11) und N-Ethoxycarbonyl-L-phenylalanyl-L-histidin [erhältlich durch PPA-Kupplung von N-Ethoxycarbonyl-L-phenylalanin und Histidinmethylester (analog Beispiel 47) und nachfolgender Verseifung (analog Beispiel 48) des Produktes] hergestellt.

(+)FAB-MS: m/z 738 (M + H)

DC-System XI: R_{f} = 0,51

HPLC-Wert, System II: Rₜ = 4,33 min

### Beispiel 90

### N-Benzoyl-L-phenylalanyl-L-histidyl-4S-amino-3S-hydroxy-5-(4-nitrophenyl)pentanoyl-L-isoleucin-methylester

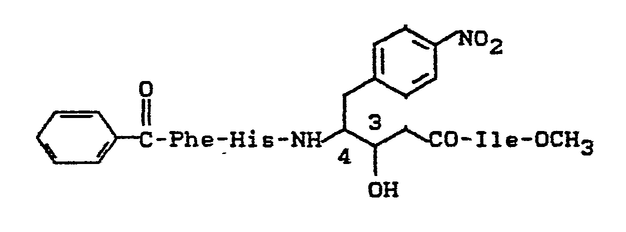

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und N-Benzoyl-L-phenylalanyl-L-histidin [erhältlich durch PPA-Kupplung von N-Benzoyl-L-phenylalanin und Histidinmethylester (analog Beispiel 47) und nachfolgender Verseifung (analog Beispiel 48) des Produktes] hergestellt.

(+)FAB-MS: m/z 770 (M + H)

DC-System XI: R_{f} = 0,48

HPLC-Wert, System II: R_{f} = 5,72 min

### Beispiel 91

### N-tert-Butoxycarbonyl-D-phenylalanyl-L-histidyl-4S-amino-3S-hydroxy-5-(4-nitrophenyl)pentanoyl-L-isoleucin- methylester

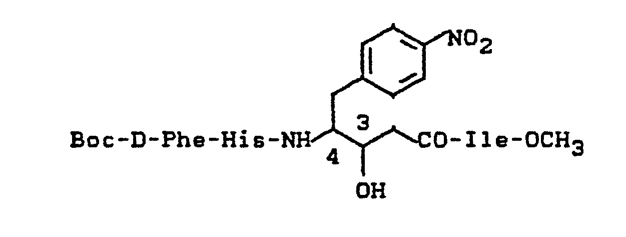

Die Titelverbindung wird analog Beispiel 12 aus 4S-Amino-3S-hydroxy-5-(4-nitrophenyl)pentanoyl-L-isoleucin-methylester-Hydrochlorid (Beispiel 11) und N-tert-Butoxycarbonyl-D-phenylalanyl-L-histidin [erhältlich durch PPA-Kupplung von N-tert-Butoxycarbonyl-D-phenylalanin und Histidinmethylester (analog Beispiel 47) und nachfolgender Verseifung (analog Beispiel 48) des Produktes] hergestellt.

(+)FAB-MS: m/z 766 (M + H)

DC-System XI: R_{f} = 0,44

HPLC-Wert, System II: Rₜ = 7,13 min

### Beispiel 92

### N-tert-Butoxycarbonyl-L-phenyl-alanyl-L-histidyl-4S-amino-3RS-hydroxy-5-(3-aminophenyl)pentanoyl-L-isoleucin-methylester

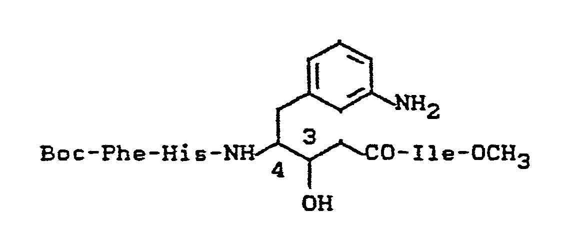

Die Titelverbindung wird analog Beispiel 42 durch katalytische Reduktion aus N-tert-Butoxycarbonyl-L-phenylalanyl-L-histidyl-4S-amino-3RS-hydroxy-5-(3-nitrophenyl)pentanoyl-L-isoleucin-methylester (Beispiel 61) erhalten.

(+) FAB-MS: 736 (M + H)

### Beispiel 93

### N-tert-Butoxycarbonyl-4S-amino-3RS-hydroxy-5-(4-nitrophenyl)pentanoyl-L-isoleucin-methylester

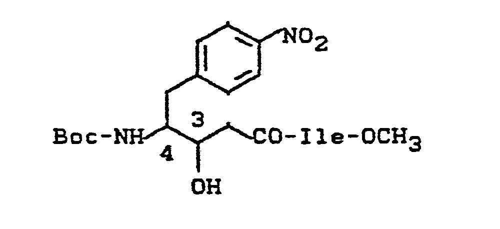

Die Titelverbindung wird ausgehend von N-tert-Butoxycarbonyl-4S-amino-3RS-hydroxy-5-(4-nitrophenyl)pentansäure (Beispiel 64) und Isoleucinmethylester analog Beispiel 10 erhalten.

### Beispiel 94

### 4S-Amino-3RS-hydroxy-5-(4-nitrophenyl)pentanoyl-L-isoleucin-methylester Hydrochlorid

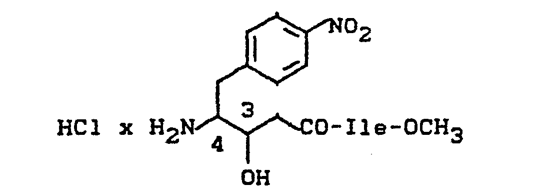

Die Titelverbindung wird analog Beispiel 11 aus N-tert-Butoxycarbonyl-4S-amino-3RS-hydroxy-5-(4-nitrophenyl)-pentanoyl-L-isoleucin-methylester (Beispiel 93) erhaltent

### Beispiel 95

### N-tert-Butoxycarbonyl-L-tyrosyl-L-histidyl-4S-amino-3RS- hydroxy-5-(4-nitrophenyl)pentanoyl-L-isoleucin-methylester

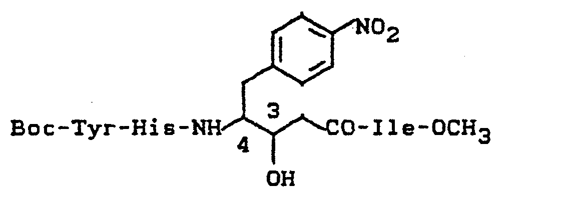

Die Titelverbindung wird analog Beispiel 12 aus 4S-Amino-3RS-hydroxy-5-(4-nitrophenyl)pentanoyl-L-isoleucin-methylester Hydrochlorid (Beispiel 94) und N-tert-Butoxycarbonyl-L-tyrosyl-L-histidin [erhältlich durch PPA-Kupplung von N-tert-Butoxycarbonyl-L-tyrosin und Histidinmethylester (analog Beispiel 47) und nachfolgender Verseifung (analog Beispiel 48) des Produktes] hergestellt. (+)FAB-MS: m/z 782 (M + H)

DC-System XII: R_{f} = 0,25

HPLC-Wert, System II: Rₜ = 3,08 min

### Beispiel 96

### N-tert-Butoxycarbonyl-L-(4-methoxyphenyl)alanyl-L-histidyl-4S-amino-3RS-hydroxy-5-(4-nitrophenyl)pentanoyl-L-isoleucin-methylester

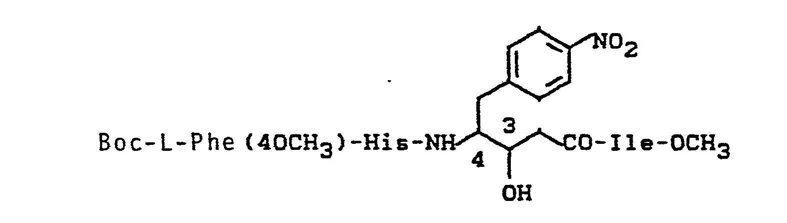

Die Titelverbindung wird analog Beispiel 12 aus 4S-Amino-3RS-hydroxy-5-(4-nitrophenyl)pentanoyl-L-isoleucin-methyi- ester Hydrochlorid (Beispiel 94) und N-tert-Butoxycarbonyl-L-(4-methoxyphenyl)alanyl-L-histidin [erhältlich durch PPA-Kupplung von N-tert-Butoxycarbonyl-L-(4-methoxyphenyl)alanin und Histidinmethylester (analog Beispiel 47) und nachfolgender Verseifung (analog Beispiel 48) des Produktesl hergestellt.

(+)FAB-MS: m/z 796 (M + H)

DC-System XII: R_{f} = 0,33

HPLC-Wert, System II: Rₜ = 6,05 min

### Beispiel 97

### N-tert-Butoxycarbonyl-L-prolyl-L-(4-methoxyphenyl)alanyl-L-histidyl-4S-amino-3RS-hydroxy-5-(4-nitrophenyl)pentanoyl-L-isoleucin-methylester

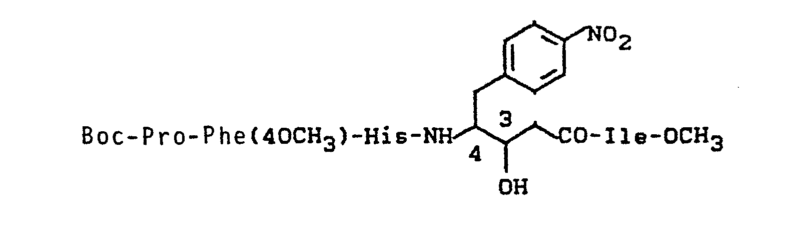

Die Titelverbindung wird durch Abspalten der N-terminalen Schutzgruppe (analog Beispiel 76) und nachfolgender Kupplung mit N-tert-Butoxycarbonyl-L-prolin (analog Beispiel 81) aus N-tert-Butoxycarbonyl-L-(4-methoxyphenyl)alanyl-L-histidyl-4S-amino-3RS-hydroxy-(4-nitrophenyl)pentanoyl-L-isoleucin-methylester (Beispiel 96) gewonnen.

(+)FAB-MS: m/z 893 (M + H)

DC-System XI: R_{f} = 0,45

HPLC-Wert, System II: Rₜ = 7,24 min

### Beispiel 98

### N-α-Phenoxyacetyl-L-histidyl-4S-amino-3RS-hydroxy-5-(4-nitrophenyl)pentanoyl-L-isoleucin-methylester

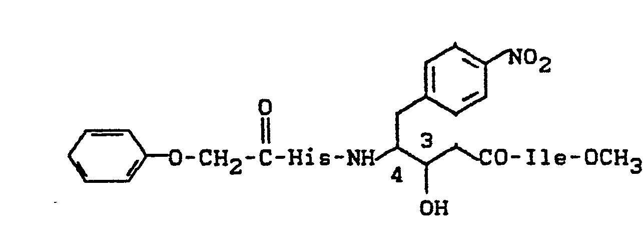

Die Titelverbindung wird analog Beispiel 12 aus 4S-Amino-3RS-hydroxy-5-(4-nitrophenyl)pentanoyl-L-isoleucin-methylester Hydrochlorid (Beispiel 94) und N-α-Phenoxyacetyl-L-histidin [erhältlich durch PPA-Kupplung von Phenoxyessigsäure und Histidinmethylester (analog Beispiel 47) und nachfolgender Verseifung (analog Beispiel 48) des Produktes] hergestellt.

(+) FAB-MS: m/z 653 (M + H)

DC-System XII: R_{f} = 0,25

HPLC-Wert, System II: Rₜ = 3,35 min

### Beispiel 99

### N-tert-Butoxycarbonyl-L-(4-methoxyphenyl)alanyl-L-histidyl-4S-amino-3RS-hydroxy-5-(4-aminophenyl)pentanoyl-L-isoleucin-methylester

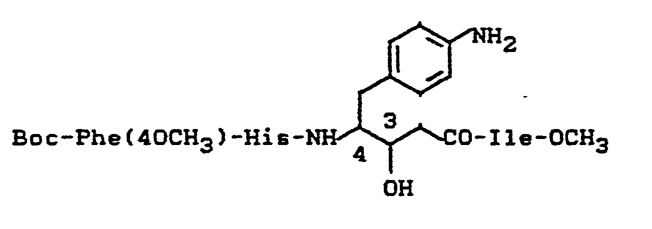

Die Titelverbindung wird durch katalytische Reduktion (analog Beispiel 27) aus N-tert-Butoxycarbonyl-L-(4-methoxyphenyl)alanyl-L-histidyl-4S-amino-3RS-hydroxy-5-(4-nitrophenyl)pentanoyl-L-isoleucin-methylester (Beispiel 96) erhalten.

(+) FAB-MS: m/z 766 (M + H)

HPLC-Wert, System II: Rₜ = 3,56 min

### Beispiel 100

### N-tert-Butoxycarbonyl-L-tyrosyl-L-histidyl-4S-amino-3RS- hydroxy-5-(4-aminophenyl)pentanoyl-L-isoleucin- methylester

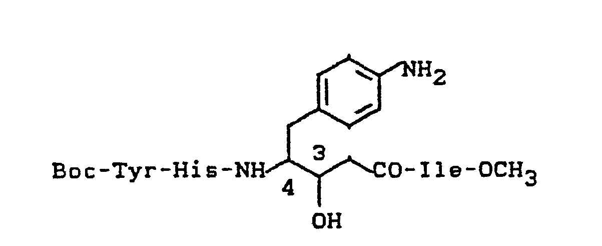

Die Titelverbindung wird analog Beispiel 99 aus N-tert-Butoxycarbonyl-L-tyrosyl-L-histidyl-4S-amino-3RS-hydroxy-5-(4-nitrophenyl)pentanoyl-L-isoleucin-methylester (Beispiel 95) erhalten.

(+)FAB-MS: m/z 752 (M + H)

HPLC-Wert, System II: Rₜ = 2,06 min

### Beispiel 101

### 4S-Amino-3S-hydroxy-5-(4-nitrophenyl)pentansäure-ethylester Hydrochlorid

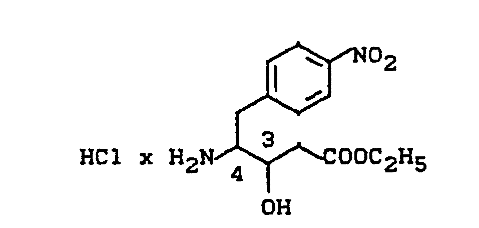

Die Titelverbindung wird ausgehend von N-tert-Butoxycarbonyl-4S-amino-3S-hydroxy-5-(4-nitrophenyl)penlansäure- ethylester (Beispiel 7) analog Beispiel 11 durch Abspalten der tert-Butoxycarbonylschutzgruppe erhalten.

DC-System I: R_{f} = 0,19

### Beispiel 102

### N-tert-Butoxycarbonyl-L-phenylalanyl-L-histidyl-4S-amino-3S-hydroxy-5-(4-nitrophenyl)pentansäure-ethylester

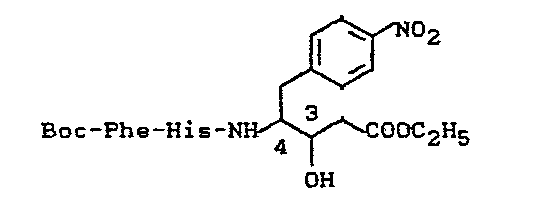

Die Titelverbindung wird ausgehend von N-tert-Butoxycarbnyl-L-phenylalanyl-L-histidin (Beispiel 48) und 4S-Amino-3S-hydroxy-5-(4-nitrophenyl)pentansäure-ethylester Hydrochlorid (Beispiel 101) durch DCC/HOBT-Kupplung und nachfolgender Chromatographie (analog Beispiel 12) erhalten.

DC-System I : R_{f} = 0,46

DC-System IV: R_{f} = 0,88

DC-System V : R_{f} = 0,61

DC-System XI: R_{f} = 0,43

HPLC-Wert, System II: Rₜ = 3,82 min

### Beispiel 103

### N-tert-Butoxycarbonyl-4S-amino-3S-hydroxy-5-(4-nitrophenyl)pentanoyl-L-leucinamid

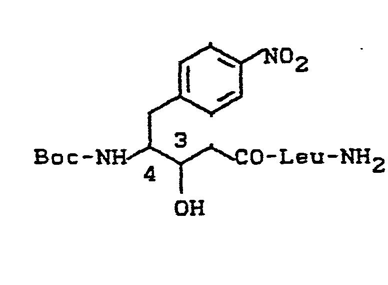

Die Titelverbindung wird ausgehend von N-tert-Butoxycarbonyl-4S-amino-3S-hydroxy-5-(4-nitrophenyl)pentansäure (Beispiel 9) und Leucinamid analog Beispiel 37 nach der PPA-Kupplungsmethode erhalten.

(+)FAB-MS: m/z 467 (M + H); m/z 489 (M + Na)

DC-System I : R_{f} = 0,40

HPLC-Wert, System II: R_{f =} 2,57 min

### Beispiel 104

### N-tert-Butoxycarbonyl-4S-amino-3S-hydroxy-5-(4-nitrophenyl)pentanoyl-L-phenylalanin-methylester

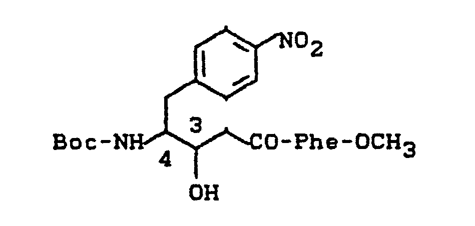

Die Titelverbindung wird analog Beispiel 103 aus N-tert-Butoxycarbonyl-4S-amino-3S-hydroxy-5-(4-nitrophenyl)pentansäure (Beispiel 9) und L-Phenylalaninmethylester Hydrochlorid erhalten.

(+) FAB-MS: mlz 516 (M + H); m/z 538 (M + Na)

DC-System I : R_{f} = 0,67

HPLC-Wert, System II: Rₜ = 7,14

### Beispiel 105

### N-tert-Butoxycarbonyl-4S-amino-3S-hydroxy-5-(4-nitrophenyl)pentanoyl-R(+)-α-methylbenzylamid

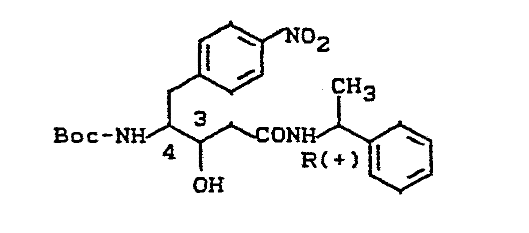

Die Titelverbindung wird analog Beispiel 103 aus N-tert-Butoxycarbonyl-4S-amino-3S-hydroxy-5-(4-nitrophenyl)-pentansäure (Beispiel 9) und R-(+1-α-methylbenzylamin erhalten.

(+) FAB-MS: m/z 458 (M + H); m/z 402, m/z 358

DC-System I : R_{f =} 0,61

HPLC-Wert, System II: Rₜ = 6,22 min

### Beispiel 106

### N-tert-Butoxycarbonyl-L-phenylalanyl-L-histidyl-4S-amino-3S-hydroxy-5-(4-nitrophenyl)pentanoyl-L-leucinamid

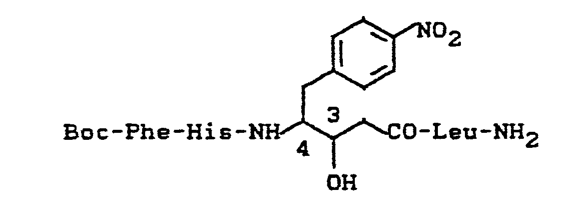

Die Titelverbindung wird ausgehend von N-tert-Butoxycarbonyl-4S-amino-3S-hydroxy-5-(4-nitrophenyl)pentanoyl-L-leucinamid (Beispiel 103) analog Beispiel 40 und Beispiel 41 durch Abspaltung der Schutzgruppe und nachfolgender Kupplung mit Boc-Phe-His-OH (Beispiel 48) erhalten. (+)FAB-MS: m/z 751 (M + H)

DC-System I : R_{f} = 0,64

HPLC-Wert, System II: Rₜ ⁼ 3,11

### Beispiel 107

### N-tert-Butoxycarbonyl-L-phenylalanyl-L-histidyl-4S-amino-3S-hydroxy-5-(4-nitrophenyl)pentanoyl-L-phenylalaninmethylester

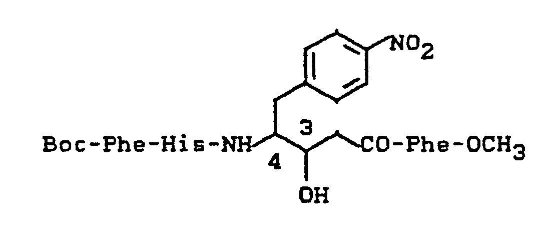

Die Titelverbindung wird ausgehend von N-tert-Butoxycarbonyl-4S-amino-3S-hydroxy-5-(4-nitrophenyl)pentanoyl-L-phenylalanin-methylester (Beispiel 104) analog Beispiel 40 und Beispiel 41 durch Abspaltung der Schutzgruppe und nachfolgender Kupplung mit Boc-Phe-His-OH (Beispiel 48) erhalten.

(+)FAB-MS: m/z 800 (M + H)

DC-System I : R_{f} = 0,72

HPLC-Wert, System II: Rₜ = 7,45 min

### Beispiel 108

### N-tert-Butoxycarbonyl-L-phenylalanyl-L-histidyl-4S-amino-3S-hydroxy-5-(4-nitrophenyl)pentanoyl-R-(+)-(a)-methylbenzylamid

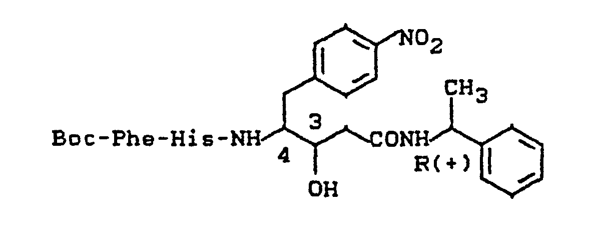

Die Titelverbindung wird ausgehend von N-tert-Butoxycarbonyl-4S-amino-3S-hydroxy-5-(4-nitrophenyl)pentanoyl-R(+)-α-methylbenzylamid (Beispiel 105) analog Beispiel 40 und Beispiel 41 durch Abspaltung der Schutzgruppe und nachfolgender Kupplung mit Boc-Phe-His-OH (Beispiel 48) erhalten.

DC-System I : Rf = 0,65

HPLC-Wert, System II: Rₜ = 6,65

### Beispiel 109

### N-tert-Butoxycarbonyl-4S-amino-3S-hydroxy-5-(4-nitrophenyl)pentanoyl-L-leucin-tert-butylester

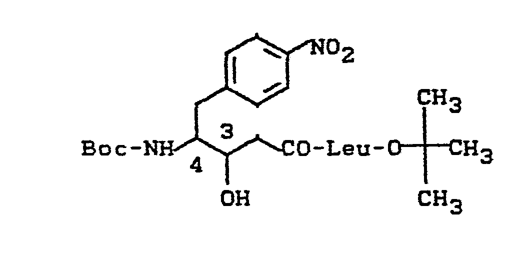

Die Titelverbindung wird ausgehend von N-tert-Butoxycarbonyl-4S-amino-3S-hydroxy-5-(4-nitrophenyl)pentansäure (Beispiel 9) und L-Leucin-tert-butylester Hydrochlorid analog Beispiel 37 nach der PPA-Kupplungsmethode erhalten. DC-System I: R_{f} = 0,76

HPLC-Wert, System II: Rₜ = 20,6 min

### Beispiel 110

### N-tert-Butoxycarbonyl-4S-amino-3S-hydroxy-5-(4-nitrophenyl)pentanoyl-L-leucin-benzylester

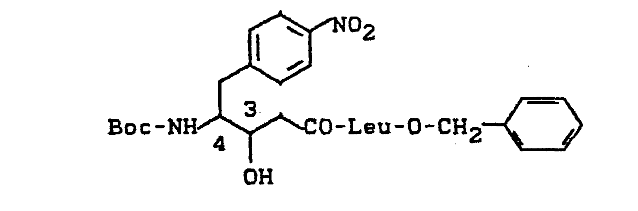

Die Titelverbindung wird ausgehend von N-tert-Butoxycarbonyl-4S-amino-3S-hydroxy-5-(4-nitrophenyl)pentansäure (Beispiel 9) und L-Leucinbenzylester analog Beispiel 37 nach der PPA-Kupplungsmethode erhalten.

DC-System I : R_{f =} 0,85

HPLC-Wert, System II: Rₜ = 19,9 min

### Beispiel 111

### N-tert-Butoxycarbonyl-L-phenylalanyl-L-histidyl-4S-amino-3S-hydroxy-5-(4-nitrophenyl)pentanoyl-L-leucin-tert-butylester

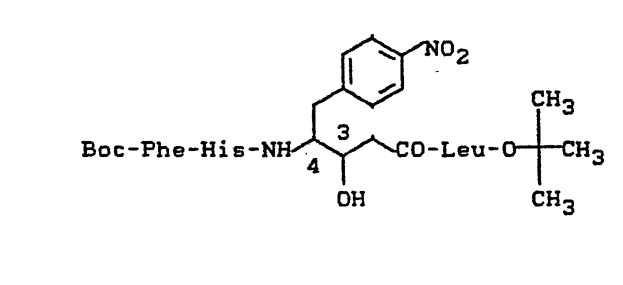

Die Titelverbindung wird aus 4S-Amino-3S-hydroxy-5-(4-nitrophenyl)pentanoyl-L-leucin-tert-butylester (Beispiel 113) und Boc-Phe-His-OH (Beispiel 48) analog Beispiel 12 hergestellt.

(+) FAB-MS: m/z 808 (M+H)

HPLC-Wert, System II: Rₜ = 13,22 min

### Beispiel 112

### N-tert-Butoxycarbonyl-L-phenylalanyl-L-histidyl-4S-amino-3S-hydroxy-5-(4-nitrophenyl)pentanoyl-L-leucin-benzylester

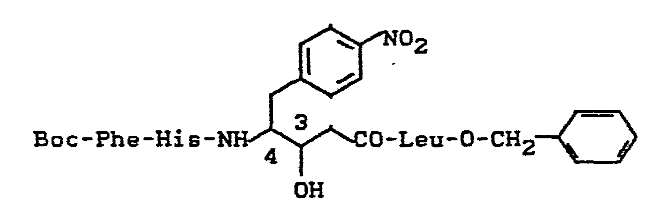

Die Titelverbindung wird ausgehend von N-tert-Butoxycarbonyl-4S-amino-3S-hydroxy-5-(4-nitrophenyl )pentanoyl-L-leucin-benzylester (Beispiel 110) analog Beispiel 40 und Beispiel 41 durch Abspaltung der Schutzgruppe und nachfolgender Kupplung mit Boc-Phe-His-OH (Beispiel 48) erhalten.

(+) FAB-MS: m/z 842 (M+H)

HPLC-Wert, System II: Rₜ = 14,66 min

### Beispiel 113

### 4S-Amino-3S-hydroxy-5-(4-nitrophenyl)-pentanoyl-leucin-tert-butylester

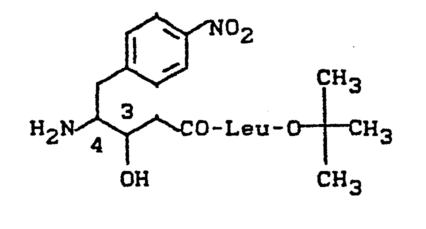

1,8 g (3,5 mmol) N-tert-Butoxycarbonyl-4S-amino-3-S-hydroxy-5-(4-nitrophenyl)pentanoyl-L-leucin-tert-butylester (Beispiel 109) werden in 15 ml CH₂C1 gelöst. Man kühlt auf 0°C, gibt 5,2 ml (1000 mol-X) Trifluoressigsäure zu der Mischung und rührt 1 Stunde bei dieser Temperatur. Man gießt den Ansatz auf 40 ml Wasser und trennt von der organischen Phase ab, die verworfen wird. Die wäßrige Phase wird mit verdünnter Natronlauge auf pH 9 gestellt und dann zweimal mit 20 ml Essigester extrahiert. Die vereinigte organische Phase wird mit 5 Xiger Natriumhydrogencarbonat- und gesättigter Natriumchloridlösung gewaschen, mit Natriumsulfat getrocknet und am Vakuum eingeengt.

Ausbeute: 1,02 g (70 % der Theorie)

(+) FAB-MS: m/z 424 (M+H); m/z 368

DC-System I:R_{f} = 0,39

### Beispiel 114

### N-tert-Butoxycarbonyl-L-phenylalanyl-L-histidyl-4S-amino-3R-hydroxy-5-(2-nitrophenyl)pentanoyl-L-isoleucin-methylester

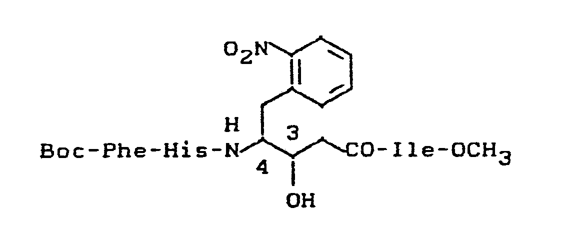

Die Titelverbindung wird ausgehend von N-tert-Butoxycarbonyl-4S-amino-3R-hydroxy-5-(2-nitrophenyl)pentanoyl-L-isoleucin-methylester (Beispiel 38) analog Beispiel 40 und Beispiel 41 durch Abspaltung der Schutzgruppe und nachfolgender Kupplung mit Boc-Phe-His-OH (Beispiel 48) erhalten.

DC-System I: R_{f} = 0,7 '

HPLC-Wert; System I: Rₜ = 5,20 min

(+) FAB-MS: m/z 766 (M + H)

### Beispiel 115

### N-tert-Butoxycarbonyl-4S-amino-3S-hydroxy-5-(3-nitrophenyl)pentansäure

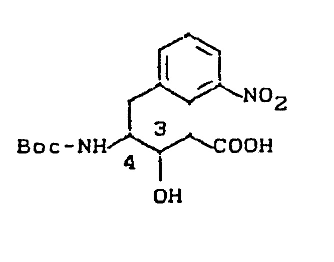

Die Titelverbindung wird analog Beispiel 9 aus 6,7 g (18,2 mmol) N-tert-Butoxycarbonyl-4S-amino-3S-hydroxy-5-(3-nitrophenyl)pentansäure-ethylester (Beispiel 56) hergestellt.

Ausbeute: 3,6 g (70% der Theorie)

Fp.: 143°C

DC-System I: R_{f} = 0,25

DC-System X: R_{f} = 0,70

### Beispiel 116

### N-tert-Butoxycarbonyl-45-amino-3S-hydroxy-5-(3-nitrophenyl)pentanoyl-L-isoleucin-methylester

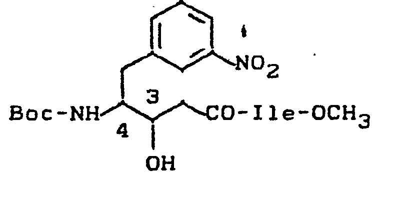

Die Titelverbindung wird analog Beispiel 37 aus 2,6 g (7,3 mmol) N-tert-Butoxycarbonyl-4S-amino-3S-hydroxy-5-(3-nitrophenyl)pentansäure (Beispiel 115) und L-Isoleucin- methylester Hydrochlorid hergestellt.

Ausbeute: 3,25 g (92,5% der Theorie)

HPLC-Wert, System II: Rₜ = 6,66 min

### Le A 24 292

### Beispiel 117

### 4S-Amino-3S-hydroxy-5-(3-nitrophenyl)-pentanoyl-L-isoleucin-methylester Hydrochlorid

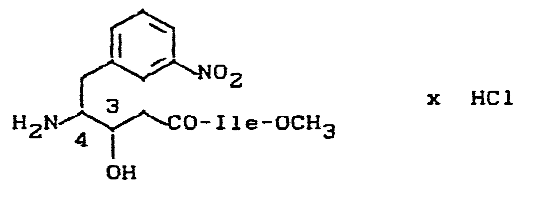

Die Titelverbindung wird analog Beispiel 11 aus 2,5 g (5,2 mmol) N-tert-Butoxycarbonyl-4S-amino-3S-hydroxy-5-(3-nitrophenyl)pentanoyl-L-isoleucin-methylester (Beispiel 116) hergestellt.

Ausbeute: 2,97 g (95% der Theorie)

### Beispiel 118

### N-tert-Butoxycarbonyl-L-phenylalanyl-L-histidyl-4S-amino-3S-hydroxy-5-(3-nitrophenyl)pentanoyl-L-isoleucin-methylester

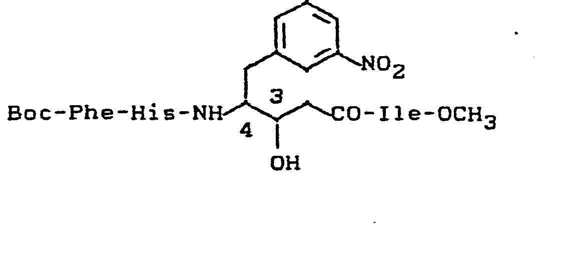

Die Titelverbindung wird aus 4S-Amino-3S-hydroxy-5-(3-nitrophenyl)pentanoyl-L-isoleucin-methylester Hydrochlorid (Beispie 117) und Boc-Phe-His-OH (Beispiel 48) analog Beispiel 12 hergestellt.

DC-System XI: R_{f} = 0,72

HPLC-Wert, System II: Rₜ = 7,10 min

(+) FAB-MS: m/z 766 (M+ H)

### Beispiel 119

### N-tert-Butoxycarbonyl-L-phenylalanyl-L-histidyl-4S-amino-3S-hydroxy-5-(3-aminophenyl)pentanoyl-L-isoleucin-methylester

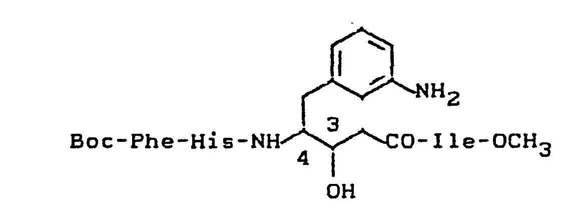

Die Titelverbindung wird durch katalytische Reduktion (analog Beispiel 27) aus N-tert-Butoxycarbonyl-L-phenylalanyl-L-histidyl-4S-amino-3S-hydroxy-5-(3-nitrophenyl)pentanoyl-L-isoleucin-methylester (Beispiel 118) erhalten.

DC-System XI : R_{f} = 0,33

HPLC-Wert; System II: R_{t =} 3,86 min

(+) FAB-MS: m/z 736 (M + H)

### Beispiel 120

### N-tert-Butoxycarbonyl-4S-amino-3R-hydroxy-5-(4-nitrophenyl)pentansäure-amid

### Le A 24 292

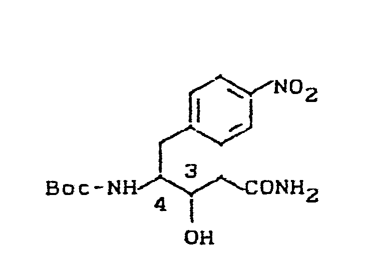

8,4 g (22 mmol) N-tert-Butoxycarbonyl-4S-amino-3R-hydroxy-5-(4-nitrophenyl)pentansäure-ethylester (Beispiel 8) werden in 150 ml ammoniakgesättigtem Methanol gelöst. Die Lösung wird drei Tage bei Raumtemperatur in einem mit einem Stopfen verschlossenen Kolben gerührt. Der weiße Niederschlag wird abgesaugt, mit Diethylether gewaschen und im Vakuum über Phosphorpentoxid getrocknet.

Ausbeute: 4,3 g (55% der Theorie)

DC-System I : R_{f} = 0,5

DC-System II: R_{f} = 0,3

HPLC-Wert; System II: Rₜ = 1,63 min

¹H-NMR (200 MHz, DMSO): δ = 8,12 (d, 2H); 7,45 (d, 2H); 7,30 (s, breit, 1H); 6,82 (s, breit, 1H); 6,69 (d, 1H); 5,10 (d, IH); 3,78 (m, 1H); 3,53 (m, 1H); 3,16 (dd, 1H); 2,60 (dd, 1H); 2,05 - 2,38 (m, 2H); 1,2 (s, 9H).

(+) FAB-MS: m/z 354 (M + H); m/z 294, m/z 254.

### Beispiel 121

### N-tert-Butoxycarbonyl-4S-amino-3S-hydroxy-5-(4-nitrophenyl)pentansäure-amid

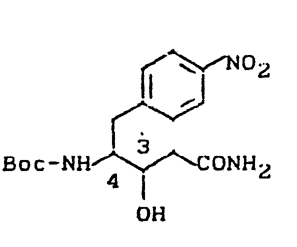

Die Titelverbindung wird ausgehend von Beispiel 7 analog Beispiel 120 erhalten.

(+) FAB-MS: m/z 354 (M + H); m/z 294; m/z 254

### Beispiel 122

### N-tert-Butoxycarbonyl-L-phenylalanyl-L-histidyl-4S-amino-3S-hydroxy-5-(4-nitrophenyl)pentansäure-amid

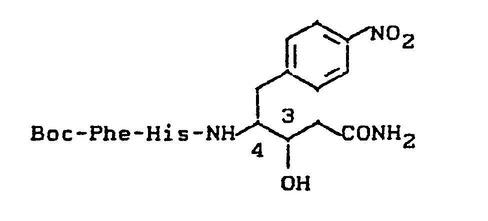

1,5 g (2,3 mmol) N-tert-Butoxycarbonyl-L-phenylalanyl-L-histidyl-4S-amino-3S-hydroxy-5-(4-nitrophenyl)pentansäure- ethylester (Beispiel 102) werden in 9 ml ammoniakgesättigtem Methanol gelöst. Die Lösung wird zwei Tage bei Raumtemperatur in einem mit einem Stopfen verschlossenen Kolben gerührt. Der Niederschlag wird abgesaugt, mit Diethylether gewaschen und getrocknet. Das so erhaltene Rohprodukt (150 mg) wird durch präparative HPLC mit dem in Beispiel 83 beschriebenen System gereinigt. Die das reine Produkt enthaltenden Fraktionen werden zusammengefaßt und lyophilisiert.

Ausbeute: 80 mg (5,5% der Theorie)

DC-System I: R_{f =} 0,10

HPLC-Wert; System 11: Rₜ = 2,22 min

(+) FAB-MS: m/z 638 (M + H); m/z 582; m/z 538

### Beispiel 123

### N-tert-Butoxycarbonyl-L-phenylalanyl-L-histidyl-4S-amino-3S-hydroxy-5-(4-nitrophenyl)pentansäure

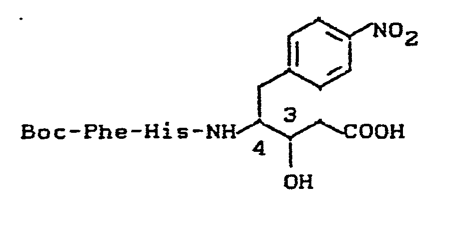

Die Titelverbindung wird aus N-tert-Butoxycarbonyl-L-phenylalanyl-L-histidyl-4S-amino-3S-hydroxy-5-(4-nitrophenyl)pentansäure-ethylester (Beispiel 102) durch alkalische Verseifung (wie in Beispiel 48 beschrieben) hergestellt.

DC-System XIII: R_{f =} 0,68

(+) FAB-MS: m/z 639 (M'+ H); m/z 583; m/z 539

### Beispiel 124

### N-tert-Butoxycarbonyl-L-(4-methoxyphenyl)alanyl-L-histidyl-4S-amino-3RS-hydroxy-5-(2-nitrophenyl)pentanoyl-L-isoleucin-methylester

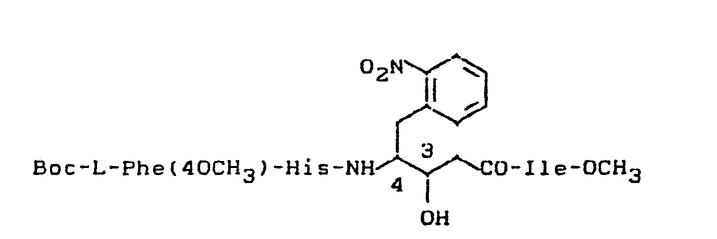

Die Titelverbindung wird analog Beispiel 12 aus 4S-Amino-3RS-hydroxy-5-(2-nitrophenyl)pentanoyl-L-isoleucin-methylester Hydrochlorid Lerhältlich aus Beispiel 37 durch Abspaltung der Boc-Schutzgruppe (analog Beispiel 11)] und N-tert-Butoxycarbonyl-L-(4-methoxyphenyl)alanyl-L-histidin [erhältlich durch PPA-Kupplung von tert-Butoxycarbonyl-L-(4-methoxyphenyl)alanin und Histidinmethylester (analog Beispiel 47) und nachfolgender Verseifung (analog Beispiel 48) des Produktes] hergestellt.

DC-System XII: R_{f} = 0,48 (ununterscheidbar)

HPLC-Wert; System II: Rₜ = 4,96 min (ununterscheidbar)

(+) FAB-MS: m/z 796 (M + H)

### Beispiel 125

### N-tert-Butoxycarbonyl-L-prolyl-L-(4-methoxyphenyl)alanyl-L-histidyl-4S-amino-3RS-hydroxy-5-(2-nitrophenyl)pentanoyl-L-isoleucin-methylester

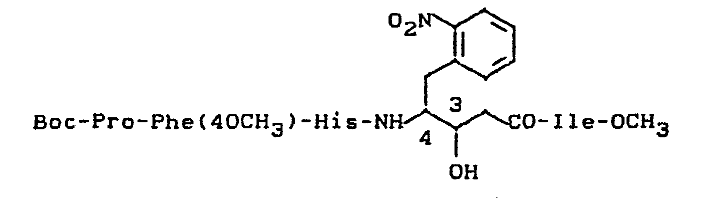

Die Titelverbindung wird durch Abspalten der N-terminalen Schutzgruppe (analog Beispiel 76) und nachfolgender Kupplung mit N-tert-Butoxycarbonyl-L-prolin (analog Beispiel 81) aus N-tert-Butoxycarbonyl-L-(4-methoxyphenyl)alanyl-L-histidyl-4S-amino-3RS-hydroxy-(2-nitrophenyl)pentanoyl-L-isoleucin-methylester (Beispiel 124) erhalten.

(+) FAB-MS: m/z 893 (M + H)

### Beispiel 126

### N-tert-Butoxycarbonyl-L-prolyl-L-(4-methoxyphenyl)alanyl-L-histidyl-4S-amino-3RS-hydroxy-5-(4-aminophenyl)pentanoyl-L-isoleucin-methylester

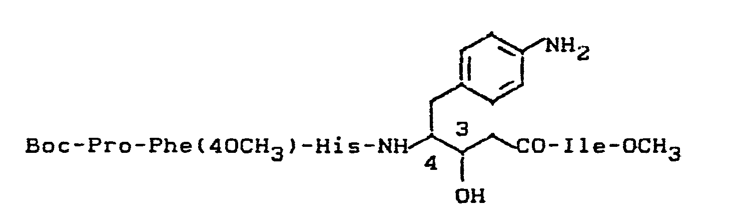

Die Titelverbindung wird durch katalytische Reduktion (analog Beispiel 27) aus N-tert-Butoxycarbonyl-L-prolyl-L-[4-methoxyphenyl)alanyl-L-histidyl-4S-amino-3R5-hydroxy-5-(4-nitrophenyl)pentanoyl-L-isoleucin-methylester (Beispiel 97) erhalten.

DC-System XI: R_{f =} 0,36 (ununterscheidbar)

HPLC-Wert; System II: R_{t =} 4,16 min (ununterscheidbar)

(+) FAB-MS: m/z 863 (M + H)

### Beispiel 127

### N-α-Phenoxyacetyl-L-histidyl-45-amino-3RS-hydroxy-5-(4-aminophenyl)pentanoyl-L-isoleucin-methylester

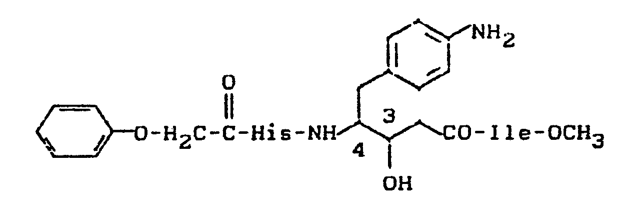

Die Titelverbindung wird durch katalytische Reduktion (analog Beispiel 27) aus N-α-Phenoxyacetyl-L-histidyl-4S-amino-3RS-hydroxy-5-(4-nitrophenyl)pentanoyl-L-isoleucin- methylester (Beispiel 98) erhalten.

DC-System XI: R_{f} = 0,31; Isomer A
R_{f} = 0,34; Isomer B

HPLC-Wert; System II: Rₜ = 2,09 min (ununterscheidbar;

(+) FAB-MS: m/z 623 (M + H)

### Beispiel 128

### N-tert-Butoxycarbonyl-L'-phenylalanyl-L-histidyl-4S-amino-3S-hydroxy-5-(4-nitrophenyl)pentanoyl-L-isoleucin-l-adamantylamid

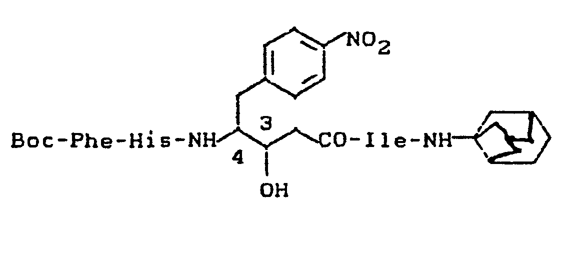

Die Titelverbindung wird nach bereits beschriebenen Methoden durch die folgenden Schritte erhalten:
a) Kupplung von N-tert-Butoxycarbonyl-4S-amino-3S-hydroxy-5-(4-nitrophenyl)pentansäure (Beispiel 9) analog Beispiel 37 mit L-Isoleucin-1-adamantylamid Hydrochlorid [erhältlich durch Kupplung von N-tert-Butoxycarbonyl-L-isoleucin mit 1-Adamtylamin (analog Beispiel 65) und nachfolgender saurer Abspaltung der N-tert-Butoxycarbonyl-Schutzgruppe aus dem Reaktionsprodukt (analog Beispiel 66)].
b) Abspaltung der Schutzgruppe aus dem Kupplungsprodukt des Schrittes a) und nachfolgender Kupplung des erhaltenen Hydrochlorides mit Boc-Phe-His-OH (analog Beispiel 40 und 41).
c) Präparative HPLC des Kupplungsproduktes aus Schritt b) mit dem in Beispiel 83 beschriebenen System.

DC-System I: R_{f} = 0,40

HPLC-Wert; System II: Rₜ = 22,04 min

(+) FAB-MS: m/z 885 (M + H)

### Beispiel 129

### N-tert-Butoxycarbonyl-L-phenylalanyl-L-histidyl-4S-amino-3S-hydroxy-5-(4-nitrophenyl)pentanoyl-L-isoleucin-2-adamantylamid

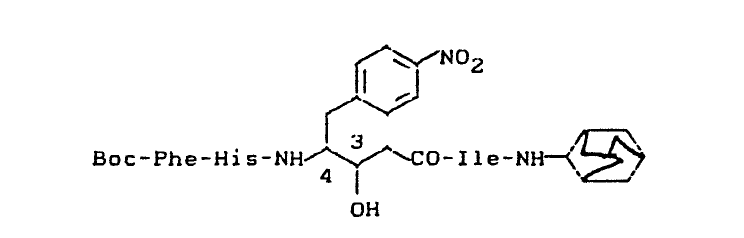

Die Titelverbindung wird nach bereits beschriebenen Methoden durch die folgenden Schritte erhalten:
a) Kupplung von N-tert-Butoxycarbonyl-4S-amino-3S-hydoxy-5-(4-nitrophenyl)pentansäure (Beispiel 9) analog Beispiel 37 mit L-Isoleucin-2-adamantylamid Hydrochlorid [erhältlich durch Kupplung von N-tert-Butoxycarbonyl-L-isoleucin mit 2-Adamtylamin (analog Beispiel 65) und nachfolgender saurer Abspaltung der N-tert-Butoxycarbonylschutzgruppe aus dem Reaktionsprodukt (analog Beispiel 66)].
b) Abspaltung der Schutzgruppe aus dem Kupplungsprodukt des Schrittes a) und nachfolgender Kupplung des erhaltenen Hydrochlorides mit Boc-Phe-His-OH (analog Beispiel 40 und 41).
c) Pärparative HPLC des Kupplungsproduktes aus Schritt b) mit dem in Beispiel 83 beschriebenen System.

DC-System XI: R_{f} = 0,49

HPLC-Wert; System II: Rₜ = 21,04 min

(+) FAB-MS: m/z 885 (M + H)

### Beispiel 130

### N-tert-Butoxycarbonyl-L-phenylalanyl-L-histidyl-4S-amino-3S-hydroxy-5-(4-nitrophenyl)pentanoyl-(2-RS-methyl)butyl- amid

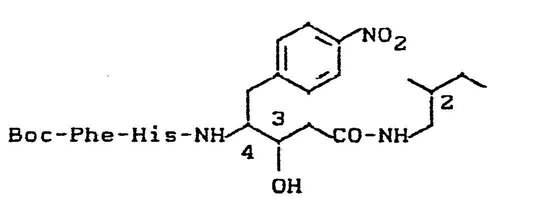

Die Titelverbindung wird nach bereits beschriebenen Methoden durch die folgenden Schritte erhalten:
a) Kupplung von N-tert-Butoxycarbonyl-4S-amino-3S-hydroxy-5-(4-nitrophenyl)pentansäure (Beispiel 9) analog Beispiel 37 mit RS-2-Methylbutylamin.
b) Abspaltung der Schutzgruppe aus dem Kupplungsprodukt des Schrittes a) und nachfolgender Kupplung des erhaltenen Hydrochlorides mit Boc-Phe-His-OH (analog Beispiel 40 und 41).
c) Präparative HPLC des Kupplungsproduktes aus Schritt b) mit dem in Beispiel 83 beschriebenen System.

DC-System I: R_{f} = 0,43 (ununterscheidbar)

HPLC-Wert; System II: Rₜ = 4,83 min (Isomer A) R_{t =} 5,13 min (Isomer B)

(+) FAB-MS: m/z 708 (M + H); m/z 652, m/z 608

### Beispiel 131

### N-tert-Butoxycarbonyl-L-phenylalanyl-L-histidyl-4S-amino-3S-hydroxy-5-(4-nitrophenyl)pentanoyl-L-isoleucin-n-propylester

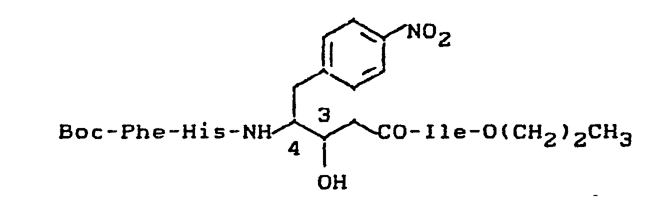

Die Titelverbindung wird nach bereits beschriebenen Methoden durch die folgenden Schritte erhalten:
a) Kupplung von N-tert-Butoxycarbonyl-4S-amino-3S-hydroxy-5-(4-nitrophenyl)pentansäure (Beispiel 9) analog Beispiel 37 mit L-Isoleucin-n-propylester Hydrochlorid (käuf) ich oder nach bekannten Methoden erhältlich).
b) Abspaltung der Schutzgruppe aus dem Kupplungsprodukt des Schrittes a) und nachfolgender Kupplung des erhaltenen Hydrochlorides mit Boc-Phe-His-OH (analog Beispiel 40 und 41).
c) Präparative HPLC des Kupplungsproduktes aus Schritt b) mit dem in Beispiel 83 beschriebenen System.

DC-System I: R_{f} = 0,55

HPLC-Wert; System II: Rₜ = 10,08 min

(+) FAB-MS: m/z 794 (M + H); m/z 694

### Beispiel 132

### N-tert-ButoxycarhonyL-L-phenylalanyl-L-histidyl-4S-amino-3S-hydroxy-5-(4-nitrophenyl)pentanoyl-L-leucin-ethylester

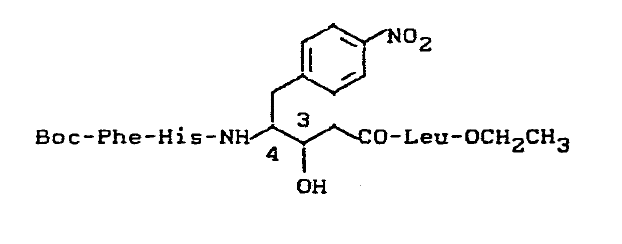

Die Titelverbindung wird nach bereits beschriebenen Methoden durch die folgenden Schritte beschrieben:
a) Kupplung von N-tert-Butoxycarbonyl-4S-amino-3S-hydroxy-5-(4-nitrophenyl)pentansäure (Beispiel 9) analog Beispiel 37 mit L-Leucin-ethylester Hydrochlorid (käuflich oder nach bekannten Methoden erhältlich).
b) Abspaltung der Schutzgruppe aus dem Kupplungsprodukt des Schrittes a) und nachfolgender Kupplung des erhaltenen Hydrochlorides mit Boc-Phe-His-OH (analog Beispiel 40 und 41).
c) Präparative HPLC des Kupplungsproduktes aus Schritt b) mit dem in Beispiel 83 beschriebenen System.

DC-System 1: R_{f =} 0,48

HPLC-Wert; System II: Rₜ = 7,38 min

(+) FAB-MS: m/z 780 (M + H)

### Beispiel 133

### N-tert-Butoxycarbonyl-L-phenylalanyl-L-histidyl-4S-amino-3R-hydroxy-5-(2-aminophenyl)pentanoyl-L-isoleucin-methylester

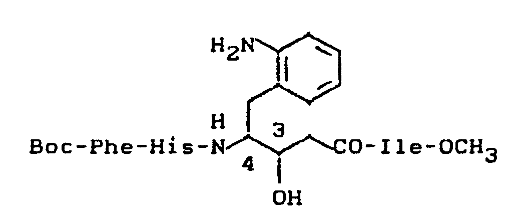

Die Titelverbindung wird durch katalytische Reduktion (analog Beispiel 27) aus N-tert-Butoxycarbonyl-L-phenylalanyl-L-histidyl-4S-amino-3R-hydroxy-5-(2-nitrophenyl)-pentanoyl-L-isoleucin-methylester (Beispiel 114) erhalten.

DC-System XI: R_{f} = 0,36

HPLC-Wert; System II: R_{t =} 4,39 min

(+) FAB-MS: m/z 736 (M + H)

## Patentansprüche

1. Peptide enthaltend bis zu acht Aminosäuregruppierungen der allgemeinen Formel I 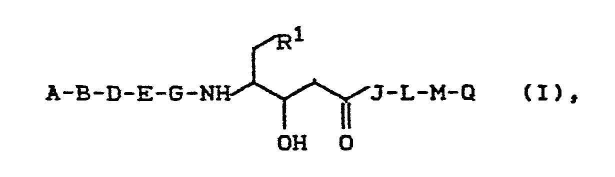 in welcher
A - für Wasserstoff, C₁-C₈-Alkyl, C₇-C₁₄-Aralkyl, Phenylsulfonyl, Tolylsulfonyl, C₁-C₈-Alkylsulfonyl oder
- für eine Aminoschutzgruppe steht,
B - für eine direkte Bindung steht, oder
- für Sarcosyl oder für eine Gruppe der Formel 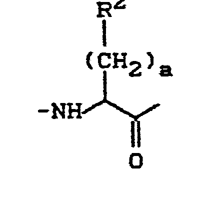 steht, worin
a - eine Zahl 0,1,2,3 oder 4 bedeutet und
R²⁻ Wasserstoff, C₁-C₆-Alkyl, Hydroxy-C₁-C₂- alkyl oder eine Gruppe der Formel -CH₂-CO-NHR³ oder -CH₂-NH-R³ bedeutet, wobei
_{R}³⁻ für Wasserstoff, C₁-C₆-Alkyl, C₇-C₁₄-Aralkyl, Tolylsulfonyl, Phenylsulfonyl, C₁-C₆-Alkylsulfonyl oder für eine Aminoschutzgruppe steht, oder
R^{2 -} Guanidinomethyl, Mercaptomethyl, Methylthiomethyl, Carboxymethyl, C₁-C₆-Alkoxycarbonylmethyl, C₇-C₁₄-Aralkoxycarbonylmethyl, Halogen, Indolylmethyl, 4-Imidazolylmethyl, Pyridyl, Triazolylmethyl, Pyrazolylmethyl oder Aryl bedeutet, das gegebenenfalls bis zu dreifach gleich oder verschieden substituiert ist durch C₁-C₄-Alkyl, C₁-C₄-Alkoxy, Trifluormethyl, Halogen, Hydroxy, Nitro oder durch eine Gruppe der Formel 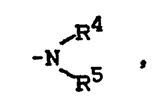 wobei
R⁴,R⁵ - gleich oder verschieden sind und
- für Wasserstoff, C₁-C₆-Alkyl, Aryl, Aralkyl, Phenylsulfonyl, Tolylsulfonyl, C₁-C₆-Alkylsulfonyl, Acetyl, Benzoyl oder für eine Aminoschutzgruppe stehen
D - für eine direkte Bindung steht, oder
für eine Gruppe der Formel 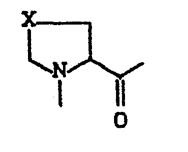 worin
X - für Methylen, Ethylen oder Schwefel steht,
E - die gleiche Bedeutung hat wie B und mit diesem gleich oder verschieden ist,
G - die gleiche Bedeutung hat wie B und mit diesem gleich oder verschieden ist,
R¹- für eine Gruppe der Formel 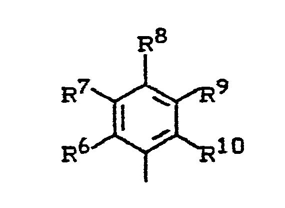 steht, worin
R⁶, R⁷, _{R}⁸, R⁹ und R¹⁰ - gleich oder verschieden sind
und
- für Wasserstoff, C₁-C₆-alkyl, C₁-C₆-Alkoxy, Aryl, Halogen, Hydroxy, Cyano, Trifluormethyl Trifluormethoxy, Trifluormethylthio, Nitro oder für eine Gruppe der Formel 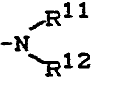 stehen, wobei
R¹¹,R¹²- die gleiche Bedeutung haben wie R⁴,R⁵ und mit diesen gleich oder verschieden sind,
mit der Maßgabe, daß mindestens einer der Substituenten R⁶, R⁷, R⁸, R⁹ oder R¹⁰ für Nitro oder die Gruppe -NR¹¹R¹² stehen m_{uß},
J, L, M - jeweils gleich oder verschieden sind und
die gleiche Bedeutung haben wie B und mit diesem gleich oder verschieden sind,
und
Q - für einen Rest der Formel -OR¹³, -NH_{R}¹⁴ oder - NR¹⁴R¹⁵ oder -_{NH}-NHR³ steht,
worin
_{R}^{13 -} für Wasserstoff oder
- für C₁-C₂₀-Alkyl steht, das gegebenenfalls durch ein Sauerstoffatom in der Kette unterbrochen ist und das substituiert sein kann durch Halogen, Hydroxy, Phenyl oder Pyridyl,
R^{14 -} für Wasserstoff C₁-C₁₀-Alkyl steht, das substituiert sein kann durch Aryl, wobei dieser Arylrest wiederum Substituenten aus der Reihe Nitro, C₁-C₄-Alkyl, C_{I}-C₄-Alkoxy, Halogen oder Cyano tragen kann, durch Halogen, Adamantyl, Chinuclidin, Piperidin, N-Methylpiperazin, N-Phenylpiperazin, N-Benzylpiperazin, Pyridyl oder Morpholin, oder - für Aryl steht, das bis zu dreifach gleich oder verschieden substituiert sein kann durch Nitro, Halogen, Cᵢ-C₄-Alkoxy, C_{I}-C₄-Alkyl, wobei Alkyl wiederum Substituenten aus der Reihe Hydroxy, Amino, Carboxy und/oder C₁-C₄-Alkoxycarbonyl tragen kann, oder
- für Adamantyl oder Chinuclidin steht,
und
_{R}^{15 -} für C₁-C₆-Alkyl steht,
oder
_{R}¹⁴ und _{R}^{15 -} gemeinsam einen 5- bis 7-gliedrigen Ring bilden, der als weiteres Heteroatom ein Sauerstoffatom, ein Schwefelatom oder die Gruppe -NH, -N-C₁-C₆-Alkyl, -N-Dimethylamino-C₁-C₄-alkyl, -N-Aryl oder -N-Aralkyl enthalten kann oder der durch C₁-C₄-Alkyl oder Aralkyl substituiert sein kann
und
R³ die angegebene Bedeutung hat
und deren physiologisch unbedenklichen Salze.

2. Verbindungen der allgemeinen Formel I gemäß Anspruch 1, in welcher
A - für Wasserstoff, C_{I}-C₄-Alkyl, Benzyl, Phenylsulfonyl, Tolylsulfonyl, C₁-C₄-Alkylsulfonyl oder
- für eine Aminoschutzgruppe steht,
B - für eine direkte Bindung oder für einen Rest der Formel steht 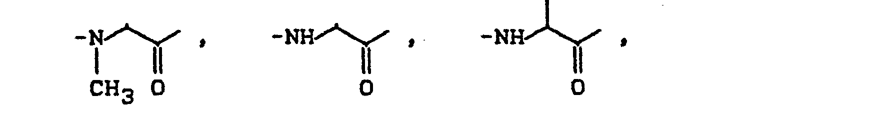 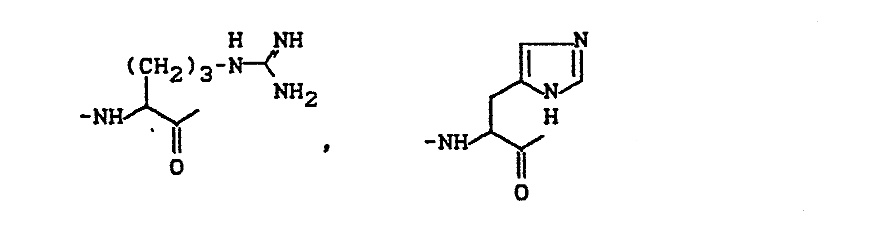 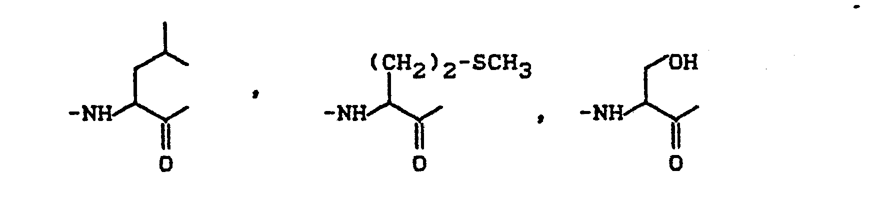 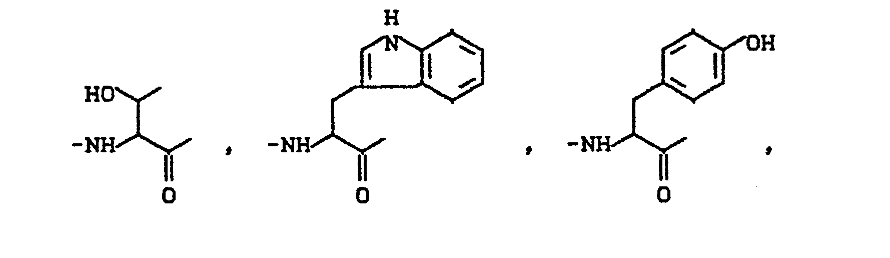 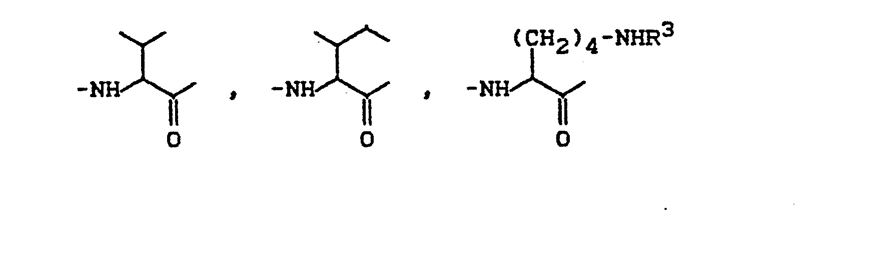 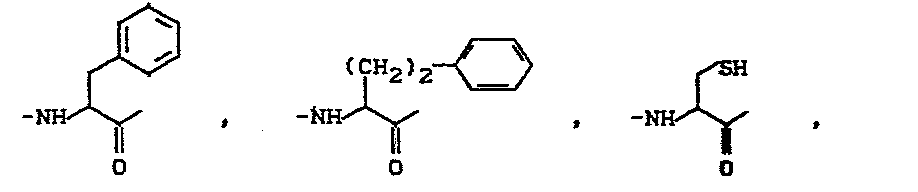 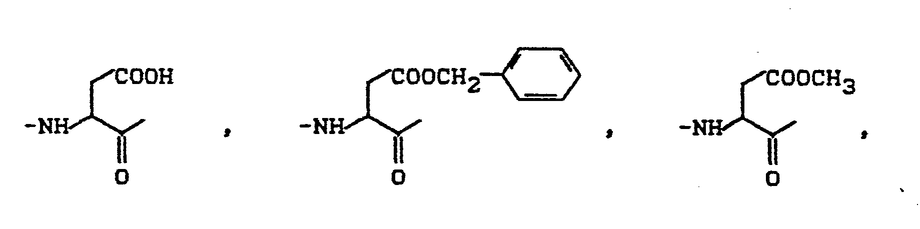 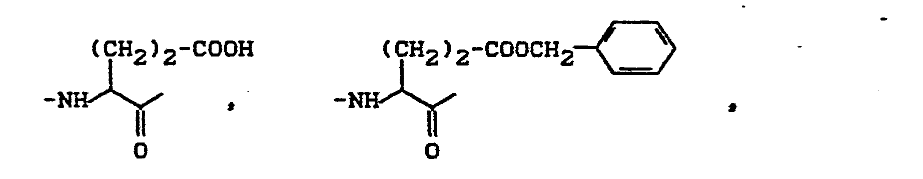 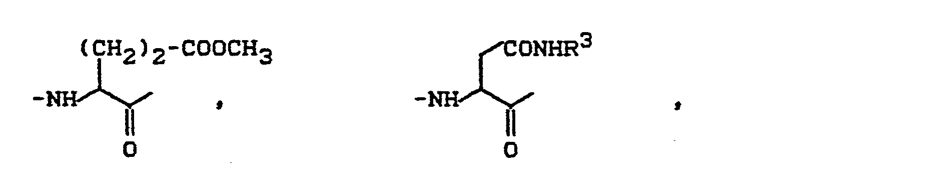 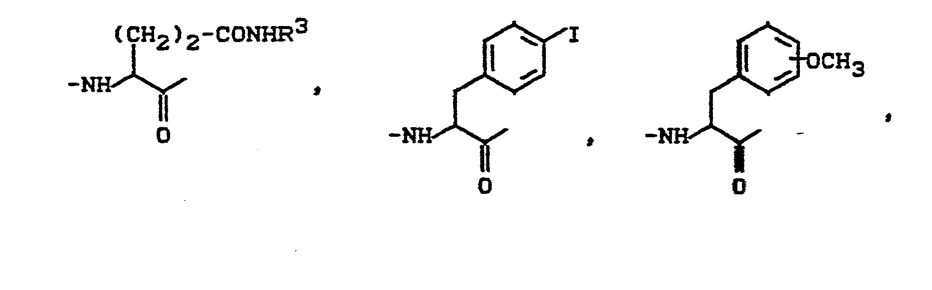 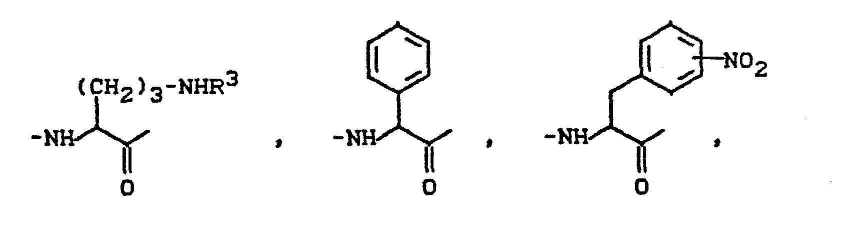 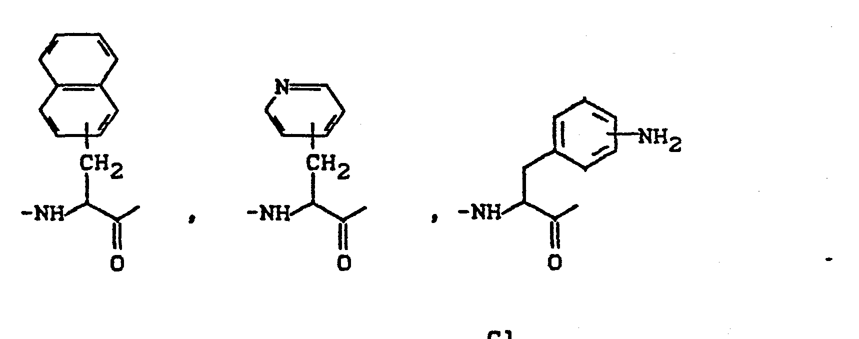 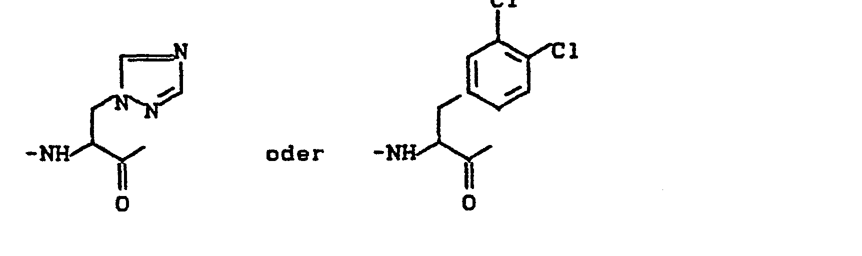 in ihrer D-Form, L-Form oder als D,L-Isomerengemisch, bevorzugt in der L-Form,
worin
R³⁻ für Wasserstoff, C₁-C₄-Alkyl, Benzyl, Phenylsulfonyl, Tolylsulfonyl, C₁-C₄-Alkyl sulfonyl oder
- für eine Aminoschutzgruppe steht,
D - für eine direkte Bindung oder für die Gruppe der Formel 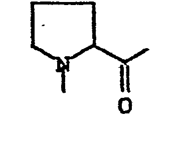 in ihrer L-Form, D-Form oder als D,L-Isomerengemisch steht,
E,G - jeweils gleich oder verschieden sind und die gleiche Bedeutung haben wie B und mit diesem gleich oder verschieden sind,
R¹⁻ für eine Gruppe der Formel 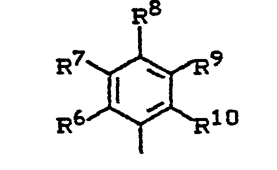 steht, worin
R⁶,R⁷,R⁸,R⁹ und R¹⁰ gleich oder verschieden sind und
- für Wasserstoff, Methyl, Methoxy, Fluor, Chlor, Brom, Iod, Hydroxy, Cyano, Trifluormethyl, Nitro oder für eine Gruppe der Formel 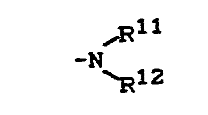 stehen, worin
R¹¹,R¹² - gleich oder verschieden sind und
- für Wasserstoff, C₁-C₄-Alkyl, Phenyl, Benzyl, Phenylsulfonyl, Tosyl, C₁-C₄-Alkylsulfonyl, Acetyl oder
- für eine Aminoschutzgruppe stehen,
mit der Maßgabe, daß mindestens einer der Substituenten R⁶, R⁷, R⁸, R⁹ oder R¹⁰ für Nitro oder für die Gruppe -NR¹¹R¹² stehen muß,
J,L,M - jeweils gleich oder verschieden sind und
- die gleiche Bedeutung haben wie B und mit diesem gleich oder verschieden sein können,
und
Q - für einen Rest der Formel -OR13, -NH-NHR3, NHR14 oder 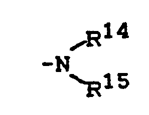 steht, worin
_{R}^{13 -} für Wasserstoff oder C₁-C₁₈-Alkyl steht, das gegebenenfalls durch Chlor, Brom, Hydroxy oder Phenyl substituiert ist,
_{R}^{14 -} für Wasserstoff, C_{I}-C₄-Alkyl steht, das gegebenenfalls substituiert ist durch gegebenenfalls durch Nitro, Methyl oder Methoxy substituiertes Phenyl, durch Fluor, Chlor, Brom, Pyridyl, Adamantyl, Chinuclidin, Piperidin, N-Methyl-, N-Phenyl-oder N-Benzylpiperazin oder Benzylcyclohexyl oder
- für Phenyl steht, das gegebenenfalls substituiert ist durch Nitro, Fluor, Chlor, Methoxy oder durch gegebenenfalls durch Hydroxy, Amino, Carboxy und/oder C₁-C₄-Alkoxycarbonyl substituiertes C_{I}-C₄-Alkyl, oder
- für Adamantyl oder Chinuclidin steht,
R¹⁵ - für Cₗ-C₄-Alkyl steht oder
R¹⁴ und R¹⁵ gemeinsam einen Ring aus der Reihe Pyrrolidin, Piperidin, Benzylpiperidin, Morpholin, Piperazin, N-Methyl-, N-Phenyl-, N-Benzyl-, -N-Dimethylaminoethylpiperazin bilden
und
R³ die angegebene Bedeutung hat
und deren physiologisch unbedenklichen Salze.

3. Verbindungen der allgemeinen Formel I gemäß Anspruch 1 in welcher
A - für Wasserstoff, Methyl, Ethyl, Tosyl oder für eine Aminoschutzgruppe, vorzugsweise aus der Reihe Benzyloxycarbonyl, 4-Methoxybenzyloxycarbonyl, 4-Nitrobenzyloxycarbonyl, 3,4,5-Trimethoxybenzyloxycarbonyl, Methoxycarbonyl, Ethoxycarbonyl Propoxycarbonyl, Butoxycarbonyl, tert.-Butoxycarbonyl, 2-Bromethoxycarbonyl, 2-Chlorethoxycarbonyl, 2,2,2-Trichlorethoxycarbonyl, Allyloxycarbonyl, Phenoxycarbonyl, 4-Nitrophenoxycarbonyl, Fluorenyl-9-methoxycarbonyl, Acetyl, Pivaloyl, Isovaleroyl, Phthaloyl, 2,2,2-Trichloracetyl, 2,2,2-Trifluoracetyl, Benzyl, Tosyl, Benzoyl, 4-Nitrobenzoyl oder Phthalimido steht,
B - für eine direkte Bindung oder
- für Sarcosyl (Sar), Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Ornithyl (Orn), Phenylalanyl (Phe) Cystyl (Cys), Asparagyl (Asp), Asparaginyl (Asn), Gluthamyl (Glu), Gluthaminyl (Gln), Phenylglycyl (Phy), 4-Nitrophenylalanyl [Phe(4-NO₂)], 3-Nitrophenylalanyl [Phe(3NO₂)], 2-Nitrophenylalanyl [Phe(2NO₂)], 2-, 3-oder 4-Aminophenylalanyl [Phe(2NH₂), Phe(3NH₂), Phe(4-NH₂)7, 3,4-Dichlorphenylalanyl [Phe(3,4-Cl₂)], 4-Iodophenylalanyl CPhe(4I)], 4-Methoxyphenylalanyl [Phe(4OCH₃)], 1-Triazolylalanyl [Trz(1)], 2-Pyridylalanyl [Pyr(2)], 3-Pyridylalanyl [Pyr(3)], 4-Pyridylalanyl [Pyr(4)], 1-Naphthylalanyl [Nal(1)] oder 2-Naphthylalanyl [Nal(2)], gegebenenfalls mit Schutzgruppen, in ihrer L-Form oder D-Form, bevorzugt jedoch in ihrer L-Form, steht
D - für eine direkte Bindung oder für D- oder L-Prolyl (Pro) steht,
E,G - jeweils gleich oder verschieden sind und
- die gleiche Bedeutung wie B haben und mit diesem gleich oder verschieden sind
R¹⁻ für einen Phenylrest steht, der in 2-, 3- oder 4-Stellung eine Nitrogruppe oder eine Aminogruppe der Formel 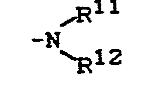 trägt, wobei
R¹¹,R¹² - gleich oder verschieden sind und
- Wasserstoff, Methyl, Ethyl, Acetyl bedeuten,
oder
R¹¹ - für Wasserstoff und R¹² für eine Aminoschutzgruppe stehen,
- oder für 2-Methyl-3-nitrophenyl, 4-Methyl-3-nitrophenyl, 6-Methyl-2-nitrophenyl, 3-Methyl-4-nitrophenyl oder 3,5-Dinitrophenyl steht,
J,L,M - jeweils gleich oder verschieden sind und die gleiche Bedeutung haben wie B und mit diesem gleich oder verschieden sind,
und
Q - für einen Rest der Formel OR¹³, NH-_{NHR}³, NHR¹⁴ oder 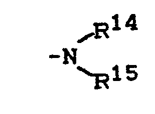 steht, worin
R^{3 -} für Wasserstoff, tert-Butoxycärbonyl oder Benzyloxycarbonyl steht,
_{R}^{13 -} für Wasserstoff, Benzyl oder C₁-C₄-Alkyl steht,
_{R}^{14 -} für Wasserstoff,Cₗ-C₄-Alkyl, Benzyl, 1-Phenylethyl, 2-Phenylethyl, Adamantyl, Chinuclidyl, Pyridylmethyl oder 4-Benzylcyclohexylmethyl steht,
^{R15 -} für C₁-C₄-Alkyl steht, oder
R¹⁴ und R¹⁵ gemeinsam einen Ring wie 4-Benzylpiperidino, N-Benzyl-, N-Phenethylpiperazin oder N-Dimethylaminoethylpiperazin bilden
und deren physiologisch unbedenklichen Salze.

4. Verbindungen der allgemeinen Formel I 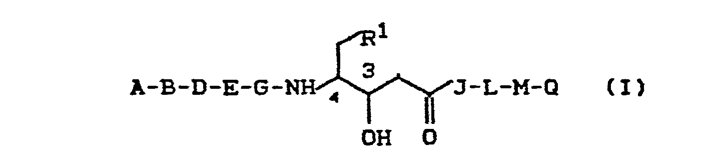 in welcher
A - für Wasserstoff, tert-Butoxycarbonyl, Benzyloxycarbonyl, Ethoxycarbonyl, Acetyl, Chloracetyl, Isovaleroyl, Benzoyl, Tosyl, Phenoxycarbonyl, Phthaloyl oder Phthalimido steht,
B - für eine direkte Bindung oder Phenylalanyl (Phe) steht,
D - für eine direkte Bindung oder Prolyl (Pro) steht,
E - für eine direkte Bindung steht oder Tyrosyl. (Tyr), Phenylalanyl (Phe), 1-Naphthylalanyl [Nal(1)], 4-Nitrophenylalanyl [Phe(4NO₂)], 3,4-Dichlorphenylalanyl [Phe(3,4-Cl₂)], 4-Aminophenylalanyl [Phe(4NH₂)], 4-Iodophenylalanyl [Phe(4I)], 4-Methoxyphenylalanyl [Phe(4OCH₃)] oder Phenylglycyl (Phg) steht,
G - für eine direkte Bindung oder
-für Phenylalanyl (Phe), Histidyl (His), 3-Pyridylalanyl [Pyr(3)], 4-Aminophenylalanyl [Phe-(4NH₂)] wobei die Aminogruppe gegebenenfalls durch Benzyloxycarbonyl (Z), tert-Butyloxycarbonyl (Boc) oder Fluorenyl-9-methoxycarbonyl (F-Moc) geschützt ist oder für 1-Triazolylalanyl [Trz(1)] steht, bevorzugt in der L-Form oder als DL-Gemisch
R¹⁻ für 2-, 3- oder 4-Nitrophenyl, 2-, 3- oder 4-Aminophenyl steht
wobei die Gruppe 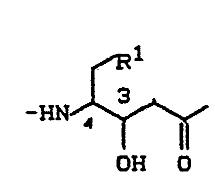 in der 3S,4S-Konfiguration, 3R,4S-Konfiguration sowie als 3RS4S-Isomerengemisch vorliegt,
J - für eine direkte Bindung steht oder
- für Leucyl (Leu) oder Isoleucyl (Ile) bevorzugt jeweils in der L-Form steht,
L - für eine direkte Bindung steht oder
- für Phenylalanyl (Phe), Histidyl (His) oder 4-Nitrophenylalanyl [Phe(4NO₂)], bevorzugt jeweils in der L-Form steht,
M - für eine direkte Bindung steht
und
Q - für Hydroxy, Methoxy, Ethoxy, n-Propoxy, Isopropoxy, tert.-Butoxy oder Benzyloxy steht, oder
- für Amino, Benzylamino, 1-Phenylethylamino, 2-Phenylethylamino, α-Pyridylmethylamino, ß-Pyridylmethylamino, γ-Pyridylmethyllamino, 4-Benzylcyclohexylmethylamino, 4-Benzylpiperidino, 4-Benzylpiperazino, N-Dimethylaminoethylpiperazino, 1-Adamantylamino, 2-Adamantylamino oder 3-Chinuclidinamino steht
und deren physiologisch unbedenklichen Salze.

5. Verfahren zur Herstellung von Peptiden enthaltend bis zu acht Aminosäuregruppierungen der allgemeinen Formel I 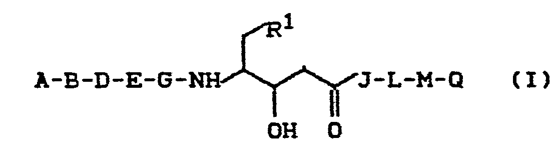 in welcher
A - für Wasserstoff, C₁-_{C}-Alkyl, C₇-C₁₄-Aralkyl, Phenylsulfonyl, Tolylsulfonyl, C₁-C₈-Alkylsulfonyl oder
- für eine Aminoschutzgruppe steht,
B - für eine direkte Bindung steht, oder
- für Sarcosyl oder für eine Gruppe der Formel 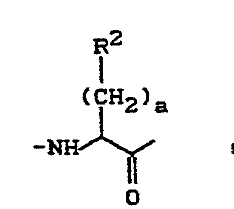 steht, worin
a - eine Zahl 0,1,2,3 oder 4 bedeutet und _{R2-} Wasserstoff, C₁-C₆-Alkyl, Hydroxy-C₁-C₂- alkyl oder eine Gruppe der Formel -CH₂-CO-NHR³ oder -CH₂-NH-R³
bedeutet,
wobei
R³⁻ für Wasserstoff, CI-C6-Alkyl, C₇-C₁₄-Aralkyl, Phenylsulfonyl, Cₗ-C₆-Alkylsulfonyl oder für eine Aminoschutzgruppe steht, oder
R² - Guanidinomethyl, Mercaptomethyl, Methylthiomethyl Carboxymethyl, C₁-C₆-Alkoxycarbonylmethyl, C₇-C₁₄-Aralkoxy carbonylmethyl, Halogen, Indolylmethyl, 4-Imidazolylmethyl, Pyridyl, Triazolylmethyl, Pyrazolylmethyl oder Aryl bedeutet, das gegebenenfalls bis zu dreifach gleich oder verschieden substituiert ist durch C₁-C₄-Alkyl,C₁-C₄-Alkoxy Trifluormethyl, Halogen, Hydroxy, Nitro oder durch eine Gruppe der Formel 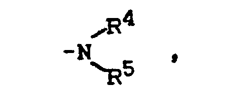 wobei
R⁴,R⁵ - gleich oder verschieden sind
und
- für Wasserstoff, C₁-C₆-Alkyl, Aryl,
Aralkyl, Phenylsulfonyl, Tolylsulfonyl, C₁-C₆-Alkylsulfonyl, Acetyl, Benzoyl oder für eine Aminoschutzgruppe stehen
D - für eine direkte Bindung steht, oder für eine Gruppe der Formel 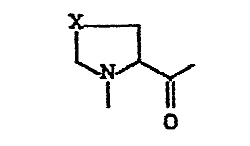 worin
X - für Methylen, Ethylen oder Schwefel steht,
E - die gleiche Bedeutung hat wie B und mit dies gleich oder verschieden ist, -
G - die gleiche Bedeutung hat wie B und mit diesem gleich oder verschieden ist,
R¹⁻ für eine Gruppe der Formel 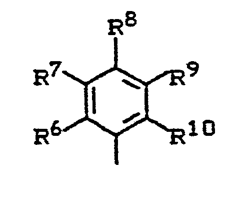 steht, worin
R⁶, R⁷, R⁸, _{R}⁹ und R¹⁰ - gleich oder verschieden sind
und
- für Wasserstoff, C₁-C₆-Alkyl, C_{I}-C₆-Alkoxy, Aryl, Halogen, Hydroxy, Cyano, Trifluormethl, Trifluormethoxy, Trifluormethylthio, Nitro oder für eine Gruppe der Formel 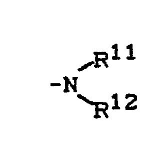 stehen, wobei
R¹¹, R¹² - die gleiche Bedeutung haben wie R⁴,R⁵ und mit diesen gleich oder verschieden sind,
mit der Maßgabe, daß mindestens einer der Substituenten R⁶, R⁷, R⁸, R⁹ oder R¹⁰ für Nitro oder die Gruppe -NR¹¹R¹² stehen muß,
J, L, M - jeweils gleich oder verschieden sind und
die gleiche Bedeutung haben wie B und mit diesem gleich oder verschieden sind,
und
Q - für einen Rest der Formel -OR¹³, -NH-N_{HR}³, _{NHR}¹⁴ oder -_{NR}¹⁴_{R}¹⁵ steht,
worin
R^{13 -} für Wasserstoff oder
- für C₁-C₂₀-Alkyl steht, das gegebenenfalls durch ein Sauerstoffatom in der Kette unterbrochen ist und das gegebenenfalls substituiert ist durch Halogen, Hydroxy, Phenyl oder Pyridyl,
_{R}^{14 -} für Wasserstoff, C₁-C₁₀-Alkyl steht, das gegebenenfall substituiert ist durch Aryl, wobei dieser Arylrest wiederum Substituenten aus der Reihe Nitro, C₁-C₁-Alkyl, C₁-C₄-Alkoxy, Halogen oder Cyano tragen kann, durch Halogen, Adamantyl, Chinuclidin, Piperidin, N-Methylpiperazin, N-Phenylpiperazin N-Benzylpiperazin, Morpholin, oder Pyridyl,
oder
- für Aryl steht, das gegebenenfalls bis zu dreifach gleich oder verschieden substituiert ist durch Nitro, Halogen, C₁-C₄-Alk- oxy, C₁-C₄-Alkyl, wobei Alkyl wiederum Substituenten aus der Reihe Hydroxy, Amino, Carboxy und/oder C₁-C₄-Alkoxycarbonyl tragen kann, oder
- für Adamantyl oder Chinuclidin steht,
und
_{R}^{15 -} für C₁-C₆-Alkyl steht,
oder
R¹⁴ und R^{15 -} gemeinsam einen 5- bis 7-gliedrigen Ring bilden, der als weiteres Heteroatom ein Sauerstoffatom, ein Schwefelatom oder die Gruppe -NH, -N-C₁-C₆-Alkyl, -N-Dimethylamino-C_{I}-C₄-alkyl, N-Aryl oder - N-Aralkyl enthalten kann oder der gegebenenfalls durch C₁-C₄-Alkyl oder Aralkyl substituiert ist
und
R³ die angegebene Bedeutung hat und deren physiologisch unbedenklichen Salze, dadurch gekennzeichnet, daß man diese Peptide gemäß konventionellen Methoden der Peptidchemie herstellt.

6. Verfahren zur Herstellung von Verbindungen der allgemeinen Formel I dadurch gekennzeichnet, daß man ein entsprechendes Bruchstück, bestehend aus einer oder mehreren Aminosäuregruppierungen, welches eine freie gegebenenfalls aktivierte Carboxylgruppe enthält, mit einem komplementierenden Bruchstück, bestehend aus einer oder mehreren Aminosäuregruppierungen, welches eine Aminogruppe, gegebenenfalls in aktivierter Form, enthält, umsetzt wobei diese Umsetzung gegebenenfalls wiederholt wird, bis das gewünschte Peptid der allgemeinen Formel I vorliegt und wobei anschließend gegebenenfalls vorhandene Schutzgruppen nach üblichen Methoden abgespalten oder gegen andere Schutzgruppen ausgetauscht werden.

7. Verfahren gemäß Anspruch 5, dadurch gekennzeichnet, daß man reaktive Gruppen in den Seitenketten der Bruchstücke, insbesondere Amino- oder Hydroxygruppen, vor der Umsetzung durch Schutzgruppen inaktiviert.

8. Verfahren gemäß Anspruch 5, dadurch gekennzeichnet, daß man als Bruchstücke Di-, Tri- oder Oligopeptidbausteine, die nach üblichen Methoden hergestellt werden einsetzt, die in einem Schritt zu den gewünschten Verbindungen der allgemeinen Formel I umgesetzt werden.

9. Verfahren gemäß Anspruch 5, dadurch gekennzeichnet, daß man Bruchstücke mit aktivierten Carboxylgruppen mit Aminogruppen enthaltenen Bruchstücken umsetzt.

10. Verfahren gemäß Ansprüchen 5 bis 9, dadurch gekennzeichnet, daß man die Umsetzung bei Temperaturen zwischen -80°C und 300°C in Gegenwart von inerten Lösungsmitteln wie Wasser oder organischen Lösungsmitteln oder Gemischen davon und gegebenenfalls in Gegenwart von Kondensationsmitteln durchführt.

11. Verbindungen der allgemeinen Formel II 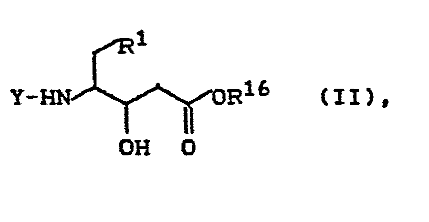 in welcher
R¹⁻ für eine Gruppe der Formel 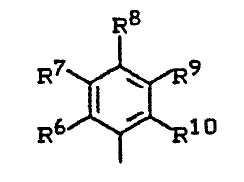 steht, worin
R⁶,R⁷,R⁸,R⁹ und R^{10 -} gleich oder verschieden sind und
- für Wasserstoff, C₁-C₆-Alkyl, C₁-C₆-Alkoxy, Halogen, Hydroxy, Cyano, Trifluormethyl, Trifluormethoxy, Trifluormethylthio, Nitro oder für eine Gruppe der Formel 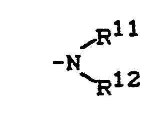 stehen, wobei
R¹¹,R¹² - gleich oder verschieden sind und
- für Wasserstoff, C₁-C₆-Alkyl, Aryl, Aralkyl, Phenylsulfonyl, Tolylsulfonyl, C₁-C₆-Alkylsulfonyl, Acetyl, Benzoyl oder für eine Aminoschutzgruppe stehen, mit der Maßgabe, daß mindestens einer der Substituenten R³,R⁷,R⁸,R⁹ oder R¹⁰ für Nitro oder die Gruppe N_{R}¹¹_{R}¹² stehen _{muß},
R¹⁶⁻ für Wasserstoff oder für geradkettiges oder verzweigtes, gegebenenfalls durch ein Sauerstoffatom in der Kette unterbrochenes C₁-C₁₂-Alkyl steht,
und
Y - für Wasserstoff, C₇-C₁₄-Aralkyl, C₁-C₈-Alkyl, Phenylsulfonyl, Tolylsulfonyl, C₁-C₈-Alkylsulfonyl oder für eine Aminoschutzgruppe steht.

12. Verfahren zur Herstellung von Verbindungen der allgemeinen Formel II 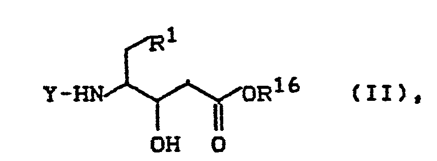 in welcher
R¹⁻ für eine Gruppe der Formel 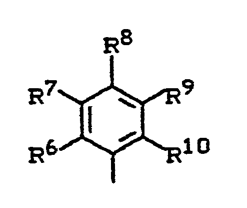 steht, worin
R⁶,R⁷,R⁸,R⁹ und R¹⁰ - gleich oder verschieden sind und
- für Wasserstoff, C₁-C₆-Alkyl, C₁-C₆-Alkoxy, Halogen, Hydroxy, Cyano, Trifluormethyl, Trifluormethoxy, Trifluormethylthio, Nitro oder für eine Gruppe der Formel 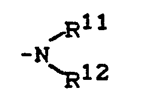 stehen, wobei
R¹¹,R¹² - gleich oder verschieden sind und -- für Wasserstoff, C₁-C₆-Alkyl, Aryl, Aralkyl, Phenylsulfonyl, Tolylsulfonyl, C₁-C₆-Alkylsulfonyl, Acetyl, Benzoyl oder für eine Aminoschutzgruppe stehen,
mit der Maßgabe, daß mindestens einer der Substituenten R⁶,R⁷,R⁸,R⁹ oder R¹⁰ für Nitro oder die Gruppe NR¹¹R¹² stehen muß,
_{R}^{16 -} für Wasserstoff oder geradkettiges oder verzweigtes, gegebenenfalls durch ein Sauerstoffatom in der Kette unterbrochenes C₁-C₁₂-Alkyl steht,
und
Y - für Wasserstoff, C₇-C₁₄-Aralkyl, C₁-C₈-Alkyl, Phenylsulfonyl, Tolylsulfonyl, C_{I}-C₈-Alkylsulfonyl oder für eine Aminoschutzgruppe steht, dadurch gekennzeichnet, daß man
Aldehyde der allgemeinen Formel III 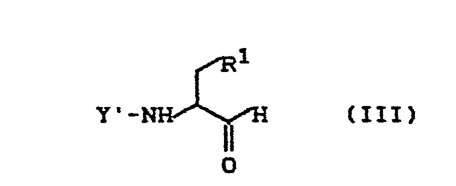 in welcher
R^{l-} die angegebene Bedeutung hat und
Y'- die für Y angegebene Bedeutung hat jedoch nicht. für Wasserstoff steht,
in inerten organischen Lösemitteln, gegebenenfalls in Anwesenheit einer Base bei Temperaturen zwischen -100°C und 50°C mit Essigsäureestern der allgemeinen Formel (IV) 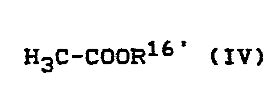 in welcher
R^{16'}- für geradkettiges oder verzweigtes gegebenenfalls durch ein Sauerstoffatom in der Kette unterbrochenes Alkyl mit bis zu 12 Kohlenstoffatomen, bevorzugt mit bis zu-6 Kohenstoffatomen, steht,
umsetzt und das anfallende Stereoisomerengemisch gegebenenfalls nach üblichen Methoden trennt und gegebenenfalls anschließend Schutzgruppen abspaltet.

13. Aldehyde der allgemeinen Formel III 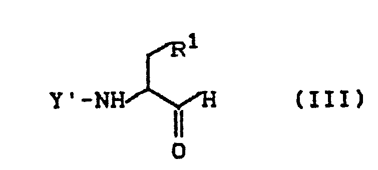 in welcher
R¹ für eine Gruppe der Formel 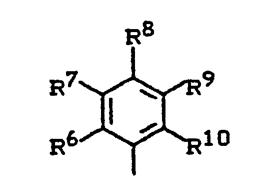 steht, worin
R⁶,R⁷,R⁸,R⁹ und R¹⁰ - gleich oder verschieden sind und
- für Wasserstoff, C₁-C₆-Alkyl, Cₗ-C₆-Alkox^{y}, Halogen, Hydroxy, Cyano, Trifluormethyl, Trifluormethoxy, Trifluormethylthio, Nitro oder für eine Gruppe der Formel 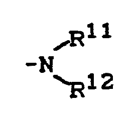 stehen, wobei
R¹¹,R^{12 -} gleich oder verschieden sind und
- für Wasserstoff, C₁-C₆-Alkyl, Aryl, Aralkyl, Phenylsulfonyl, Tolylsulfonyl, Cₗ-C₆-Alkylsulfonyl, Acetyl, Benzoyl oder für eine Aminoschutzgruppe stehen, mit der Maßgabe, daß mindestens einer der Substituenten R⁶,R⁷,R⁸,R⁹ oder R¹⁰ für Nitro oder die Gruppe NR¹¹R¹² stehen _{muß},
und
Y'- für C₁-C₈-Alkyl, C₇-C₁₄Aralkyl, Phenylsulfonyl, Tolylsulfonyl, C₁-C₈-Alkylsulfonyl oder für eine Aminoschutzgruppe steht.

14. Verfahren zur Herstellung von Aldehyden der allgemeinen Formel III 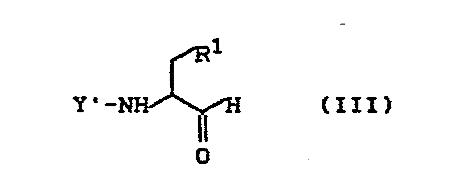 in welcher
R¹ für eine Gruppe der Formel 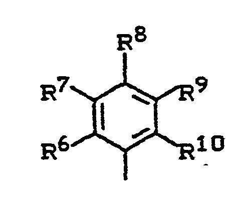 steht, worin
R⁶,R⁷,R⁸,R⁹ und R¹⁰ - gleich oder verschieden sind und - für Wasserstoff, Cₗ-C₆-Alkyl, C₁-C₆-Alkoxy, Halogen, Hydroxy, Cyano, Trifluormethyl, Trifluormethoxy, Trifluormethylthio, Nitro oder für eine Gruppe der Formel 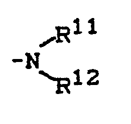 stehen, wobei
R¹¹,R¹²- gleich oder verschieden sind und
- für Wasserstoff, C₁-C₆-Alkyl, Aryl, Aralkyl, Phenylsulfonyl, Tolylsulfonyl, C₁-C₆-Alkylsulfonyl, Acetyl, Benzoyl oder für eine Aminoschutzgruppe stehen,
mit der Maßgabe, daß mindestens einer der Substituenten R⁶,R⁷,R⁸,R⁹ oder R¹⁰ für Nitro oder die Gruppe NR¹¹R¹² stehen muß,
und
Y - für Cₗ-C₈-Alkyl, C₇-C₁₄-Aralkyl, Phenylsulfonyl, Tolylsulfonyl, C₁-C₈-Alkylsulfonyl oder für eine Aminoschutzgruppe steht,
dadurch gekennzeichnet, daß man
[A] Alkohole der allgemeinen Formel (IV) 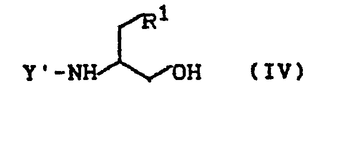 in welcher Y' und R¹ die angegebene Bedeutung haben
gegebenenfalls in Anwesenheit einer Base und/oder eines Hilfsstoffs in inerten organischen Lösemitteln oxidiert,
oder daß man
[B] Aminosäurederivate der allgemeinen Formel (V) 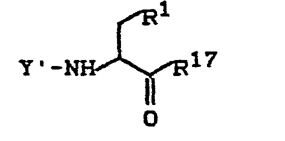 in welcher
R^{l} und Y' die angegebene Bedeutung haben
und
R¹⁷ - für Hydroxy, für C₁-C₆-Alkoxy, für Di-C₁-C₄- alkylamino, oder für N-Methoxy-N-methylamino 1-Imidazolyl steht,
gegebenenfalls in Anwesenheit eines Hilfsmittels reduziert.

15. Arzneimittel enthaltend mindestens eine Verbindung der allgemeinen Formel I gemäß Anspruch 1.

16. Verfahren zur Herstellung von Arzneimitteln, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.

17. Verwendung von Verbindungen der allgemeinen Formel I gemäß Anspruch 1 bei der Herstellung von kreislaufwirksamen Arzneimitteln.
